(19) Europäisches Patentamt
European Patent Office
Office européen des brevets

(11) EP 1 246 791 B1

(12) **EUROPÄISCHE PATENTSCHRIFT**

(45) Veröffentlichungstag und Bekanntmachung des Hinweises auf die Patenterteilung:
**27.10.2004 Patentblatt 2004/44**

(21) Anmeldenummer: **00990824.5**

(22) Anmeldetag: **27.12.2000**

(51) Int Cl.7: **C07C 225/18**, A61K 31/135, A61P 23/00

(86) Internationale Anmeldenummer:
**PCT/EP2000/013282**

(87) Internationale Veröffentlichungsnummer:
**WO 2001/049651 (12.07.2001 Gazette 2001/28)**

(54) **AMINOMETHYL-PHENYL-CYCLOHEXANONDERIVATE**

AMINOMETHYL-PHENYL-CYCLOHEXANONE DERIVATIVES

DERIVES D'AMINOMETHYLE-PHENYLE-CYCLOHEXANE

(84) Benannte Vertragsstaaten:
**AT BE CH CY DE DK ES FI FR GB GR IE IT LI LU MC NL PT SE TR**
Benannte Erstreckungsstaaten:
**LT LV SI**

(30) Priorität: **05.01.2000 DE 10000311**

(43) Veröffentlichungstag der Anmeldung:
**09.10.2002 Patentblatt 2002/41**

(73) Patentinhaber: **Grünenthal GmbH**
**52078 Aachen (DE)**

(72) Erfinder:
- **PÜTZ, Claudia**
  **52349 Düren (DE)**
- **BUSCHMANN, Helmut**
  **52066 Aachen (DE)**
- **KÖGEL, Babette-Yvonne**
  **52379 Langerwehe-Hamich (DE)**

(56) Entgegenhaltungen:
**EP-A- 0 780 369** **WO-A-95/21813**
**DE-A- 19 525 137**

- **FLICK K ET AL: "UNTERSUCHUNGEN ZUR CHEMISCHEN STRUKTUR UND ANALGETISCHEN WIRKUNG VON PHENYLSUBSTITUIERTEN AMINOMETHYLCYCLOHEXANOLEN STUDIES ON CHEMICAL STRUCTURE AND ANALGETIC ACTIVITY OF PHENYL SUBSTITUTED AMINOMETHYLCYCLOHEXANOLES" ARZNEIMITTEL FORSCHUNG. DRUG RESEARCH, EDITIO CANTOR. AULENDORF, DE, Bd. 28, Nr. 1A, 1978, Seiten 107-113, XP000608150 ISSN: 0004-4172**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft Aminomethyl-Phenyl-Cyclohexanonderivate, sowie Verfahren zu deren Herstellung, die Verwendung von Aminomethyl-Phenyl-Cyclohexanonderivaten zur Herstellung von Arzneimitteln und Arzneimittel enthaltend Aminomethyl-Phenyl-Cyclohexanonderivate.

Die Behandlung chronischer und nicht chronischer Schmerzzustände hat in der Medizin eine große Bedeutung. Es besteht ein weltweiter Bedarf an gut wirksamen Schmerztherapien für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist. Dies zeigt sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide wie z.B. Morphin sind bei der Therapie starker bis stärkster Schmerzen gut wirksam. Ihr Einsatz wird jedoch durch die bekannten Nebenwirkungen z.B. Atemdepression, Erbrechen, Sedierung, Obstipation, Sucht, Abhängigkeit und Toleranzentwicklung limitiert. Sie können daher nur unter besonderen Vorsichtsmaßnahmen wie z.B. speziellen Verordnungsvorschriften über einen längeren Zeitraum oder in höheren Dosierungen gegeben werden (Goodman, Gilman, The Pharmacological Basis of Therapeutics, Pergamon Press, New York 1990). Außerdem zeigen sie bei einigen Schmerzzuständen, insbesondere bei neuropathischen Schmerzen, eine geringere Wirksamkeit.

Ein weiteres bekanntes Therapeutikum zur Behandlung starker Schmerzen ist Tramadolhydrochlorid - (1RS, 2RS)-2-[(Dimethylamino)methyl]-1-(3-methoxyphenyl)cyclohexanol, Hydrochlorid. Es nimmt unter den zentralwirksamen Analgetika insofern eine Sonderstellung ein, daß dieser Wirkstoff eine starke Schmerzhemmung ohne die für Opioide bekannten Nebenwirkungen hervorruft (J. Pharmacol. Exptl. Ther. 267, 331 (1993)), wobei sowohl die Enantiomeren von Tramadol als auch die Enantiomeren der Tramadolmetabolite an der analgetischen Wirkung beteiligt sind (J. Pharmacol. Exp. Ther. 260, 275 (1992)). Auch Tramadol ist allerdings nicht nebenwirkungsfrei.

Substituierte Aminomethyl-Phenyl-Cyclohexanonderivate werden in der DE 195 25 137 A1 (Grünenthal GmbH) bereits ebenso beschrieben wie in der deutschen Patentanmeldung 198 30 105.7-44 (Grünenthal GmbH). Es handelt sich aber stets um Synthesevorstufen, die selbst nicht als Wirkstoffe in Arzneimitteln oder als analgetisch wirksam beschrieben werden.

Eine der Erfindung zugrundeliegende Aufgabe bestand darin, analgetisch wirksame Substanzen zur Verfügung zu stellen, die sich zur Schmerztherapie eignen. Darüber hinaus sollten diese Substanzen möglichst wenig Nebenwirkungen wie z.B. Übelkeit, Erbrechen, Abhängigkeit, Atemdepression oder Obstipation aufweisen.

Diese Aufgabe wird durch die erfindungsgemäßen Aminomethyl-Phenyl-Cyclohexanonderivate gelöst. Gegenstand der Erfindung sind daher Aminomethyl-Phenyl-Cyclohexanonderivate der allgemeinen Formel I bzw. Ia, auch in Form ihrer Diastereomere oder Enantiomere sowie ihrer freien Basen oder eines mit einer physiologisch verträglichen Säure gebildeten Salzes, insbesondere des Hydrochloridsalzes,

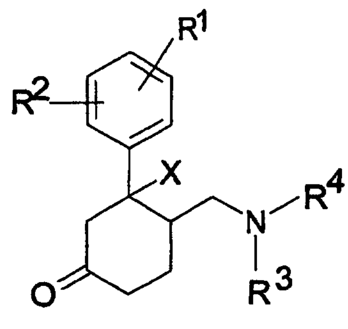

I

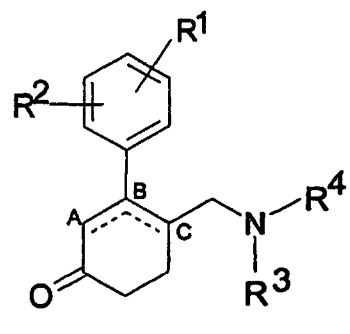

Ia

, worin

$R^1$ und $R^2$ unabhängig voneinander ausgewählt sind aus $R^5$ oder $YR^5$ mit Y = $C_1$-$C_{10}$-Alkyl, $C_2$-$C_{10}$-Alkenyl oder $C_2$-$C_{10}$-Alkinyl, verzweigt oder unverzweigt, einfach oder mehrfach substituiert oder unsubstituiert, wobei $R^5$ ausgewählt ist aus

H, F, Cl, Br, I, CN, $NO_2$; $C_1$-$C_8$-Alkyl, $C_2$-$C_8$-Alkenyl oder $C_2$-$C_8$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^6$ ersetzt ist, mit $R^6$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

Aryl oder Heteroaryl, jeweils einfach oder mehrfach substituiert oder unsubstituiert;

$OR^7$, $OC(O)R^7$, $OC(O)OR^7$, $OC(S)R^7$, $C(O)R^7$, $C(O)OR^7$, $C(S)R^7$, $C(S)OR^7$, $SR^7$, $S(O)R^7$ bzw. $S(O_2)R^7$, wobei $R^7$ ausgewählt ist aus

H, $C_1$-$C_{18}$-Alkyl, $C_2$-$C_{18}$-Alkenyl oder $C_2$-$C_{18}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^8$ ersetzt ist, mit $R^8$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

Alkylaryl, gesättigt oder ungesättigt, einfach oder mehrfach substituiert oder unsubstituiert; Aryl oder Heteroaryl, jeweils einfach oder mehrfach substituiert oder unsubstituiert; oder

$NR^9R^{10}$, $C(O)NR^9R^{10}$ oder $S(O_2)NR^9R^{10}$, wobei $R^9$ und $R^{10}$ unabhängig voneinander ausgewählt sind aus

H, $C_1$-$C_{18}$-Alkyl, $C_2$-$C_{18}$-Alkenyl oder $C_2$-$C_{18}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^{11}$ ersetzt ist, mit $R^{11}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder

$R^9$ und $R^{10}$ zusammen ein $C_3$-$C_7$-Cycloalkyl, gesättigt oder ungesättigt, einfach oder mehrfach substituiert oder unsubstituiert, bilden, bzw. einen entsprechenden Heterocyclus, bei dem ein C-Atom im Ring durch S, O oder $NR^{12}$ ersetzt ist, mit $R^{12}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

oder

$R^1$ und $R^2$ zusammen -CH=CH-CH=CH- bilden, wobei das entstehende Naphtylsystem ein- oder mehrfach substituiert sein kann,

X ausgewählt ist aus

H, F, Cl, Br, I, $CF_3$, $O$-$S(O_2)$-$C_6H_4$-$pCH_3$, $OR^{13}$ oder $OC(O)R^{13}$, wobei $R^{13}$ ausgewählt ist aus

H; $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch N, S oder O ersetzt ist; Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder,

wenn die Verbindung kein X aufweist, gemäß Formel Ia zwischen C-Atom A und C-Atom B oder C-Atom B und C-Atom C eine Doppelbindung ausgebildet ist;

und

$R^3$, $R^4$ unabhängig voneinander ausgewählt sind aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch N, S oder O ersetzt ist; Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder

$R^3$ und $R^4$ zusammen ein $C_3$-$C_7$-Cycloalkyl, gesättigt oder ungesättigt, einfach oder mehrfach substituiert oder unsubstituiert,bilden, bzw. einen entsprechenden Heterocyclus, bei dem ein C-Atom im Ring durch S, O oder $NR^{14}$ ersetzt ist, mit $R^{14}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert.

Nicht Gegenstand dieser Erfindung sind hingegen

- Verbindungen als solche, in denen X = OH, $R^2$ = H, $R^3$ und $R^4$ = $CH_3$, und $R^1$ gleichzeitig $R^5$ mit $R^5$ = $OR^7$ mit $R^7$ = H, $CH_3$ bzw. eine unsubstituierte oder substituierte Pyridyl-, Thienyl-, Thiazoyl- oder Phenylgruppe, oder $R^5$ = $OC(O)OR^7$ mit $R^7$ = $C_1$-$C_5$-Alkyl, oder $R^5$ = $OC(O)R^7$ mit $R^7$ = $C_{1-5}$-Alkyl, NH-$C_6H_4$-$C_{1-3}$-Alkyl, $C_6H_4$-$OC(O)C_{1-3}$-Alkyl, $C_6H_4$-$CH_2$-N($C_{1-4}$-Alkyl)$_2$, $C_6H_4$-$CH_2$-(N-4-Morpholino) bzw. CHZ'-NHZ" mit Z' und Z" gleich oder verschieden ausgewählt aus H oder $C_{1-6}$-Alkyl, ist,

- oder Verbindungen als solche, in denen X = H oder gemäß Formel Ia zwischen C-Atom A und C-Atom B eine Doppelbindung ausgebildet ist, $R^2$ = H, $R^3$ und $R^4$ = $C_{1-6}$-Alkyl, Aryl oder $C_{3-7}$-Cycloalkyl und $R^1$ gleichzeitig H, $C_{1-6}$-Alkoxy, O-$C_{3-7}$-Cycloalkyl, O-Aryl oder O-Heterocyclyl sind.

Dabei versteht man im Zusammenhang mit Alkyl, Alkenyl Alkinyl und Cycloalkyl bzw. dem "entsprechenden Heterocyclus" unter dem Begriff substituiert im Sinne dieser Erfindung die Substitution eines Wasserstoffrestes durch F, Cl, Br, I, $NH_2$, SH oder OH, wobei unter mehrfach substituierten Resten Reste zu verstehen sind, die sowohl an verschiedenen als auch an gleichen Atomen mehrfach substituiert sind, beispielsweise dreifach am gleichen C-Atom wie im Falle von $CF_3$ oder an verschiedenen Stellen wie im Falle von -CH(OH)-CH=CH-$CHCl_2$.

Weiter bedeutet -C(O)-

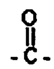

, was auch für -C(S)- oder -S(O)- bzw. -S($O_2$)- gilt.

Der Ausdruck "$C_1$-$C_8$-Alkyl" bzw. "$C_1$-$C_{10}$-Alkyl" bedeutet im Sinne dieser Erfindung Kohlenwasserstoffe mit 1 bis 8 bzw. 10 Kohlenstoffatomen.

Bespielhaft seien Methyl, Ethyl, Propyl, Isopropyl, n-Butan, sek.-Butyl, tert-Butyl, n-Pentan, Neopentyl, n-Hexan, n-Heptan, n-Octan, n-Nonan oder n-Decan genannt

Der Ausdruck "$C_1$-$C_{18}$-Alkyl" bedeutet im Sinne dieser Erfindung Kohlenwasserstoffe mit 1 bis 18 Kohlenstoffatomen. Beispielhaft seien Methyl, Ethyl, Propyl, Isopropyl, n-Butan, sek. Butyl, tert. Butyl, n-Pentan, Neopentyl, n-Butan, sek. Butyl, tert. Butyl, n-Hexan, n-Heptan, n-Octan, n-Nonan, n-Decan, n-Undecan, n-Dodecan, n-Dodecan, n-Tridecan, n-Tetradecan, n-Pentadecan, n-Hexadecan, n-Heptadecan oder n-Octadecan unsubstituiert oder ein oder mehrfach substituiert genannt.

Der Ausdruck "$C_2$-$C_{10}$-Alkenyl" bzw. "$C_2$-$C_{10}$-Alkinyl" oder "$C_2$-$C_{18}$-Alkenyl" bzw. "C2-$C_{18}$-Alkinyl" bedeutet im Sinne dieser Erfindung Kohlenwasserstoffe mit 2 bis 8 bzw. 2 bis 18 Kohlenstoffatomen. Beispielhaft genannt seien Propenyl, Butenyl, Pentenyl, Hexenyl, Heptenyl, Octenyl unsubstituiert oder ein oder mehrfach substituiert bzw. Propinyl, Butinyl, Pentinyl, Hexinyl, Heptinyl, Octinyl unsubstituiert oder ein oder mehrfach substituiert.

Der Ausdruck $C_3$-$C_7$-Cycloalkyl bedeutet im Sinne dieser Erfindung cyclische Kohlenwasserstoffe mit 3 bis 7 Kohlenstoffatomen. Beispielhaft seien Cyclopropyl, Cyclobutyl, Cyclopentyl, Cyclohexyl, Cycloheptyl, Cyclohexenyl oder Cycloheptenyl gesättigt oder ungesättigt, unsubstituiert oder ein oder mehrfach substituiert genannt. Dabei versteht man im Sinne der Erfindung unter einem einem "entsprechenden Heterocyclus" ein $C_3$-$C_7$-Cycloalkyl, bei dem ein C-Atom im Ring durch S, O oder N ersetzt ist. Beispielhaft seien hierfür Pyrrolidin, Pyran, Thiolan, Piperidin oder Tetrahydrofuran aufgeführt.

Der Ausdruck "Aryl" bedeutet im Sinne dieser Erfindung Phenyle oder Naphthyle.

Der Ausdruck "Alkylaryl" bedeutet im Sinne dieser Erfindung mindestens mit $C_1$-$C_{10}$-Alkylen substituierte Aryle, wobei die Ausdrücke Aryl und Alkyl die gleiche Bedeutung haben wie oben. In dieser Gruppe sei insbesondere Benzaryl erwähnt.

Der Ausdruck "Heteroaryl" bedeutet im Sinne dieser Erfindung 5- oder 6-gliedrige, gegebenenfalls mit einem ankondensierten Ringsystem versehene, aromatische Verbindungen, die ein oder zwei Heteroatome aus der Gruppe Stickstoff, Sauerstoff und/oder Schwefel enthalten. In dieser Gruppe seien beispielhaft Furan, Thiophen, Pyrrol, Pyridin, Pyrimidin, Chinolin Isochinolin, Phtlalazin oder Chinazolin aufgeführt.

In Bezug auf Aryl, Alkylaryl oder Heteroaryl versteht man im Sinne dieser Erfindung unter ein- oder mehrfach substituiert, die Substitution des Ringsystems mit F, Cl, Br, I, $NH_2$, SH, OH, $CF_3$; ein- oder mehrfach substituiertem oder unsubstituiertem $C_1$-$C_6$-Alkyl, $C_1$-$C_6$-Alkoxy, $C_2$-$C_8$-Alkenyl, $C_2$-$C_8$-Alkinyl; oder Aryl, insbesondere Phenyl; an einem oder verschiedenen Atomen.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid.

In bevorzugten erfindungsgemäßen Aminomethyl-Phenyl-Cyclohexanon-derivaten gemäß Formel I bzw. Ia ist $R^1 = R^5$, wobei $R^5$ ausgewählt ist aus

H, F, Cl, Br, I, $CHF_2$, $CF_3$, $NO_2$, $NH_2$; $C_1$-$C_4$-Alkyl oder $C_2$-$C_4$-Alkenyl, verzweigt oder unverzweigt, einfach oder mehrfach substituiert oder unsubstituiert; Aryl substituiert oder unsubstituiert; $OR^7$, $C(O)OR^7$ bzw. $SR^7$ wobei $R^7$ ausgewählt ist aus

H; $C_1$-$C_4$-Alkyl, verzweigt oder unverzweigt, einfach oder mehrfach substituiert oder unsubstituiert; vorzugsweise H, $CF_3$ oder $CH_3$,

oder $S(O_2)NR^9R^{10}$, wobei $R^9$ und $R^{10}$ unabhängig voneinander ausgewählt sind aus

H; $C_1$-$C_4$-Alkyl, verzweigt oder unverzweigt, einfach oder mehrfach substituiert oder unsubstituiert;

, wobei insbesondere bevorzugt ist, daß R1 = R5 mit R5 ausgewählt aus

H, F, Cl, OH, $CH_3$, $C_2H_5$, $C_2H_3$, $CHF_2$, $CF_3$, $SCH_3$, $OCF_3$, $OCH_3$, $OC_2H_5$, $C(O)OCH_3$, $C(O)OC_2H_5$ oder Phenyl,

während $R^2$, X, $R^3$ und $R^4$ eine der bereits genannten Bedeutungen haben.

In weiteren bevorzugten erfindungsgemäßen Aminomethyl-Phenyl-Cyclohexanon-derivaten gemäß Formel I bzw. Ia ist $R^2 = R^5$, wobei $R^5$ ausgewählt ist aus

H, F, Cl, Br, I, $SCH_3$; $C_1$-$C_4$-Alkyl, $C_2$-$C_4$-Alkenyl verzweigt oder unverzweigt, einfach oder mehrfach substituiert oder unsubstituiert, vorzugsweise $CF_3$; $OR^7$, mit $R^7$ ausgewählt aus $C_1$-$C_4$-Alkyl, verzweigt oder unverzweigt, einfach oder mehrfach substituiert oder unsubstituiert, vorzugsweise $CH_3$;

während $R^1$, X, $R^3$ und $R^4$ eine der bereits genannten Bedeutungen haben.

In weiteren bevorzugten erfindungsgemäßen Aminomethyl-Phenyl-Cyclohexanon-derivaten gemäß Formel I bzw. Ia haben $R^1$ und $R^2$ unterschiedliche Bedeutungen oder $R^1$ und $R^2$ bilden zusammen -CH=CH-CH=CH-, wobei das entstehende Naphthylsystem ein- oder mehrfach, vorzugsweise mit Halogen, $OC_{1-3}$-Alkyl oder $C_{1-3}$-Alkyl, unsubstituiert oder ein oder mehrfach substituiert, insbesondere mit $OCH_3$; substituiert, sein kann.

In ebenfalls bevorzugten erfindungsgemäßen Aminomethyl-Phenyl-Cyclohexanon-derivaten gemäß Formel I bzw. Ia ist X ausgewählt aus

H, F, Cl, OH, $CF_3$, O-$S(O_2)$-$C_6H_4$-p$CH_3$ bzw. $OC(O)R^7$ mit $R^7$ = H; $C_1$-$C_4$-Alkyl oder $C_2$-$C_4$-Alkeny), verzweigt oder unverzweigt, einoder mehrfach substituiert oder unsubstituiert,

vorzugsweise H, F, Cl, OH, O-$S(O_2)$-$C_6H_4$-p$CH_3$, $OC(O)R^7$ mit $R^7$ = $C_1$-$C_4$-Alkyl, vorzugsweise $CH_3$, insbesondere H oder OH;

oder

, wenn die Verbindung kein X aufweist, ist gemäß Formel Ia zwischen C-Atom A und C-Atom B oder C-Atom B und C-Atom C eine Doppelbindung ausgebildet,

während $R^1$, $R^2$, $R^3$ und $R^4$ eine der bereits genannten Bedeutungen haben.

In weiteren erfindungsgemäßen Aminomethyl-Phenyl-Cyclohexanonderivaten gemäß Formel I bzw. Ia

sind $R^3$ und $R^4$ unabhängig voneinander ausgewählt aus

$C_1$-$C_4$-Alkyl, verzweigt oder unverzweigt, einfach oder mehrfach substituiert oder unsubstituiert, vorzugsweise $CH_3$,

oder

bilden $R^3$ und $R^4$ zusammen ein $C_3$-$C_7$-Cycloalkyl, gesättigt oder ungesättigt, einfach oder mehrfach substituiert oder unsubstituiert,

während $R^1$, $R^2$ und X eine der bereits genannten Bedeutungen haben.

Ein weiterer Gegenstand der Erfindung ist ein Verfahren zur Herstellung der als solche bereits als Gegenstand der Erfindung beschriebenen Aminomethyl-Phenyl-Cyclohexanonderivate nach Formel Ia oder nach Formel I mit X = H, bei dem Verbindungen der Formel II

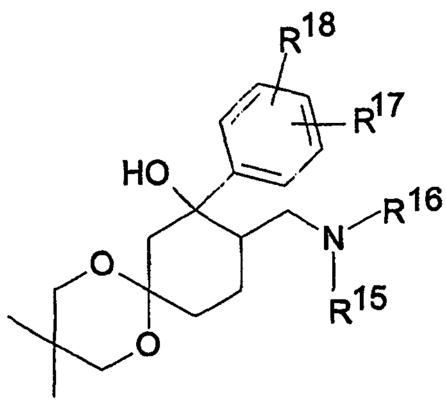

II

, in denen $R^{15}$ eine $R^3$, $R^{16}$ eine $R^4$, $R^{17}$ eine $R^1$ und $R^{18}$ eine $R^2$ entsprechende Bedeutung wie bereits beschrieben haben, mit Säuren, vorzugsweise Salzsäure, Ameisensäure oder Essigsäure, bei Raumtemperatur umgesetzt werden. Anschließend wird das Produkt gemäß Formel Ia entweder als Endprodukt aufgereinigt oder das Produkt mit katalytisch aktiviertem Wasserstoffzu einem Produkt gemäß Formel I mit X = H hydriert und dann aufgereinigt. Die Hydrierung erfolgt vorzugsweise mit auf einem Trägermaterial wie Aktivkohle absorbiertem Platin oder Palladium als Katalysator, in Essigsäureethylester oder einem $C_1$ - $C_4$-Alkylalkohol, bei Drücken von 0,1 bis 10 bar und/oder Temperaturen von 20°C bis 80°C.

Ebenfalls Gegenstand der Erfindung ist ein Verfahren zur Herstellung der als solche bereits als Gegenstand der Erfindung beschriebenen Aminomethyl-Phenyl-Cyclohexanonderivate nach Formel I mit X ≠ H, bei dem Verbindungen der Formel II

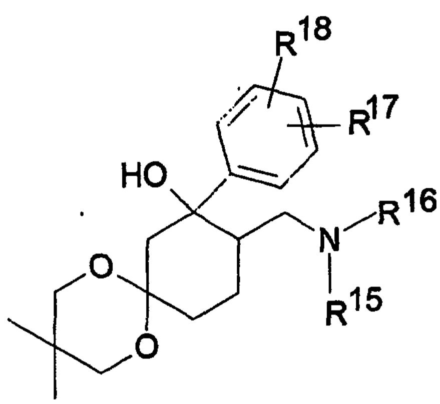

II

, in denen $R^{15}$ eine $R^3$, $R^{16}$ eine $R^4$, $R^{17}$ eine $R^1$ und $R^{18}$ eine $R^2$ entsprechende Bedeutung wie bereits beschrieben haben, mit Säuren, vorzugsweise Salzsäure, Ameisensäure oder Essigsäure, bei Temperaturen zwischen 0°C und 5°C umgesetzt werden. Anschließend wird das Produkt gemäß Formel I mit X = OH und unveränderten $R^{15}$ bis $R^{18}$ entweder als Endprodukt aufgereinigt oder weiterverarbeitet. Wenn das Produkt zu einer Verbindung mit X gleich F, Cl, Br, I bzw. $CF_3$ umgesetzt werden soll, so wird nach bekannten Verfahren die für das X stehende OH-Gruppe gegen F bzw. Cl bzw. Br bzw. I bzw. $CF_3$ ausgetauscht. Wenn das Produkt zu einer Verbindung mit X gleich $OR^{13}$ umgesetzt werden soll, wobei $R^{13}$ eine der bereits beschriebenen Bedeutungen hat, so wird die für das X stehende OH-Gruppe mit einem Halogenid der Formel III

$R^{19}Cl$

**III**

verethert, wobei $R^{19}$ eine $R^{13}$ analoge Bedeutung hat. Wenn das Produkt zu einer Verbindung mit X gleich O-S($O_2$) -$C_6H_4$-$CH_3$, oder OC(O)$R^{13}$ umgesetzt werden soll, wobei $R^{13}$ eine der bereits beschriebenen Bedeutungen hat, so wird die für das X stehende OH-Gruppe mit einem Säurechlorid der Formel IV

$R^{20}COCl$

**IV** (IV

oder Cl-S($O_2$)-$C_6H_4$-$CH_3$ verestert, wobei $R^{20}$ eine $R^{13}$ analoge Bedeutung hat. Abschließend wird das Endprodukt aufgereinigt.

Gegenstand der Erfindung ist auch ein Herstellungsverfahren für das Ausgangsprodukts gemäß Formel II der oben beschriebenen Verfahren. Dabei wird zunächst 3,3-Dimethyl-1,5-dioxa-spiro[5.5]undecan-8-on

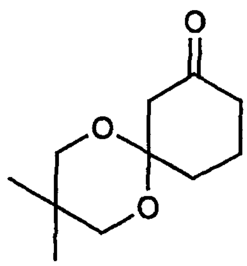

mit Immoniumsalzen der Formel V bzw. mit Formaldehyd und einem Amin der Formel VI umgesetzt werden, wobei $R^{15}$ eine $R^3$ und $R^{16}$ eine $R^4$ entsprechende Bedeutung wie bereits beschrieben haben.

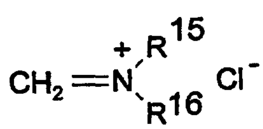

**V**

$R^{15}R^{16}NH$ VI

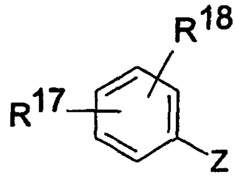

**VII**

Anschließend werden die so erhaltenen Mannich-Basen mit einer metallorganischen Verbindung der Formel VII, in der Z MgCl, MgBr, MgI oder Lithium bedeuten und $R^{17}$ eine $R^1$ und $R^{18}$ eine $R^2$ entsprechende Bedeutung wie bereits beschrieben haben, bei Temperaturen zwischen -70°C und 60°C umgesetzt. Bevorzugte Lösungsmittel bei dieser Reaktion sind Diethylether oder Tetrahydrofuran. Die Umsetzung mit einer Grignard-Verbindung der Formel **VII** kann mit oder ohne Zusatz eines Mitführreagenzes erfolgen. Bei Einsatz eines Mitführreagenzes ist 1,2-Dibromethan bevorzugt.

Grignard-Verbindungen der Formel **VII,** in der Z gleich MgCl, MgBr oder MgI bedeutet, sind überwiegend käuflich zu erwerben, lassen sich aber auch durch Umsetzung von Halogenverbindungen der Formel **VIII,** in der A gleich Cl, Br oder I bedeutet und $R^{17}$ eine $R^1$ und $R^{18}$ eine $R^2$ entsprechende Bedeutung wie bereits beschrieben haben, mit Magnesium darstellen. Lithiumorganische Verbindungen der Formel **VII**, in der Z Li bedeutet, lassen sich durch Umsetzung von Halogenverbindungen der Formel **VIII**, in der A gleich Cl, Br oder I bedeutet mit beispielsweise n-Butyllithium/Hexan-Lösung durch Halogen-Lithiumaustausch erhalten.

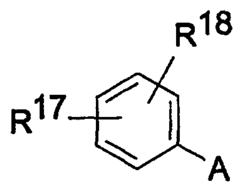

**VIII**

Unter den genannten Reaktionsbedingungen können OH- SH und $NH_2$-Gruppen unerwünschte Nebenreaktionen eingehen. Es ist daher bevorzugt, diese mit Schutzgruppen zu versehen oder im Falle von $NH_2$ durch $NO_2$ zu ersetzen und im letzten Reaktionsschritt vor der Aufreinigung die Schutzgruppe abzuspalten, bzw. die $NO_2$-Gruppe zu reduzieren. Ein weiterer Gegenstand der Anmeldung ist daher eine Abwandlung der oben beschriebenen Verfahren, bei dem in $R^{15}$ bis $R^{20}$ gemäß Formeln II bis VII, aber gegebenenfalls auch VIII, mindestens eine OH-Gruppe durch eine $OSi(Ph)_2$tert.but.-Gruppe, mindestens eine SH-Gruppe durch eine S-p-Methoxybenzylgruppe und/oder mindestens eine $NH_2$-Gruppe durch eine $NO_2$-Gruppe ersetzt wurde und nach Abschluß der gesamten Reaktionssequenz vor der Aufreinigung des Endprodukts eine $OSi(Ph)_2$tert.but.-Gruppe mit Tetrabutylammoniumflluorid in Tetrahydrofuran und/oder mindestens eine p-Methoxybenzylgruppe mit einem Metallamin bevorzugt Natriumamin abgespalten und/oder mindestens eine $NO_2$-Gruppe zu $NH_2$ reduziert wird.

Weiter sind Carbonsäure- oder Thiocarbonsäure-Gruppen unter den genannten Reaktionsbedingungen unter Umständen nicht stabil, so daß es bevorzugt ist, deren Methylester umzusetzen und das Verfahrensprodukt nach Abschluß der gesamten Reaktionssequenz vor der Aufreinigung mit KOH-Lösung bzw. NaOH-Lösung in Methanol bei 40°C - 60°C zu verseifen. Ein weiterer Gegenstand der Erfindung ist daher eine Abwandlung der oben beschriebenen Verfahren, in dem nach Abschluß der gesamten Reaktionssequenz vor der Aufreinigung als Endprodukt ein Verfahrensprodukt mit mindestens einer $C(O)OCH_3$-, $OC(O)OCH_3$- und/oder $C(S)OCH_3$-Gruppe mit KOH-Lösung bzw. NaOH-Lösung in Methanol bei 40°C - 60°C verseift wird.

Die Reinigung der in den einzelnen Reaktionssequenzen erhaltenen Verbindungen erfolgt durch Kristallisation oder Säulenchromatographie.

Die Verbindungen der Formel **I** bzw. **Ia** lassen sich mit physiologisch verträglichen Säuren wie Salzsäure, Bromwasserstoffsäure, Schwefelsäure, Methansulfonsäure, Ameisensäure, Essigsäure, Oxalsäure, Bernsteinsäure,

Weinsäure, Mandelsäure, Fumarsäure, Milchsäure, Zitronensäure, Glutaminsäure und/oder Asparaginsäure in an sich bekannter Weise in ihre Salze überführen. Vorzugsweise wird die Salzbildung in einem Lösungsmittel wie Diisopropylether, Essigsäurealkylester, Aceton und/oder 2-Butanon durchgeführt. Zur Herstellung der Hydrochloride ist Trimethylchlorsilan in wasserhaltiger Lösung besonders geeignet.

Die erfindungsgemäßen Aminomethyl-Phenyl-Cyclohexanonderivate sind toxikologisch unbedenklich, so daß sie sich als pharmazeutischer Wirkstoff in Arzneimitteln eignen.

Ein weiterer Gegenstand der Erfindung sind daher auch Arzneimittel, die als Wirkstoff mindestens ein Aminomethyl-Phenyl-Cyclohexanonderivat der allgemeinen Formel I bzw. Ia

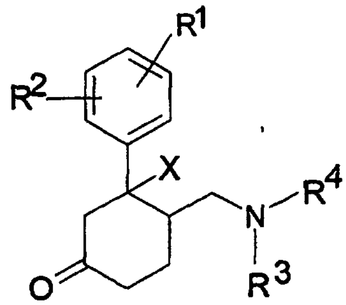

I

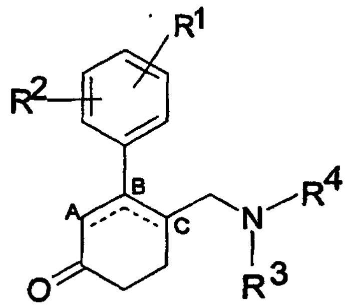

Ia

, worin

$R^1$ und $R^2$ unabhängig voneinander ausgewählt sind aus $R^5$ oder $YR^5$ mit Y = $C_1$-$C_{10}$-Alkyl, $C_2$-$C_{10}$-Alkenyl oder $C_2$-$C_{10}$-Alkinyl, verzweigt oder unverzweigt, einfach oder mehrfach substituiert oder unsubstituiert, wobei $R^5$ ausgewählt ist aus

H, F, Cl, Br, I, CN, $NO_2$; $C_1$-$C_8$-Alkyl, $C_2$-$C_8$-Alkenyl oder $C_2$-$C_8$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^6$ ersetzt ist, mit $R^6$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

Aryl oder Heteroaryl, jeweils einfach oder mehrfach substituiert oder unsubstituiert;

$OR^7$, $OC(O)R^7$, $OC(O)OR^7$, $OC(S)R^7$, $C(O)R^7$, $C(O)OR^7$, $C(S)R^7$, $C(S)OR^7$, $SR^7$, $S(O)R^7$ bzw. $S(O_2)R^7$, wobei $R^7$ ausgewählt ist aus

H, $C_1$-$C_{18}$-Alkyl, $C_2$-$C_{18}$-Alkenyl oder $C_2$-$C_{18}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^8$ ersetzt ist, mit $R^8$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

Alkylaryl, gesättigt oder ungesättigt, einfach oder mehrfach substituiert oder unsubstituiert; Aryl oder Heteroaryl, jeweils einfach oder mehrfach substituiert oder unsubstituiert; oder

$NR^9R^{10}$, $C(O)NR^9R^{10}$ oder $S(O_2)NR^9R^{10}$, wobei $R^9$ und $R^{10}$ unabhängig voneinander ausgewählt sind aus

H, $C_1$-$C_{18}$-Alkyl, $C_2$-$C_{18}$-Alkenyl oder $C_2$-$C_{18}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^{11}$ ersetzt ist, mit $R^{11}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert; Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder

$R^9$ und $R^{10}$ zusammen ein $C_3$-$C_7$-Cycloalkyl, gesättigt oder ungesättigt, einfach oder mehrfach substituiert

oder unsubstituiert, bilden, bzw. einen entsprechenden Heterocyclus, bei dem ein C-Atom im Ring durch S, O oder $NR^{12}$ ersetzt ist, mit $R^{12}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

oder

$R^1$ und $R^2$ zusammen -CH=CH-CH=CH- bilden, wobei das entstehende Naphthylsystem ein- oder mehrfach substituiert sein kann,

X ausgewählt ist aus

H, F, Cl, Br, I, $CF_3$, O-S($O_2$)-$C_6H_4$-p$CH_3$, $OR^{13}$ oder OC(O)$R^{13}$, wobei $R^{13}$ ausgewählt ist aus

H; $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch N, S oder O ersetzt ist; Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder,

wenn die Verbindung kein X aufweist, gemäß Formel Ia zwischen C-Atom A und C-Atom B oder C-Atom B und C-Atom C eine Doppelbindung ausgebildet ist;

und

$R^3$, $R^4$ unabhängig voneinander ausgewählt sind aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch N, S oder O ersetzt ist; Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder

$R^3$ und $R^4$ zusammen ein $C_3$-$C_7$-Cycloalkyl, gesättigt oder ungesättigt, einfach oder mehrfach substituiert oder unsubstituiert,bilden, bzw. einen entsprechenden Heterocyclus, bei dem ein C-Atom im Ring durch S, O oder $NR^{14}$ ersetzt ist, mit $R^{14}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

in Form seiner Diastereomere oder Enantiomere sowie seiner freien Base oder eines mit einer physiologisch verträglichen Säure gebildeten Salzes, insbesondere des Hydrochloridsalzes enthalten.

Bevorzugt sind Arzneimittel enthaltend mindestens ein Aminomethyl-Phenyl-Cyclohexanonderivat der allgemeinen Formel I bzw. Ia

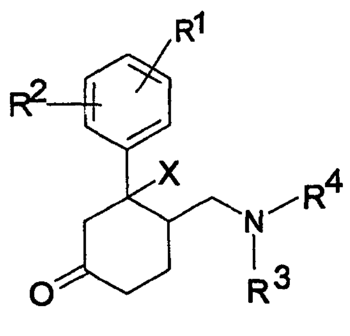

I .

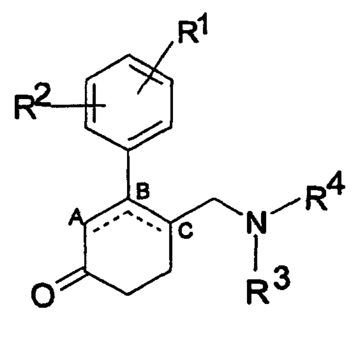

Ia

, in welchem unabhängig voneinander

$R^1$ = $R^5$ ist, wobei $R^5$ ausgewählt ist aus

H, F, Cl, Br, I, $CHF_2$, $CF_3$, $NO_2$, $NH_2$; $C_1$-$C_4$-Alkyl oder $C_2$-$C_4$-Alkenyl, verzweigt oder unverzweigt, einfach oder mehrfach substituiert oder unsubstituiert; Aryl substituiert oder unsubstituiert; $OR^7$, C(O)$OR^7$ bzw. $SR^7$ wobei $R^7$ ausgewählt ist aus

H; $C_1$-$C_4$-Alkyl, verzweigt oder unverzweigt, einfach oder mehrfach substituiert oder unsubstituiert; vor-

zugsweise H, $CF_3$ oder $CH_3$,

oder $S(O_2)NR^9R^{10}$, wobei $R^9$ und $R^{10}$ unabhängig voneinander ausgewählt sind aus

H; $C_1$-$C_4$-Alkyl, verzweigt oder unverzweigt, einfach oder mehrfach substituiert oder unsubstituiert;

, wobei insbesondere bevorzugt ist, daß R1 = R5 mit R5 ausgewählt aus

H, F, Cl, OH, $CH_3$, $C_2H_5$, $C_2H_3$, $CHF_2$, $CF_3$, $SCH_3$, $OCF_3$, $OCH_3$, $OC_2H_5$, $C(O)OCH_3$, $C(O)OC_2H_5$ oder Phenyl,

**und/oder** $R^2$ = $R^5$ ist, wobei $R^5$ ausgewählt ist aus

H, F, Cl, Br, I, $SCH_3$; $C_1$-$C_4$-Alkyl, $C_2$-$C_4$-Alkenyl verzweigt oder unverzweigt, einfach oder mehrfach substituiert oder unsubstituiert, vorzugsweise $CF_3$; $OR^7$, mit $R^7$ ausgewählt aus $C_1$-$C_4$-Alkyl, verzweigt oder unverzweigt, einfach oder mehrfach substituiert oder unsubstituiert, vorzugsweise $CH_3$;

**und/oder** $R^1$ und $R^2$ unterschiedliche Bedeutungen haben oder $R^1$ und $R^2$ zusammen -CH=CH-CH=CH- bilden, wobei das entstehende Naphthylsystem ein- oder mehrfach, vorzugsweise mit Halogen, $OC_{1-3}$-Alkyl oder $C_{1-3}$-Alkyl, unsubstituiert oder ein oder mehrfach substituiert, insbesondere mit $OCH_3$; substituiert, sein kann,

**und/oder** X ausgewählt ist aus

H, F, Cl, OH, $CF_3$, O-$S(O_2)$-$C_6H_4$-p$CH_3$ bzw. $OC(O)R^7$ mit $R^7$ = H; $C_1$-$C_4$-Alkyl oder $C_2$-$C_4$-Alkenyl, verzweigt oder unverzweigt, einoder mehrfach substituiert oder unsubstituiert,

vorzugsweise H, F, Cl, OH, O-$S(O_2)$-$C_6H_4$-p$CH_3$, $OC(O)R^7$ mit $R^7$ = $C_1$-$C_4$-Alkyl, vorzugsweise $CH_3$, insbesondere H oder OH; oder

, wenn die Verbindung kein X aufweist, gemäß Formel Ia zwischen C-Atom A und C-Atom B oder C-Atom B und C-Atom C eine Doppelbindung ausgebildet ist

**und/oder** $R^3$ und $R^4$ unabhängig voneinander ausgewählt sind aus $C_1$-$C_4$-Alkyl, verzweigt oder unverzweigt, einfach oder mehrfach substituiert oder unsubstituiert, vorzugsweise $CH_3$; oder

$R^3$ und $R^4$ zusammen ein $C_3$-$C_7$-Cycloalkyl, gesättigt oder ungesättigt, einfach oder mehrfach substituiert oder unsubstituiert, bilden,

**und/oder** $R^1$, $R^2$, X, $R^3$ und/oder $R^4$ eine der bereits genannten Bedeutungen haben.

Besonders bevorzugt sind dabei Arzneimittel, die als Wirkstoff mindestens ein aus folgender Gruppe ausgewähltes Aminomethyl-Phenyl-Cyclohexanonderivat enthalten:

- *rac-cis*-[4-Dimethylaminomethyl-3-hydroxy-3-(3-methoxy-phenyl)-cyclohexanon],
- *rac-cis*-[4-Dimethylaminomethyl-3-(3-methoxy-phenyl)-cyclohexanon],
- *rac-trans*-[4-Dimethylaminomethyl-3-(3-methoxy-phenyl)-cyclohexanon],
- *rac-cis*-[4-Dimethylaminomethyl-3-(3-hydroxy-phenyl)-cyclohexanon,
- *rac-trans*-[4-Dimethylaminomethyl-3-(3-hydroxy-phenyl)-cyclohexanon oder
- 4-Dimethylaminomethyl-3-(3-methoxy-phenyl)-cyclohex-2-enon
- 4-Dimethylaminomethyl-3-phenyl-cyclohex-2-enon; e
- 4-Dimethylaminomethyl-3-(3-hydroxy-phenyl)-cyclohex-2-enon;
- rac-trans-4-Dimethylaminomethyl-3-phenyl-cyclohexanon;
- rac-cis-4-Dimethylaminomethyl-3-phenyl-cyclohexanon;
- 4-Dimethylaminomethyl-3-(4-methoxy-phenyl)-cyclohex-2-enon;
- 4-Dimethylaminomethyl-3-naphthalen-2-yl-cyclohex-2-enon;
- rac-trans-4-Dimethylaminomethyl-3-hydroxy-3-naphthalen-2-ylcyclohexanon;
- rac-trans-4-Dimethylaminomethyl-3-(3-fluoro-phenyl)-cyclohexanon;
- rac-cis-4-Dimethylaminomethyl-3-(3-fluoro-phenyl)-cyclohexanon;
- rac-cis-4-Dimethylaminomethyl-3-hydroxy-3-(2-methoxy-phenyl)-cyclohexanon
- rac-cis-4-Dimethylaminomethyl-3-hydroxy-3-(6-methoxy-naphthalen-2-yl)-cyclohexanon;
- 4-Dimethylaminomethyl-3-(6-methoxy-naphthalen-2-yl)-cyclohex-2-enon;
- rac-cis-3-Biphenyl-4-yl-4-dimethylaminomethyl-3-hydroxy-cyclohexanon
- 3-(3-Difluoromethyl-phenyl)-4-dimethylaminomethyl-cyclohex-2-enon;
- 4-Dimethylaminomethyl-3-(3-fluoro-phenyl)-cyclohex-2-enon;
- 3-(3,4-Difluoro-phenyl)-4-dimethylaminomethyl-cyclohex-2-enon;
- 4-Dimethylaminomethyl-3-(4-fluoro-phenyl)-cyclohex-2-enon;
- 4-Dimethylaminomethyl-3-(4-trifluoromethyl-phenyl)-cyclohex-2-enon;
- 3-(3,5-Dichloro-phenyl)-4-dimethylaminomethyl-cyclohex-2-enon;
- 3-(3,5-Difluoro-phenyl)-4-dimethylaminomethyl-cyclohex-2-enon;
- rac-cis-3-(3,5-Difluoro-phenyl)-4-dimethylaminomethyl-3-hydroxycyclohexanon

als freie Base oder in Form eines mit einer physiologisch verträglichen Säure gebildeten Salzes, insbesondere des Hydrochloridsalzes.

Die erfindungsgemäßen Arzneimittel enthalten neben mindestens einem Aminomethyl-Phenyl-Cyclohexanonderivat Trägermaterialien Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster oder Aerosolen verabreicht werden. Die Auswahl der Hilfsstoffe sowie die einzusetzenden Mengen derselben hängt davon ab, ob das Arzneimittel oral, peroral, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße substituierte Aminomethyl-Phenyl-Cyclohexanderivate in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die Aminomethyl-Phenyl-Cyclohexanonderivate verzögert freisetzen. Die an den Patienten zu verabreichende Wirkstoffmenge variiert in Abhängigkeit vom Gewicht des Patienten, von der Applikationsart, der Indikation und dem Schweregrad der Erkrankung. Üblichweise werden 50 bis 500 mg/kg wenigstens eines Aminomethyl-Phenyl-Cyclohexanonderivats appliziert.

Vorzugsweise werden die Aminomethyl-Phenyl-Cyclohexanonderivate zur Schmerzbehandlung eingesetzt, so daß ein weiterer Erfindungsgegenstand die Verwendung mindestens eines Aminomethyl-Phenyl-Cyclohexanonderivats der allgemeinen Formel I bzw. Ia

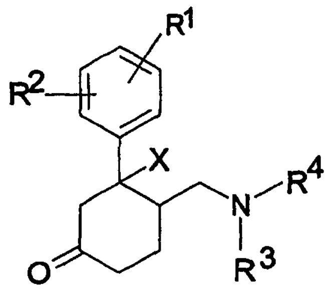

I

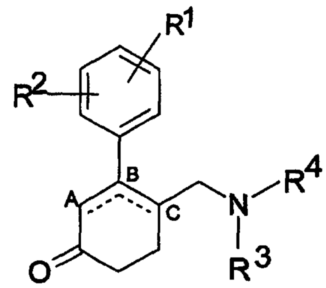

Ia

, worin

$R^1$ und $R^2$ unabhängig voneinander ausgewählt sind aus $R^5$ oder $YR^5$ mit Y = $C_1$-$C_{10}$-Alkyl, $C_2$-$C_{10}$-Alkenyl oder $C_2$-$C_{10}$-Alkinyl, verzweigt oder unverzweigt, einfach oder mehrfach substituiert oder unsubstituiert, wobei $R^5$ ausgewählt ist aus

H, F, Cl, Br, I, CN, $NO_2$; $C_1$-$C_8$-Alkyl, $C_2$-$C_8$-Alkenyl oder $C_2$-$C_8$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^6$ ersetzt ist, mit $R^6$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

Aryl oder Heteroaryl, jeweils einfach oder mehrfach substituiert oder unsubstituiert;

$OR^7$, $OC(O)R^7$, $OC(O)OR^7$, $OC(S)R^7$, $C(O)R^7$, $C(O)OR^7$, $C(S)R^7$, $C(S)OR^7$, $SR^7$, $S(O)R^7$ bzw. $S(O_2)R^7$, wobei $R^7$ ausgewählt ist aus

H, $C_1$-$C_{18}$-Alkyl, $C_2$-$C_{18}$-Alkenyl oder $C_2$-$C_{18}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^8$ ersetzt ist, mit $R^8$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

Alkylaryl, gesättigt oder ungesättigt, einfach oder mehrfach substituiert oder unsubstituiert; Aryl oder Heteroaryl, jeweils einfach oder mehrfach substituiert oder unsubstituiert; oder

$NR^9R^{10}$, $C(O)NR^9R^{10}$ oder $S(O_2)NR^9R^{10}$, wobei $R^9$ und $R^{10}$ unabhängig voneinander ausgewählt sind aus

H, $C_1$-$C_{18}$-Alkyl, $C_2$-$C_{18}$-Alkenyl oder $C_2$-$C_{18}$-Alkinyl, jeweils verzweigt oder unverzweigt, einfach oder mehrfach

substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^{11}$ ersetzt ist, mit $R^{11}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder

$R^9$ und $R^{10}$ zusammen ein $C_3$-$C_7$-Cycloalkyl, gesättigt oder ungesättigt, einfach oder mehrfach substituiert oder unsubstituiert, bilden, bzw. einen entsprechenden Heterocyclus, bei dem ein C-Atom im Ring durch S, O oder $NR^{12}$ ersetzt ist, mit $R^{12}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

oder $R^1$ und $R^2$ zusammen -CH=CH-CH=CH- bilden, wobei das entstehende Naphthylsystem ein- oder mehrfach substituiert sein kann,

X ausgewählt ist aus

H, F, Cl, Br, I, $CF_3$, O-S($O_2$)-$C_6H_4$-p$CH_3$, $OR^{13}$ oder OC(O)$R^{13}$, wobei $R^{13}$ ausgewählt ist aus

H; $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert; $C_3$-$C_7$Cycloalkyl, gesättigt oder ungesättigt, einfach oder mehrfach substituiert oder unsubstituiert, bzw. einem entsprechenden Heterocyclus, bei dem ein C-Atom im Ring durch N, S oder O ersetzt ist; Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder,

wenn die Verbindung kein X aufweist, gemäß Formel Ia zwischen C-Atom A und C-Atom B oder C-Atom B und C-Atom C eine Doppelbindung ausgebildet ist;

und

$R^3$, $R^4$ unabhängig voneinander ausgewählt sind aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch N, S oder O ersetzt ist; Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder

$R^3$ und $R^4$ zusammen ein $C_3$-$C_7$-Cycloalkyl, gesättigt oder ungesättigt, einfach oder mehrfach substituiert oder unsubstituiert,bilden, bzw. einen entsprechenden Heterocyclus, bei dem ein C-Atom im Ring durch S, O oder $NR^{14}$ ersetzt ist, mit $R^{14}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

in Form seiner Diastereomere oder Enantiomere sowie seiner freien Base oder eines mit einer physiologisch verträglichen Säure gebildeten Salzes, insbesondere des Hydrochloridsalzes, zur Herstellung eines Arzneimittels zur Behandlung von Schmerz ist.

Überraschenderweise hat es sich herausgestellt, daß die erfindungsgemäßen substituierten Aminomethyl-Phenyl-Cyclohexanderivate für weitere Indikationen, insbesondere zur Behandlung von Harninkontinenz, Juckreiz und/oder Diarrhoe aber auch in andereren indikationen sehr geeignet sind. Ein weiterer Gegenstand der Anmeldung ist daher die Verwendung mindestens eines substituierten Aminomethyl-Phenyl-Cyclohexanderivats der allgemeinen Formel I bzw. Ia

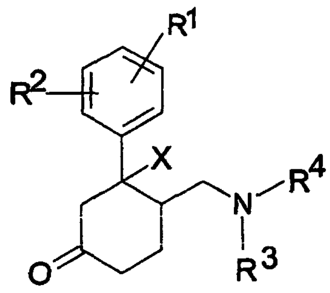

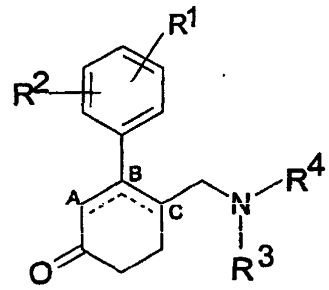

**I** **Ia**

, worin

$R^1$ und $R^2$ unabhängig voneinander ausgewählt sind aus $R^5$ oder $YR^5$ mit Y = $C_1$-$C_{10}$-Alkyl, $C_2$-$C_{10}$-Alkenyl oder $C_2$-$C_{10}$-Alkinyl, verzweigt oder unverzweigt, einfach oder mehrfach substituiert oder unsubstituiert, wobei $R^5$ ausgewählt ist aus

H, F, Cl, Br, I, CN, $NO_2$; $C_1$-$C_8$-Alkyl, $C_2$-$C_8$-Alkenyl oder $C_2$-$C_8$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^6$ ersetzt ist, mit $R^6$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

Aryl oder Heteroaryl, jeweils einfach oder mehrfach substituiert oder unsubstituiert;

$OR^7$, $OC(O)R^7$, $OC(O)OR^7$, $OC(S)R^7$, $C(O)R^7$, $C(O)OR^7$, $C(S)R^7$, $C(S)OR^7$, $SR^7$, $S(O)R^7$ bzw. $S(O_2)R^7$, wobei $R^7$ ausgewählt ist aus

H, $C_1$-$C_{18}$-Alkyl, $C_2$-$C_{18}$-Alkenyl oder $C_2$-$C_{18}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^8$ ersetzt ist, mit $R^8$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

Alkylaryl, gesättigt oder ungesättigt, einfach oder mehrfach substituiert oder unsubstituiert; Aryl oder Heteroaryl, jeweils einfach oder mehrfach substituiert oder unsubstituiert; oder

$NR^9R^{10}$, $C(O)NR^9R^{10}$ oder $S(O_2)NR^9R^{10}$, wobei $R^9$ und $R^{10}$ unabhängig voneinander ausgewählt sind aus

H, $C_1$-$C_{18}$-Alkyl, $C_2$-$C_{18}$-Alkenyl oder $C_2$-$C_{18}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^{11}$ ersetzt ist, mit $R^{11}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder

$R^9$ und $R^{10}$ zusammen ein $C_3$-$C_7$-Cycloalkyl, gesättigt oder ungesättigt, einfach oder mehrfach substituiert oder unsubstituiert, bilden, bzw. einen entsprechenden Heterocyclus, bei dem ein C-Atom im Ring durch S, O oder $NR^{12}$ ersetzt ist, mit $R^{12}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

oder

$R^1$ und $R^2$ zusammen -CH=CH-CH=CH- bilden, wobei das entstehende Naphthylsystem ein- oder mehrfach substituiert sein kann,

X ausgewählt ist aus

H, F, Cl, Br, I, $CF_3$, O-$S(O_2)$-$C_6H_4$-p$CH_3$, $OR^{13}$ oder $OC(O)R^{13}$, wobei $R^{13}$ ausgewählt ist aus

H; $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch N, S oder O ersetzt ist; Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder,

wenn die Verbindung kein X aufweist, gemäß Formel Ia zwischen C-Atom A und C-Atom B oder C-Atom B und C-Atom C eine Doppelbindung ausgebildet ist;

und

$R^3$, $R^4$ unabhängig voneinander ausgewählt sind aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch N, S oder O ersetzt ist; Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder

$R^3$ und $R^4$ zusammen ein $C_3$-$C_7$-Cycloalkyl, gesättigt oder ungesättigt, einfach oder mehrfach substituiert oder unsubstituiert,bilden, bzw. einen entsprechenden Heterocyclus, bei dem ein C-Atom im Ring durch S, O oder $NR^{14}$ ersetzt ist, mit $R^{14}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

in Form seiner Diastereomere oder Enantiomere sowie seiner freien Base oder eines mit einer physiologisch verträglichen Säure gebildeten Salzes, insbesondere des Hydrochloridsalzes, zur Herstellung eines Arzneimittels zur Behandlung von inflammatorischen und allergischen Reaktionen, Depressionen, Drogen- und/oder Alkoholmißbrauch, Gastritis, cardiovaskulären Erkrankungen, Atemwegserkrankungen, Husten, seelischen Erkrankungen und/oder Epilepsie sowie insbesondere von Haminkontinenz, Juckreiz und/oder Diarrhoe.

Bevorzugt ist dabei die Verwendung mindestens eines Aminomethyl-Phenyl-Cyclohexanonderivats gemäß Formel I bzw. Ia

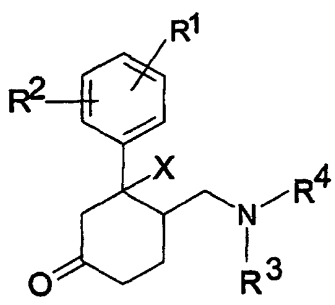

**I**

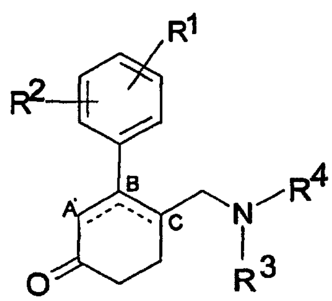

**Ia**

, in denen unabhängig voneinander

$R^1 = R^5$ ist, wobei $R^5$ ausgewählt ist aus

H, F, Cl, Br, I, $CHF_2$, $CF_3$, $NO_2$, $NH_2$; $C_1$-$C_4$-Alkyl oder $C_2$-$C_4$-Alkenyl, verzweigt oder unverzweigt, einfach oder mehrfach substituiert oder unsubstituiert; Aryl substituiert oder unsubstituiert; $OR^7$, $C(O)OR^7$ bzw. $SR^7$ wobei $R^7$ ausgewählt ist aus

H; $C_1$-$C_4$-Alkyl, verzweigt oder unverzweigt, einfach oder mehrfach substituiert oder unsubstituiert; vorzugsweise H, $CF_3$ oder $CH_3$,

oder $S(O_2)NR^9R^{10}$, wobei $R^9$ und $R^{10}$ unabhängig voneinander ausgewählt sind aus

H; $C_1$-$C_4$-Alkyl, verzweigt oder unverzweigt, einfach oder mehrfach substituiert oder unsubstituiert;

, wobei insbesondere bevorzugt ist, daß R1 = R5 mit R5 ausgewählt aus

H, F, Cl, OH, $CH_3$, $C_2H_5$, $C_2H_3$, $CHF_2$, $CF_3$, $SCH_3$, $OCF_3$, $OCH_3$, $OC_2H_5$, $C(O)OCH_3$, $C(O)OC_2H_5$ oder Phenyl,

**und/oder** $R^2 = R^5$ ist, wobei $R^5$ ausgewählt ist aus

H, F, Cl, Br, I, $SCH_3$; $C_1$-$C_4$-Alkyl, $C_2$-$C_4$-Alkenyl verzweigt oder unverzweigt, einfach oder mehrfach substituiert oder unsubstituiert, vorzugsweise $CF_3$; $OR^7$, mit $R^7$ ausgewählt aus $C_1$-$C_4$-Alkyl, verzweigt oder unverzweigt, einfach oder mehrfach substituiert oder unsubstituiert, vorzugsweise $CH_3$;

**und/oder** $R^1$ und $R^2$ unterschiedliche Bedeutungen haben, oder

$R^1$ und $R^2$ zusammen -CH=CH-CH=CH- bilden, wobei das entstehende Naphthylsystem ein- oder mehrfach, vorzugsweise mit Halogen, $OC_{1-3}$-Alkyl oder $C_{1-3}$-Alkyl, unsubstituiert oder ein oder mehrfach substituiert, insbesondere mit $OCH_3$; substituiert, sein kann,

**und/oder** X ausgewählt ist aus

H, F, Cl, OH, $CF_3$, O-$S(O_2)$-$C_6H_4$-$pCH_3$ bzw. $OC(O)R^7$ mit $R^7$ = H; $C_1$-$C_4$-Alkyl oder $C_2$-$C_4$-Alkenyl, verzweigt oder unverzweigt, einoder mehrfach substituiert oder unsubstituiert,

vorzugsweise H, F, Cl, OH, O-$S(O_2)$-$C_6H_4$-$pCH_3$, $OC(O)R^7$ mit $R^7$ = $C_1$-$C_4$-Alkyl, vorzugsweise $CH_3$, insbesondere H oder OH;

oder

, wenn die Verbindung kein X aufweist, gemäß Formel Ia zwischen C-Atom A und C-Atom B oder C-Atom B und C-Atom C eine Doppelbindung ausgebildet ist

**und/oder** $R^3$ und $R^4$ unabhängig voneinander ausgewählt sind aus $C_1$-$C_4$-Alkyl, verzweigt oder unverzweigt, einfach oder mehrfach substituiert oder unsubstituiert, vorzugsweise $CH_3$;

oder

$R^3$ und $R^4$ zusammen ein $C_3$-$C_7$-Cycloalkyl, gesättigt oder ungesättigt, einfach oder mehrfach substituiert oder unsubstituiert, bilden,

**und/oder** $R^1$, $R^2$, X, $R^3$ und/oder $R^4$ eine der in den vorangehenden Ansprüchen genannten Bedeutungen haben.

Besonders bevorzugt ist die Verwendung mindestens eines Aminomethyl-Phenyl-Cyclohexanonderivats ausgewählt aus der Gruppe

- *rac-cis*-[4-Dimethylaminomethyl-3-hydroxy-3-(3-methoxy-phenyl)-cyclohexanon],
- *rac-cis*-[4-Dimethylaminomethyl-3-(3-methoxy-phenyl)-cyclohexanon],
- *rac-trans*-[4-Dimethylaminomethyl-3-(3-methoxy-phenyl)-cyclohexanon],
- *rac-cis*-[4-Dimethylaminomethyl-3-(3-hydroxy-phenyl)-cyclohexanon,
- *rac-trans*-[4-Dimethylaminomethyl-3-(3-hydroxy-phenyl)-cyclohexanon oder
- 4-Dimethylaminomethyl-3-(3-methoxy-phenyl)-cyclohex-2-enon
- 4-Dimethylaminomethyl-3-phenyl-cyclohex-2-enon; e
- 4-Dimethylaminomethyl-3-(3-hydroxy-phenyl)-cyclohex-2-enon;
- rac-trans-4-Dimethylaminomethyl-3-phenyl-cyclohexanon;
- rac-cis-4-Dimethylaminomethyl-3-phenyl-cyclohexanon;
- 4-Dimethylaminomethyl-3-(4-methoxy-phenyl)-cyclohex-2-enon;
- 4-Dimethylaminomethyl-3-naphthalen-2-yl-cyclohex-2-enon;
- *rac-trans*-4-Dimethylaminomethyl-3-hydroxy-3-naphthalen-2-ylcyclohexanon;
- rac-trans-4-Dimethylaminomethyl-3-(3-fluoro-phenyl)-cyclohexanon;
- rac-cis-4-Dimethylaminomethyl-3-(3-fluoro-phenyl)-cyclohexanon;
- rac-cis-4-Dimethylaminomethyl-3-hydroxy-3-(2-methoxy-phenyl)-cyclohexanon
- rac-cis-4-Dimethylaminomethyl-3-hydroxy-3-(6-methoxy-naphthalen-2-yl)-cyclohexanon;
- 4-Dimethylaminomethyl-3-(6-methoxy-naphthalen-2-yl)-cyclohex-2-enon;
- rac-cis-3-Biphenyl-4-yl-4-dimethylaminomethyl-3-hydroxy-cyclohexanon
- 3-(3-Difluoromethyl-phenyl)-4-dimethylaminomethyl-cyclohex-2-enon;
- 4-Dimethylaminomethyl-3-(3-fluoro-phenyl)-cyclohex-2-enon;
- 3-(3,4-Difluoro-phenyl)-4-dimethylaminomethyl-cyclohex-2-enon;
- 4-Dimethylaminomethyl-3-(4-fluoro-phenyl)-cyclohex-2-enon;
- 4-Dimethylaminomethyl-3-(4-trifluoromethyl-phenyl)-cyclohex-2-enon;
- 3-(3,5-Dichloro-phenyl)-4-dimethylaminomethyl-cyclohex-2-enon;
- 3-(3,5-Difluoro-phenyl)-4-dimethylaminomethyl-cyclohex-2-enon;
- rac-cis-3-(3,5-Difluoro-phenyl)-4-dimethylaminomethyl-3-hydroxycyclohexanon

als freie Base oder in Form eines mit einer physiologisch verträglichen Säure gebildeten Salzes, insbesondere des Hydrochloridsalzes.

Im folgenden wird die Erfindung weiter durch Beispiele erläutert, ohne sie darauf zu beschränken.

**Beispiele**

Die folgenden Beispiele zeigen erfindungsgemäße Verbindungen sowie deren Darstellung und mit diesen durchgeführte Wirksamkeitsuntersuchungen.

Dabei gelten generell folgende Angaben:

Die Ausbeuten der hergestellten Verbindungen sind nicht optimiert.

Alle Temperaturen sind unkorrigiert.

Als stationäre Phase für die Säulenchromatographie wurde Kieselgel 60 (0.040 - 0.063 mm) der Firma E. Merck, Darmstadt, eingesetzt.

Die dünnschichtchromatographischen Untersuchungen wurden mit HPTLC-Fertigplatten, Kieselgel 60 F 254, der Firma E. Merck, Darmstadt, durchgeführt.

Die Mischungsverhältnisse der Laufmittel für alle chromatographischen Untersuchungen sind stets in Volumen/Volumen angegeben.

Die Angabe Ether bedeutet Diethylether.

Soweit nicht anders angegeben, wurde Petrolether mit dem Siedebereich von 50°C-70°C benutzt.

**Beispiel 1**

*rac-cis*-[4-Dimethylaminomethyl-3-hydroxy-3-(3-methoxy-phenyl)-cyclohexanon] (Verbindung 1) und 4-Dimethylaminomethyl-3-(3-methoxy-phenyl)-cyclohex-2-enon (Verbindung 6)

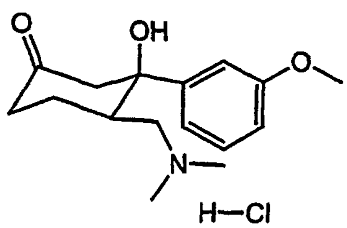

**(1)**

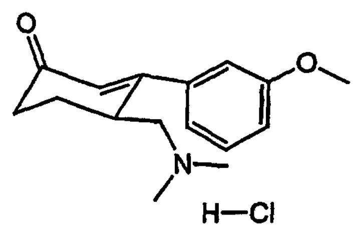

**(6)**

**Stufe 1:**

3,3-Dimethyl-1,5-dioxa-spiro[5.5]undecan-8-on

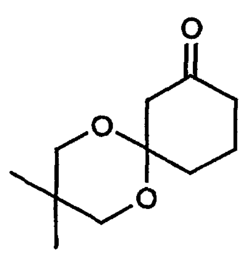

448,5 g 1,3-Cyclohexandion, 416 g 2,2-Dimethyl-1,3-propandiol, und 12 g p-Toluolsulfonsäure wurden in 3000 ml Dichlormethan gegeben und 24 Stunden am Wasserabscheider unter Rückfluß erhitzt. Nach Beendigung der Reaktion wurde die Mischung auf Raumtemperatur abgekühlt und mit 2000 ml 32%iger Natriumhydroxid-Lösung versetzt. Die Phasen wurden getrennt und die organische Phase wurde zunächst mit Natriumhydrogencarbonat-Lösung (44,4 g Natriumhydrogencarbonat-Lösung/ 850 ml Wasser) und dann mit 350 ml Wasser gewaschen. Die organische Phase

wurde Ober Natriumsulfat . getrocknet und anschließend wurde das Lösemittel im Vakuum evaporiert. Man erhielt so 345 g (50 % d.Th.) 3,3-Dimethyl-1,5-dioxaspiro[5.5]undecan-8-on.

**Stufe 2:**

9-Dimethylaminomethyl-1,5-dioxa-spiro[5.5]undecan-8-on

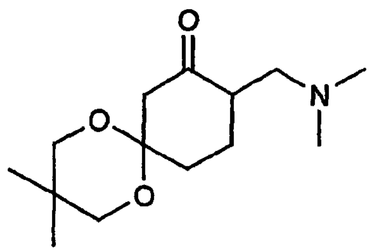

345 g 3,3-Dimethyl-1,5-dioxa-spiro[5.5]undecan-8-on wurden in 1900 ml Acetonitril p.a. gelöst. Die Mischung wurde auf 0°C abgekühlt und anschließend mit 140 g N,N-Dimethylmethylenimmoniumchlorid sowie einem Tropfen Acetylchlorid versetzt. Man ließ 6 Stunden bei 0°C rühren. Das Reaktionsgemisch wurde Ober Nacht bei Raumtemperatur belassen. Der ausgefallene weiße Feststoff wurde abgesaugt, mit Aceton gewaschen und im Vakuum getrocknet. Man erhielt so 310 g 9-Dimethylaminomethyl-1,5-dioxa-splro[5.5]undecan-8-on Hydrochlorid als weißen Feststoff. Zur Freisetzung der Base wurde der Feststoff in Wasser gelöst und mit einer äquimolaren Menge gesättigter Natriumcarbonat-Lösung versetzt. Man ließ eine Stunde rühren. Die Mischung wurde 3 Mal mit 500 ml Essigsäureethylester extrahiert. Die vereinten organischen Phasen wurden über Natriumsulfat getrocknet und das Lösemittel im Vakuum evaporiert. Man erhielt so 300 g (68% d.Th.) 9-Dimethylaminomethyl-1,5-dioxa-spiro[5.6]undecan-8-on.

**Stufe 3:**

9-Dimethylaminomethyl-8-(3-methoxy-phenyl)-3,3-dimethyl1,5-dioxaspiro[5.5]undecan-8-ol

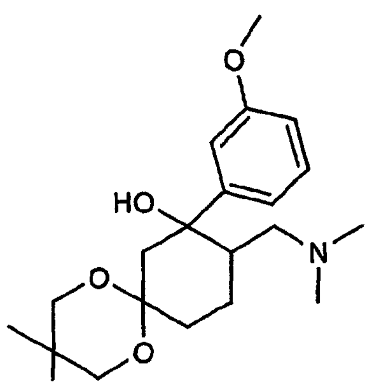

36 g Magnesium wurden in 150 ml Tetrahydrofuran p.a. vorgelegt. Anschließend wurden 280.5 g 3-Bromanisol in 300 ml Tetrahydrofuran p.a. zugetropft. Man ließ 1 Stunde nachreagieren und kühlte dann die Reaktionsmischung auf 20°C ab. Bei dieser Temperatur wurden 290 g 9-Dimethylaminomethyl-1,5-dioxa-spiro[5.5]undecan-8-on in 700 ml Tetrahydrofuran p.a. zugetropft. Man ließ über Nacht bei Raumtemperatur nachreagieren. Anschließend wurden bei einer Temperatur von 10 - 15°C zunächst 200 ml einer 20%igen Ammoniumchlorid, dann 300 ml Wasser und dann 100 ml einer 32%igen Salzsäure-Lösung zugetropft. Die Phasen wurden getrennt und die wäßrige Phase wurde 2 Mal mit je 400 ml Essigsäureethylester extrahiert. Die organische Phase wurde über Magnesiumsulfat getrocknet und

anschließend das Lösemittel im Vakuum evaporiert. Die so erhaltene Rohbase wurde mit 1000 ml Petrolether versetzt . Die Titelverbindung begann nach kurzer Zeit auszukristallisieren. Zur Komplettierung der Kristallisation wurde mit 2100 ml Diisopropylether versetzt und 2 Stunden bei 5°C nachgerührt. Der Feststoff wurde abgesaugt, mit Diisopropylether gewaschen und anschließend im Vakuum getrocknet.. Man erhielt so 156 g (41.2% d.Th.) in Form eines weißen Feststoffs.

**Stufe 4**

*rac-cis*-[4-Dimethylaminomethyl-3-hydroxy-3-(3-methoxy-phenyl)-cyclohexanon] Hydrochlorid(1) und 4-Dimethylaminomethyl-3-(3-methoxy-phenyl)-cyclohex-2-enon Hydrochlorid (6)

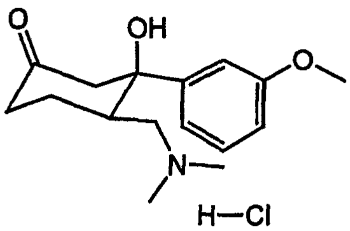

(1)

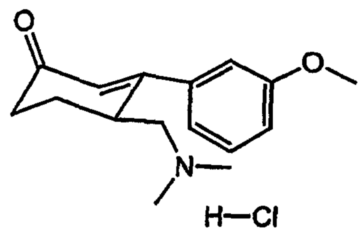

(6)

a) 4-Dimethylaminomethyl-3-(3-methoxy-phenyl)-cyclohex-2-enon Hydrochlorid (Verbindung 6)

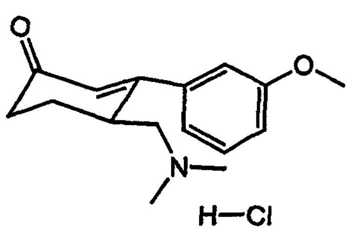

182 g 9-Dimethylaminomethyl-8-(3-methoxy-phenyl)-3,3-dimethyl1,5-dioxa-spiro[5.5]undecan-8-ol wurden in 1200 ml Tetrahydrofuran gelöst und die Reaktionsmischung wurde auf 5°C abgekühlt. Bei dieser Temperatur wurde eine Mischung aus 600 ml 32% ige Salzsäure-Lösung und 600 ml Wasser zugesetzt. Es wurde 72 Stunden bei Raumtemperatur gerührt. Nach Beendigung der Reaktion wurden bei 5°C 500 ml Essigsäureethylester zugegeben und dann die Reaktionsmischung mit 32%iger Natriumhydroxid-Lösung auf pH 12 eingestellt. Die Phasen wurden getrennt und die wäßrige Phase wurde drei Mal mit je 300 ml Essigester gewaschen. Die vereinten organischen Phasen wurden anschließend zwei Mal mit je 100 ml gesättigter Natriumchlorid-Lösung gewaschen, über Magnesiumsulfat getrocknet und anschließend vom Lösemittel befreit. Die Reinigung erfolgte über die Hydrochloridbildung. Dazu wurde 4-Dimethylaminomethyl-3-(3-methoxy-phenyl)-cyclohex-2-enon Base in 500 ml Aceton gelöst und mit einer äquimolaren Menge Wasser und Trimethylchlorsilan versetzt. Das ausgefallene Hydrochlorid wurde abgesaugt und im Vakuum getrocknet . Man erhielt so 63.6 g (78.9% d.Th.) 4-Dimethylaminomethyl-3-(3-methoxy-phenyl)-cyclohex-2-enon Hydrochlorid.

b) *rac-cis*-[4-Dimethylaminomethyl-3-hydroxy-3-(3-methoxy-phenyl)cyclohexanon] Hydrochlorid (Verbindung 1)

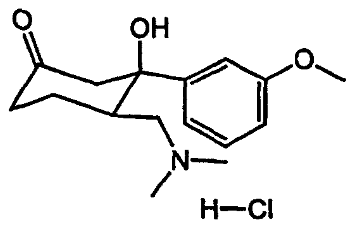

150 g 9-Dimethylaminomethyl-8-(3-methoxy-phenyl)-3,3-dimethyl1,5-dioxa-spiro[5.5]undecan-8-ol wurden in Tetrahydrofuran gelöst und auf - 10°C abcekühlt. Anschließend wurde 1000 ml eines Gemisches aus Wasser und 32%iger Salzsäure (5 : 1) zugetropft. Der Ansatz wurde über Nacht bei 5°C belassen. Anschließend wurden 750 ml Diethylether zugegeben und dann wurde mit 200 ml 32%iger Natriumhydroxid-Lösung auf pH 12 eingestellt. Die Phasen wurden getrennt und die wäßrige Phase wurde zwei Mal mit je 200 ml Diethylether extrahiert. Die vereinten oragnischen Phasen wurden mit 200 ml gesättigter Natriumchlorid-Lösung gewaschen, über Natriumsulfat getrocknet und anschließend das Lösemittel im Vakuum evaporiert. *Rac-cis*-[4-Dimethylaminomethyl-3-hydroxy-3-(3-methoxy-phenyl)-cyclohexanon

Base wurde in Aceton gelöst und mit einer äquimolaren Menge Wasser und Trimethylchlorsilan versetzt. Das ausgefallene Hydrochlorid wurde abgesaugt und im Vakuum getrocknet. Man erhielt so 67g (59% d.Th.) *rac-cis*-[4-Dimethylaminomethyl-3-hydroxy-3-(3-methoxy-phenyl)-cyclohexanon] Hydrochlorid.

**Beispiel 2**

*rac-cis*-[4-Dimethylaminomethyl-3-(3-methoxy-phenyl)-cyclohexanon] Hydrochlorid (Verbindung 2) und *rac-trans*-[4-Dimethylaminomethyl-3-(3-methoxy-phenyl)-cyclohexanon] Hydrochlorid( Verbindung 3)

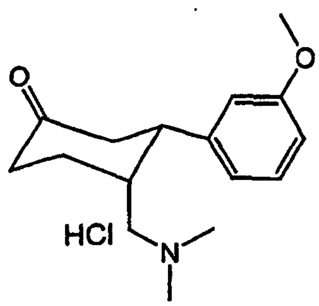

(2)

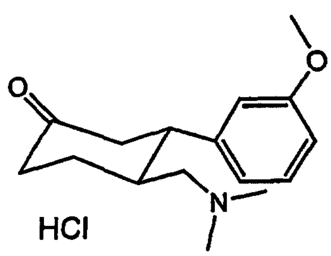

(3)

28.5 g 4-Dimethylaminomethyl-3-(3-methoxy-phenyl)-cyclohex-2-enon, das gemäß Beispiel 1 Stufe 4a) hergestellt wurde, wurden in 250 ml absolutem Methanol gelöst. Unter Rühren und Überleiten von trockenem Stickstoff wurden 2.8 g Palladium-Kohle (10 %ig) als Katalysator zugesetzt. Anschließend wurde fünf Stunden bei einem Druck von 0.2 bar und einer Temperatur von 20°C hydriert. Nach Filtration wurde das Lösemittel im Vakuum abgedampft und der Rückstand säulenchromatographisch an Kieselgel mit Essigsäureethylester/Methanol/Diisopropylether = 4/1/5 als Elutionsmittel gereinigt. Als erste Produktfraktion erhielt man 7.5 g *rac*cis-[4-Dimethylaminomethyl-3-(3-methoxy-phenyl)]-cyclohexanon in Form eines Öls. Zur Darstellung des Hydrochlorids wurde die Base in Aceton gelöst und mit einer äquimolaren Menge Wasser und Trimethylchlorsilan versetzt. Das ausgefallene Hydrochlorid wurde abgesaugt und im Vakuum getrocknet. Man erhielt so 7.2 g (25,4 % d. Th.) *rac-cis*-[4-Dimethylaminomethyl-3-(3-methoxy-phenyl)]-cyclohexanon Hydrochlorid. Die zweite Produktfraktion lieferte 7,8g *rac*trans-[4-Dimethylaminomethyl-3-(3-methoxy-phenyl)]-cyclohexanon gleichfalls als Öl. Zur Darstellung des Hydrochlorids wurde die Base in Aceton gelöst und mit einer äquimolaren Menge Wasser und Trimethylchlorsilan versetzt. Das ausgefallene Hydrochlorid wurde abgesaugt und im Vakuum getrocknet. Man erhielt so 7.4 g (26,1 % d. Th.) *rac-trans*-[4-Dimethylaminomethyl-3-(3-methoxy-phenyl)]-cyclohexanon Hydrochlorid.

**Beispiel 3**

*rac-cis*-[4-Dimethylaminomethyl-3-(3-hydroxy-phenyl)-cyclohexanon (Verbindung 4) und *rac-trans*-[4-Dimethylaminomethyl-3-(3-hydroxyphenyl)-cyclohexanon (Verbindung 5)

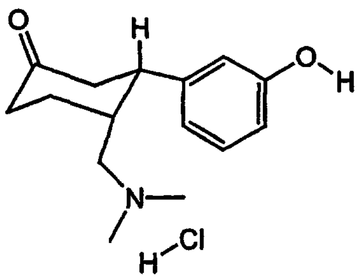

(4)

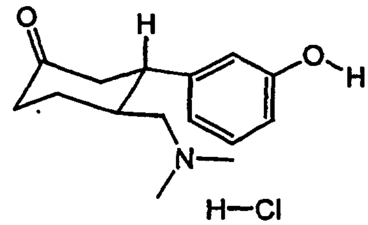

(5)

**Stufe 1**

8-[3-(tert.Butyl-diphenyl-silanyloxy)-phenyl]-9-dimethylaminomethyl-3,3-dimethyl-1,5-dioxa-spiro-[5.5]undecan-8-ol

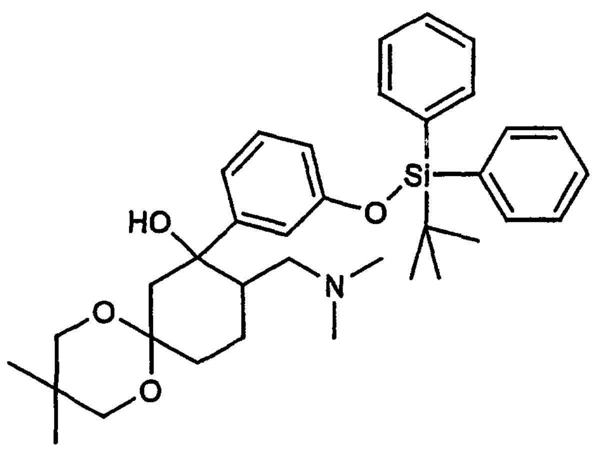

17,3 g Magnesiumspäne wurden in Tetrahydrofuran vorgelegt. Anschließend wurden 293 g (3-Bromo-phenoxy)-tert-butyl-diphenylsilan in 150 ml Tetrahydrofuran zugetropft (unter leichtem Rückfluß). Man ließ 1 Stunde nachreagieren. Anschließend wurden bei 20°C 142 g 9-Dimethylaminomethyl-1,5-dioxa-spiro[5.5]undecan-8-on, das gemäß Beispiel 1, Stufe 2 hergestellt, zugetropft. Es wurde 10 Stunden bei Raumtemperatur gerührt. Anschließend wurde bei 10 - 15°C mit 200 ml 20%ige Ammoniumchlorid-Lösung und dann mit 500 ml Wasser hydrolysiert. Die wäßrige

Phase wurde zwei Mal mit je 300 ml Essigsäureethylester extrahiert. Die vereinten organischen Phasen wurden über Magnesiumsulfat getrocknet und anschließend wurde das Lösemitel im Vakuum evaporiert. Der Rückstand wurde an Kieselgel mit Essigester/Methanol = 9/1 gereinigt. Man erhielt so 197 g (60% d.Th.) der Titelverbindung.

**Stufe 2**

3-[3-(tert.Butyl-diphenyl-silanyloxy)-phenyl]-4-dimethylaminomethylcyclohex-2-enon

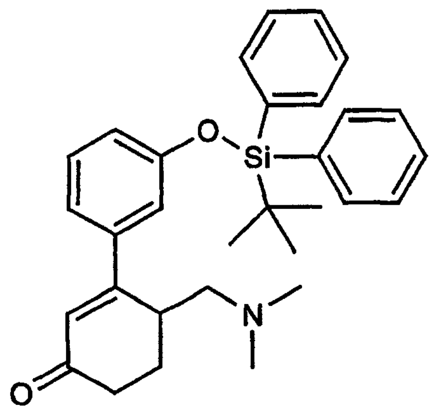

197 g 8-[3-(tert.Butyl-diphenyl-silanyloxy)-phenyl]-9-dimethylaminomethyl-3,3-dimethyl-1,5-dioxa-spiro-[5.5] undecan-8-ol wurden in Tetrahydrofuran gelöst und auf 5°C gekühlt. Bei dieser Temperatur wurde eine Mischung aus 300 ml konzentrierter Salzsäure und 300 ml Wasser zugetropft. Anschließend wurde 10 Stunden bei Raumtemperatur gerührt.. Dann wurden unter Eiskühlung 400 ml Essigsäureethylester zugegeben und dann wurde mit 32%iger Natriumhydroxid-Lösung auf pH 12 eingestellt. Die Phasen wurden getrennt und die wäßrige Phase wurde drei Mal mit je 400 ml Essigsäureethylester extrahiert. Die vereinten organischen Phasen wurden zwei Mal mit je 100 ml gesättigter Natriunchlorid-Lösung gewaschen, über Magnesiumsulfat getrocknet und anschließend wurde im Vakuum das Lösemittel evaporiert. Der Rückstand wurde an Kieselgel mit Essigester/Methanol = 1/1 gereinigt. Man erhielt so 68 g (42% d.Th.) der Titelverbindung.

**Stufe 3**

*rac-cis*-[4-Dimethylaminomethyl-3-(3-hydroxy-phenyl)-cyclohexanon (4) *und rac-trans*-[4-Dimethylaminomethyl-3-(3-hydroxy-phenyl)-cyclohexanon (5)

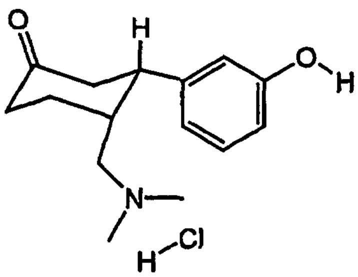

(4)

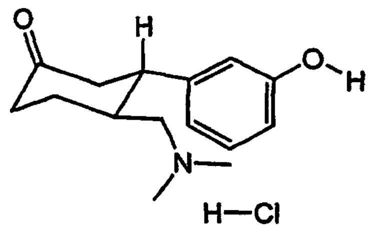

(5)

25 g 3-[3-(tert.Butyl-diphenyl-silanyloxy)-phenyl]-4-dimethylaminomethyl-cyclohex-2-enon wurden in 110 ml absolutem Methanol gelöst. Unter Rühren und Überleiten von trockenem Stickstoff wurden 2.8 g Palladium-Kohle (10 %ig) als Katalysator zugesetzt. Anschließend wurde fünf Stunden bei einem Druck von 0.2 bar und einer Temperatur von 20°C hydriert. Nach Filtration wurde das Lösemittel im Vakuum abgedampft und der Rückstand in 60 ml Tetrahydrofuran gelöst. Anschließend wurden 15 ml Tetrabutylammoniumfluorid bei Raumtemperatur zugegeben. Nach Beendigung der Reaktion wurde der Ansatz mit Wasser gequenscht und anschließend mit Esssigsäureethylester extrahiert. Die organische Phase wurde mit gesättigter Natriumchlorid-Lösung gewaschen. Anschließend wurde die organische Phase über Magnesiumsulfat getrocknet und das Lösemittel wurde im Vakuum evaporiert. Der Rückstand wurde säulenchromatographisch an Kieselgel mit Essigsäureethylester/Methanol/Diisopropylether = 4/1/5 als Elutionsmittel gereinigt. Als erste Produktfraktion erhielt man 5 g *rac-cis*-[4-Dimethylaminomethyl-3-(3-hydroxy-phenyl)-cyclohexanon in Form eines Öls. Zur Darstellung des Hydrochlorids wurde die Base in Aceton gelöst und mit einer äquimolaren Menge Wasser und Trimethylchlorsilan versetzt. Das ausgefallene Hydrochlorid wurde abgesaugt und im Vakuum getrocknet. Man erhielt so 4.8 g (32,9 % d. Th.) *rac-cis*-[4-Dimethylaminomethyl-3-(3-hydroxy-phenyl)-cyclohexanon Hydrochlorid. Die zweite Produktfraktion lieferte 3.5 g *ractrans*-[4-Dimethylaminomethyl-3-(3-hydroxy-phenyl)-cyclohexanon gleichfalls als Öl. Zur Darstellung des Hydrochlorids wurde die Base in Aceton gelöst und mit einer äquimolaren Menge Wasser und Trimethylchlorsilan versetzt. Das ausgefallene Hydrochlorid wurde abgesaugt und im Vakuum getrocknet. Man erhielt so 3.2 g (21.9% d. Th.) *rac-trans*-[4-Dimethylaminomethyl-3-(3-hydroxy-phenyl)-cyclohexanon Hydrochlorid.

**Beispiel 4**

Die folgenden Beispiele wurden gemäß den angegebenen Vorschriften synthetisiert und die Strukturen sind durch NMR-Untersuchungen belegt.

- 4-Dimethylaminomethyl-3-phenyl-cyclohex-2-enon; e
- 4-Dimethylaminomethyl-3-(3-hydroxy-phenyl)-cyclohex-2-enon;
- rac-trans-4-Dimethylaminomethyl-3-phenyl-cyclohexanon;
- rac-cis-4-Dimethylaminomethyl-3-phenyl-cyclohexanon;
- 4-Dimethylaminomethyl-3-(4-methoxy-phenyl)-cyclohex-2-enon;
- 4-Dimethylaminomethyl-3-naphthalen-2-yl-cyclohex-2-enon;

- rac-trans-4-Dimethylaminomethyl-3-hydroxy-3-naphthalen-2-ylcyclohexanon;
- rac-trans-4-Dimethylaminomethyl-3-(3-fluoro-phenyl)-cyclohexanon;
- rac-cis-4-Dimethylaminomethyl-3-(3-fluoro-phenyl)-cyclohexanon;
- rac-cis-4-Dimethylaminomethyl-3-hydroxy-3-(2-methoxy-phenyl)-cyclohexanon
- rac-cis-4-Dimethylaminomethyl-3-hydroxy-3-(6-methoxy-naphthalen-2-yl)-cyclohexanon;
- 4-Dimethylaminomethyl-3-(6-methoxy-naphthalen-2-yl)-cyclohex-2-enon;
- rac-cis-3-Biphenyl-4-yl-4-dimethylaminomethyl-3-hydroxy-cyclohexanon
- 3-(3-Difluoromethyl-phenyl)-4-dimethylaminomethyl-cyclohex-2-enon;
- 4-Dimethylaminomethyl-3-(3-fluoro-phenyl)-cyclohex-2-enon;
- 3-(3,4-Difluoro-phenyl)-4-dimethylaminomethyl-cyclohex-2-enon;
- 4-Dimethylaminomethyl-3-(4-fluoro-phenyl)-cyclohex-2-enon;
- 4-Dimethylaminomethyl-3-(4-trifluoromethyl-phenyl)-cyclohex-2-enon;
- 3-(3,5-Dichloro-phenyl)-4-dimethylaminomethyl-cyclohex-2-enon;
- 3-(3,5-Difluoro-phenyl)-4-dimethylaminomethyl-cyclohex-2-enon;
- rac-cis-3-(3,5-Difluoro-phenyl)-4-dimethylaminomethyl-3-hydroxycyclohexanon

**Beispiel 5**

**Pharmakologische Untersuchungen**

**Writhing-Test**

Die antinociceptive Wirksamkeit der erfindungsgemäßen Verbindungen wurde im Phenylchinon-induzierten Writhing-Test, modifiziert nach I.C. Hendershot, J. Forsaith, J.Pharmacol. Exp. Ther. 125, 237 - 240 (1959) an der Maus untersucht. Dazu wurden männliche NMRI-Mäuse mit einem Gewicht von 25 - 30 g eingesetzt. Gruppen von 10 Tieren pro Substanzdosis wurden 10 Minuten nach intravenöser Gabe einer erfindungsgemäßen Verbindung 0.3 ml/ Maus einer 0,02 %igen wäßrigen Lösung von Phenylchinon (Phenylbenzochinon, Fa. Sigma, Deisenhofen; Herstellung der Lösung unter Zusatz von 5 % Ethanol und Aufbewahrung im Wasserbad bei 45 °C) intraperitoneal appliziert. Die Tiere wurden einzeln in Beobachtungskäfige gesetzt. Mittels eines Drucktastenzählers wurde die Anzahl der Schmerz-induzierten Streckbewegungen (sogenannte Writhingreaktionen = Durchdrücken des Körpers mit Abstrecken der Hinterextremitäten) 5 - 20 Minuten nach der Phenylchinon-Gabe ausgezählt. Als Kontrolle wurden Tiere mitgeführt, die physiologische Kochsalzlösung i.v. und Phenyylchinon i.v. erhielten.

Alle Substanzen wurden in der Standarddosis von 10 mg/kg getestet. Die prozentuale Hemmung (% Hemmung) der Writhingreaktionen durch eine Substanz wurde nach folgender Formel berechnet:

$$\%\text{Hemmung} = 100 - [\text{WR behandelte Tiere/WR Kontrolle} \times 100]$$

Alle untersuchten erfindungsgemäßen Verbindungen zeigten eine mittelstarke bis starke analgetische Wirkung.

Die Ergebnisse ausgewählter Wrihting-Untersuchungen sind in der Tabelle 1 zusammengefaßt

**Tabelle 1: Analgesieprüfung im Writhing-Test an der Maus**

| Verbindung | % Hemmung der Writhing-Reaktionen 10 mg/kg i.v. |
|---|---|
| 2 | 96 |
| 3 | 92 |
| 4 | 100 |

## Patentansprüche

1. Aminomethyl-Phenyl-Cyclohexanonderivate der allgemeinen Formel I bzw. Ia auch in Form ihrer Diastereomere oder Enantiomere sowie ihrer freien Base oder eines mit einer physiologisch verträglichen Säure gebildeten Salzes, insbesondere des Hydrochloridsalzes,

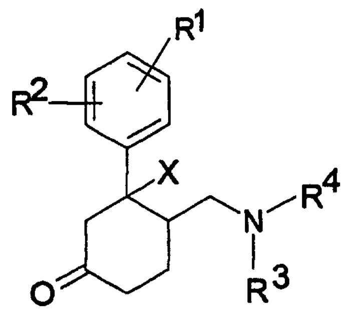

I

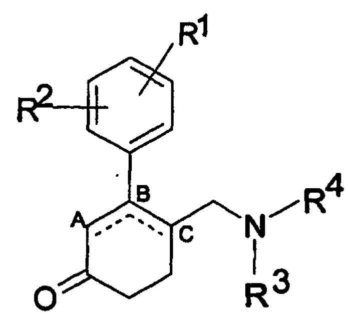

Ia

,worin
$R^1$ und $R^2$ unabhängig voneinander ausgewählt sind aus $R^5$ oder $YR^5$ mit Y = $C_1$-$C_{10}$-Alkyl, $C_2$-$C_{10}$-Alkenyl oder $C_2$-$C_{10}$-Alkinyl, verzweigt oder unverzweigt, einfach oder mehrfach substituiert oder unsubstituiert, wobei $R^5$ ausgewählt ist aus

H, F, Cl, Br, I, CN, $NO_2$; $C_1$-$C_8$-Alkyl, $C_2$-$C_8$-Alkenyl oder $C_2$-$C_8$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^6$ ersetzt ist, mit $R^6$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

Aryl oder Heteroaryl, jeweils einfach oder mehrfach substituiert oder unsubstituiert;

$OR^7$, $OC(O)R^7$, $OC(O)OR^7$, $OC(S)R^7$, $C(O)R^7$, $C(O)OR^7$, $C(S)R^7$, $C(S)OR^7$, $SR^7$, $S(O)R^7$ bzw. $S(O_2)R^7$, wobei $R^7$ ausgewählt ist aus

H, $C_1$-$C_{18}$-Alkyl, $C_2$-$C_{18}$-Alkenyl oder $C_2$-$C_{18}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^8$ ersetzt ist, mit $R^8$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

Alkylaryl, gesättigt oder ungesättigt, einfach oder mehrfach substituiert oder unsubstituiert; Aryl oder Heteroaryl, jeweils einfach oder mehrfach substituiert oder unsubstituiert; oder

$NR^9R^{10}$, $C(O)NR^9R^{10}$ oder $S(O_2)NR^9R^{10}$, wobei $R^9$ und $R^{10}$ unabhängig voneinander ausgewählt sind aus

H, $C_1$-$C_{18}$-Alkyl, $C_2$-$C_{18}$-Alkenyl oder $C_2$-$C_{18}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^{11}$ ersetzt ist, mit $R^{11}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder

$R^9$ und $R^{10}$ zusammen ein $C_3$-$C_7$-Cycloalkyl, gesättigt oder ungesättigt, einfach oder mehrfach substituiert oder unsubstituiert, bilden, bzw. einen entsprechenden Heterocyclus, bei dem ein C-Atom im Ring durch S, O oder $NR^{12}$ ersetzt ist, mit $R^{12}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

oder

$R^1$ und $R^2$ zusammen -CH=CH-CH=CH- bilden, wobei das entstehende Naphthylsystem ein- oder mehrfach substituiert sein kann,

X ausgewählt ist aus

H, F, Cl, Br, I, $CF_3$, O-$S(O_2)$-$C_6H_4$-$pCH_3$, $OR^{13}$ oder $OC(O)R^{13}$, wobei $R^{13}$ ausgewählt ist aus

H; $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch N, S oder O ersetzt ist; Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder,

wenn die Verbindung kein X aufweist, gemäß Formel Ia zwischen C-Atom A und C-Atom B oder C-Atom B und C-Atom C eine Doppelbindung ausgebildet ist;

und

$R^3$, $R^4$ unabhängig voneinander ausgewählt sind aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch N, S oder O ersetzt ist; Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder

$R^3$ und $R^4$ zusammen ein $C_3$-$C_7$-Cycloalkyl, gesättigt oder ungesättigt, einfach oder mehrfach substituiert oder unsubstituiert,bilden, bzw. einen entsprechenden Heterocyclus, bei dem ein C-Atom im Ring durch S, O oder $NR^{14}$ ersetzt ist, mit $R^{14}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

mit der Maßgabe, daß,

wenn X = OH, $R^2$ = H und $R^3$ und $R^4$ jeweils $CH_3$ sind, $R^1$ nicht gleichzeitig $R^5$ mit $R^5 = OR^7$ mit $R^7$ = H, $CH_3$ bzw. eine unsubstituierte oder substituierte Pyridyl-, Thienyl-, Thiazoyl- oder Phenylgruppe, oder $R^5 = OC(O)OR^7$ mit $R^7$ = $C_1$-$C_5$-Alkyl, oder $R^5 = OC(O)R^7$ mit $R^7$ = $C_{1-5}$-Alkyl, NH-$C_6H_4$-$C_{1-3}$-Alkyl, $C_6H_4$-$OC(O)C_{1-3}$-Alkyl, $C_6H_4$-$CH_2$-N($C_{1-4}$-Alkyl)$_2$, $C_6H_4$-$CH_2$-(N-4-Morpholino) bzw. CHZ'-NHZ" mit Z' und Z" gleich oder verschieden ausgewählt aus H oder $C_{1-6}$-Alkyl, sein kann,

oder,
wenn X = H oder gemäß Formel Ia zwischen C-Atom A und C-Atom B eine Doppelbindung ausgebildet ist, $R^2$ = H und $R^3$ und $R^4$ jeweils unabhängig voneinander $C_{1-6}$-Alkyl, Aryl oder $C_{3-7}$-Cycloalkyl sind, $R^1$ nicht gleichzeitig H, $C_{1-6}$-Alkoxy, O-$C_{3-7}$-Cycloalkyl, O-Aryl oder O-Heterocyclyl sein kann.

**2.** Aminomethyl-Phenyl-Cyclohexanonderivate nach Anspruch 1, **dadurch gekennzeichnet, daß** $R^1$ = $R^5$, wobei $R^5$ ausgewählt ist aus

H, F, Cl, Br, I, $CHF_2$, $CF_3$, $NO_2$, $NH_2$; $C_1$-$C_4$-Alkyl oder $C_2$-$C_4$-Alkenyl, verzweigt oder unverzweigt, einfach oder mehrfach substituiert oder unsubstituiert; Aryl substituiert oder unsubstituiert; $OR^7$, $C(O)OR^7$ bzw. $SR^7$ wobei $R^7$ ausgewählt ist aus

H; $C_1$-$C_4$-Alkyl, verzweigt oder unverzweigt, einfach oder mehrfach substituiert oder unsubstituiert; vorzugsweise H, $CF_3$ oder $CH_3$,

oder $S(O_2)NR^9R^{10}$, wobei $R^9$ und $R^{10}$ unabhängig voneinander ausgewählt sind aus

H; $C_1$-$C_4$-Alkyl, verzweigt oder unverzweigt, einfach oder mehrfach substituiert oder unsubstituiert;

, wobei insbesondere bevorzugt ist, daß R1 = R5 mit R5 ausgewählt aus

H, F, Cl, OH, $CH_3$, $C_2H_5$, $C_2H_3$, $CHF_2$, $CF_3$, $SCH_3$, $OCF_3$, $OCH_3$, $OC_2H_5$, $C(O)OCH_3$, $C(O)OC_2H_5$ oder Phenyl

während $R^2$, X, $R^3$ und $R^4$ eine der in den vorangehenden Ansprüchen genannten Bedeutungen haben.

**3.** Aminomethyl-Phenyl-Cyclohexanonderivate nach einem der Ansprüche 1 oder 2, **dadurch gekennzeichnet, daß** $R^2$ = $R^5$, wobei $R^5$ ausgewählt ist aus

H, F, Cl, Br, I, $SCH_3$; $C_1$-$C_4$-Alkyl, $C_2$-$C_4$-Alkenyl verzweigt oder unverzweigt, einfach oder mehrfach substituiert oder unsubstituiert, vorzugsweise $CF_3$; $OR^7$, mit $R^7$ ausgewählt aus $C_1$-$C_4$-Alkyl, verzweigt oder unverzweigt, einfach oder mehrfach substituiert oder unsubstituiert, vorzugsweise $CH_3$;

während $R^1$, X, $R^3$ und $R^4$ eine der in den vorangehenden Ansprüchen genannten Bedeutungen haben.

**4.** Aminomethyl-Phenyl-Cyclohexanonderivate nach einem der Ansprüche 1 bis 3, **dadurch gekennzeichnet, daß** $R^1$ und $R^2$ unterschiedliche Bedeutungen haben oder

$R^1$ und $R^2$ zusammen -CH=CH-CH=CH- bilden, wobei das entstehende Naphthylsystem ein- oder mehrfach, vorzugsweise mit Halogen, $OC_{1-3}$-Alkyl oder $C_{1-3}$-Alkyl, unsubstituiert oder ein oder mehrfach substituiert, insbesondere mit $OCH_3$; substituiert, sein kann.

**5.** Aminomethyl-Phenyl-Cyclohexanonderivate nach einem der Ansprüche 1 bis 4, **dadurch gekennzeichnet, daß** X ausgewählt ist aus

H, F, Cl, OH, $CF_3$, O-$S(O_2)$-$C_6H_4$-p$CH_3$ bzw. $OC(O)R^7$ mit $R^7$ = H; $C_1$-$C_4$-Alkyl oder $C_2$-$C_4$-Alkenyl, verzweigt oder unverzweigt, einoder mehrfach substituiert oder unsubstituiert,

vorzugsweise H, F, Cl, OH, O-$S(O_2)$-$C_6H_4$-p$CH_3$, $OC(O)R^7$ mit $R^7$ = $C_1$-$C_4$-Alkyl, vorzugsweise $CH_3$, insbesondere H oder OH,

oder

, wenn die Verbindung kein X aufweist, gemäß Formel la zwischen C-Atom A und C-Atom B oder C-Atom B und C-Atom C eine Doppelbindung ausgebildet ist

, während $R^1$, $R^2$, $R^3$ und $R^4$ eine der in den vorangehenden Ansprüchen genannten Bedeutungen haben.

**6.** Aminomethyl-Phenyl-Cyclohexanonderivate nach einem der Ansprüche 1 bis 5, **dadurch gekennzeichnet, daß** $R^3$ und $R^4$ unabhängig voneinander ausgewählt sind aus

$C_1$-$C_4$-Alkyl, verzweigt oder unverzweigt, einfach oder mehrfach substituiert oder unsubstituiert, vorzugsweise $CH_3$,

oder

$R^3$ und $R^4$ zusammen ein $C_3$-$C_7$-Cycloalkyl, gesättigt oder ungesättigt, einfach oder mehrfach substituiert oder unsubstituiert, bilden, , während $R^1$, $R^2$ und X eine der in den vorangehenden Ansprüchen genannten Bedeutungen haben.

**7.** Verfahren zur Herstellung von Aminomethyl-Phenyl-Cyclohexanonderivaten gemäß Anspruch 1 nach Formel Ia oder nach Formel I mit X = H, **dadurch gekennzeichnet, daß** Verbindungen der Formel II

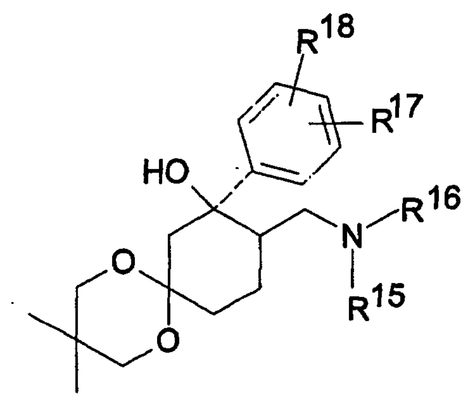

**II**

, in denen $R^{15}$ eine $R^3$, $R^{16}$ eine $R^4$, $R^{17}$ eine $R^1$ und $R^{18}$ eine $R^2$ entsprechende Bedeutung gemäß Anspruch 1 haben, mit Säuren, vorzugsweise Salzsäure, Ameisensäure oder Essigsäure, bei Raumtemperatur umgesetzt werden und anschließend das Produkt gemäß Formel Ia entweder aufgereinigt oder mit katalytisch aktiviertem Wasserstoff, vorzugsweise mit auf einem Trägermaterial wie Aktivkohle absorbiertem Platin oder Palladium als Katalysator, hydriert wird, vorzugsweise in Essigsäureethylester oder einem $C_1$ - $C_4$-Alkylalkohol, bei Drücken von 0,1 bis 10 bar und/oder Temperaturen von 20°C bis 80°C und dann das Produkt gemäß Formel I mit X = H aufgereinigt wird.

**8.** Verfahren zur Herstellung von Aminomethyl-Phenyl-Cyclohexanonderivaten gemäß Anspruch 1 nach Formel I mit X ≠ H, **dadurch gekennzeichnet, daß** Verbindungen der Formel II

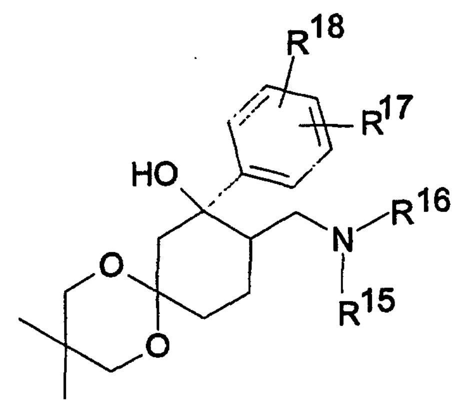

**II**

, in denen $R^{15}$ eine $R^3$, $R^{16}$ eine $R^4$, $R^{17}$ eine $R^1$ und $R^{18}$ eine $R^2$ entsprechende Bedeutung gemäß Anspruch 1 haben, mit Säuren, vorzugsweise Salzsäure, bei Temperaturen zwischen 0°C und 5°C umgesetzt und die so entstandenen Produkte gemäß Formel I mit X = OH und unveränderten $R^{15}$ bis $R^{18}$ entweder aufgereinigt oder weiterverarbeitet werden, wobei, wenn X gleich F, Cl, Br, I bzw. $CF_3$ sein soll, die X = OH-Gruppe gegen F bzw. Cl bzw. Br bzw. I bzw. $CF_3$ ausgetauscht wird, wenn X = $OR^{13}$ sein soll, wobei $R^{13}$ eine der in Anspruch 1 beschriebenen Bedeutungen hat, die X = OH-Gruppe mit einem Halogenid der Formel III

$$R^{19}Cl$$

**III**

verethert wird, wobei $R^{19}$ eine $R^{13}$ analoge Bedeutung hat, oder, wenn X = $O\text{-}S(O_2)\text{-}C_6H_4\text{-}CH_3$, oder $OC(O)R^{13}$,

wobei $R^{13}$ eine der in Anspruch 1 beschriebenen Bedeutungen hat, die X = OH-Gruppe mit einem Säurechlorid der Formel IV

$$R^{20}COCl$$

**IV**

oder $Cl\text{-}S(O_2)\text{-}C_6H_4\text{-}CH_3$ verestert wird, wobei $R^{20}$ eine $R^{13}$ analoge Bedeutung hat, und abschließend aufgereinigt wird.

**9.** Verfahren nach einem der Ansprüche 7 oder 8, **dadurch gekennzeichnet, daß** zur Herstellung des Ausgangsprodukts gemäß Formel II zunächst 3,3-Dimethyl-1,5-dioxa-spiro[5.5]undecan-8-on

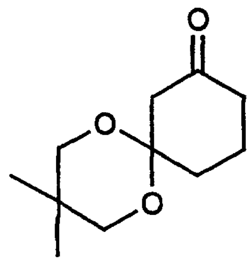

mit Immoniumsalzen der Formel V bzw. mit Formaldehyd und einem Amin der Formel VI umgesetzt werden, wobei $R^{15}$ eine $R^3$ und $R^{16}$ eine $R^4$ entsprechende Bedeutung gemäß Anspruch 1 haben,

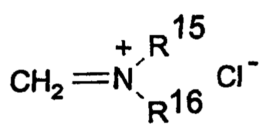

**V**

$$R^{15}R^{16}NH$$

**VI**

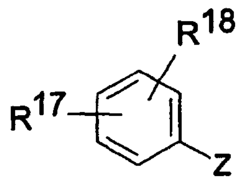

VII

und anschließend die so erhaltenen Mannich-Basen mit einer metallorganischen Verbindung der Formel VII, in der Z MgCl, MgBr, MgI oder Lithium bedeuten und $R^{17}$ eine $R^1$ und $R^{18}$ eine $R^2$ entsprechende Bedeutung gemäß Anspruch 1 haben, bei Temperaturen zwischen -70°C und 60°C, vorzugsweise in Diethylether und/oder Tetrahydrofuran reagieren.

**10.** Verfahren nach einem der Ansprüche 7 bis 9 **dadurch gekennzeichnet, daß** vor der abschließenden Aufreinigung ein Verfahrensprodukt mit mindestens einer $C(O)OCH_3$-, $OC(O)OCH_3$- und/oder $C(S)OCH_3$-Gruppe mit KOH-Lösung bzw. NaOH-Lösung in Methanol bei 40°C - 60°C verseift wird.

**11.** Verfahren gemäß einem der Ansprüche 7 bis 10, **dadurch gekennzeichnet, daß** in $R^{15}$ bis $R^{20}$ gemäß Formeln II bis VII, mindestens eine OH-Gruppe durch eine $OSi(Ph)_2$tert.but.-Gruppe, mindestens eine SH-Gruppe durch eine S-p-Methoxybenzylgruppe und/oder mindestens eine $NH_2$-Gruppe durch eine $NO_2$-Gruppe ersetzt wurde und unmittelbar vor der Aufreinigung des Endprodukts mindestens eine $OSi(Ph)_2$tert.but.-Gruppe mit Tetrabutylammoniumflluorid in Tetrahydrofuran und/oder mindestens eine p-Methoxybenzylgruppe mit einem Metallamin, bevorzugt Natriumamin, abgespalten und/oder mindestens eine $NO_2$-Gruppe zu $NH_2$ reduziert wird.

**12.** Arzneimittel enthaltend als Wirkstoff mindestens ein Aminomethyl-Phenyl-Cyclohexanonderivat der allgemeinen Formel I bzw. Ia

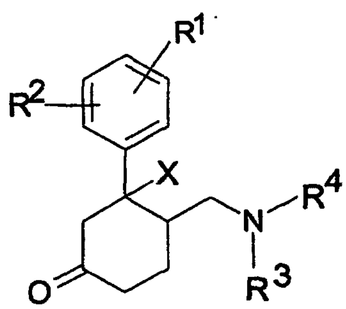

i

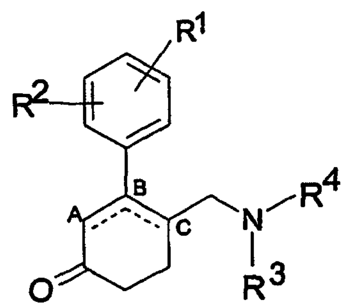

Ia

, worin
$R^1$ und $R^2$ unabhängig voneinander ausgewählt sind aus $R^5$ oder $YR^5$ mit Y = $C_1$-$C_{10}$-Alkyl, $C_2$-$C_{10}$-Alkenyl oder $C_2$-$C_{10}$-Alkinyl, verzweigt oder unverzweigt, einfach oder mehrfach substituiert oder unsubstituiert, wobei $R^5$ ausgewählt ist aus

H, F, Cl, Br, I, CN, $NO_2$, $C_1$-$C_8$-Alkyl, $C_2$-$C_8$-Alkenyl oder $C_2$-$C_8$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^6$ ersetzt ist, mit $R^6$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

Aryl oder Heteroaryl, jeweils einfach oder mehrfach substituiert oder unsubstituiert;

$OR^7$, $OC(O)R^7$, $OC(O)OR^7$, $OC(S)R^7$, $C(O)R^7$, $C(O)OR^7$, $C(S)R^7$, $C(S)OR^7$, $SR^7$, $S(O)R^7$ bzw. $S(O_2)R^7$, wobei $R^7$ ausgewählt ist aus

H, $C_1$-$C_{18}$-Alkyl, $C_2$-$C_{18}$-Alkenyl oder $C_2$-$C_{18}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^8$ ersetzt ist, mit $R^8$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

Alkylaryl, gesättigt oder ungesättigt, einfach oder mehrfach substituiert oder unsubstituiert; Aryl oder Heteroaryl, jeweils einfach oder mehrfach substituiert oder unsubstituiert; oder

$NR^9R^{10}$, $C(O)NR^9R^{10}$ oder $S(O_2)NR^9R^{10}$, wobei $R^9$ und $R^{10}$ unabhängig voneinander ausgewählt sind aus

H, $C_1$-$C_{18}$-Alkyl, $C_2$-$C_{18}$-Alkenyl oder $C_2$-$C_{18}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^{11}$ ersetzt ist, mit $R^{11}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder

$R^9$ und $R^{10}$ zusammen ein $C_3$-$C_7$-Cycloalkyl, gesättigt oder ungesättigt, einfach oder mehrfach substituiert oder unsubstituiert, bilden, bzw. einen entsprechenden Heterocyclus, bei dem ein C-Atom im Ring durch S, O oder $NR^{12}$ ersetzt ist, mit $R^{12}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

oder $R^1$ und $R^2$ zusammen -CH=CH-CH=CH- bilden, wobei das entstehende Naphthylsystem ein- oder mehrfach substituiert sein kann,

X ausgewählt ist aus

H, F, Cl, Br, I, $CF_3$, O-$S(O_2)$-$C_6H_4$-$pCH_3$, $OR^{13}$ oder $OC(O)R^{13}$, wobei $R^{13}$ ausgewählt ist aus

H; $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch N, S oder O ersetzt ist; Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder,

wenn die Verbindung kein X aufweist, gemäß Formel Ia zwischen C-Atom A und C-Atom B oder C-Atom B und C-Atom C eine Doppelbindung ausgebildet ist;

und

$R^3$, $R^4$ unabhängig voneinander ausgewählt sind aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch N, S oder O ersetzt ist; Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder

$R^3$ und $R^4$ zusammen ein $C_3$-$C_7$-Cycloalkyl, gesättigt oder ungesättigt, einfach oder mehrfach substituiert oder unsubstituiert,bilden, bzw. einen entsprechenden Heterocyclus, bei dem ein C-Atom im Ring durch S, O oder $NR^{14}$ ersetzt ist, mit $R^{14}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

in Form seiner Diastereomere oder Enantiomere sowie seiner freien Base oder eines mit einer physiologisch verträglichen Säure gebildeten Salzes, insbesondere des Hydrochloridsalzes.

**13.** Arzneimittel nach Anspruch 12, **dadurch gekennzeichnet, daß** unabhängig voneinander in den allgemeinen Formeln I bzw. Ia

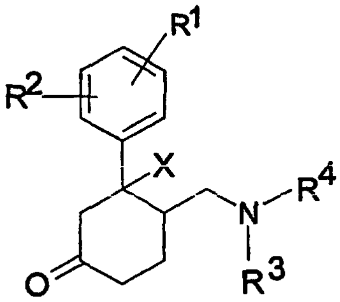

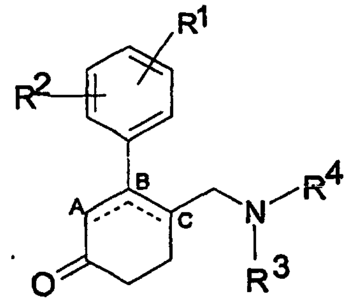

I . Ia .

$R^1 = R^5$ ist, wobei $R^5$ ausgewählt ist aus

H, F, Cl, Br, I, $CHF_2$, $CF_3$, $NO_2$, $NH_2$; $C_1$-$C_4$-Alkyl oder $C_2$-$C_4$-Alkenyl, verzweigt oder unverzweigt, einfach oder mehrfach substituiert oder unsubstituiert; Aryl substituiert oder unsubstituiert; $OR^7$, $C(O)OR^7$ bzw. $SR^7$ wobei $R^7$ ausgewählt ist aus

H; $C_1$-$C_4$-Alkyl, verzweigt oder unverzweigt, einfach oder mehrfach substituiert oder unsubstituiert; vorzugsweise H, $CF_3$ oder $CH_3$,

oder $S(O_2)NR^9R^{10}$, wobei $R^9$ und $R^{10}$ unabhängig voneinander ausgewählt sind aus

H; $C_1$-$C_4$-Alkyl, verzweigt oder unverzweigt, einfach oder mehrfach substituiert oder unsubstituiert;

, wobei insbesondere bevorzugt ist, daß R1 = R5 mit R5 ausgewählt aus

H, F, Cl, OH, $CH_3$, $C_2H_5$, $C_2H_3$, $CHF_2$, $CF_3$, $SCH_3$, $OCF_3$, $OCH_3$, $OC_2H_5$, $C(O)OCH_3$, $C(O)OC_2H_5$ oder Phenyl,

**und/oder** $R^2 = R^5$, wobei $R^5$ ausgewählt ist aus

H, F, Cl, Br, I, $SCH_3$; $C_1$-$C_4$-Alkyl, $C_2$-$C_4$-Alkenyl verzweigt oder unverzweigt, einfach oder mehrfach substituiert oder unsubstituiert, vorzugsweise $CF_3$; $OR^7$, mit $R^7$ ausgewählt aus $C_1$-$C_4$-Alkyl, verzweigt oder unverzweigt, einfach oder mehrfach substituiert oder unsubstituiert, vorzugsweise $CH_3$;

**und/oder** $R^1$ und $R^2$ unterschiedliche Bedeutungen haben oder $R^1$ und $R^2$ zusammen -CH=CH-CH=CH- bilden, wobei das entstehende Naphthylsystem ein- oder mehrfach, vorzugsweise mit Halogen, $OC_{1-3}$-Alkyl oder $C_{1-3}$-Alkyl, unsubstituiert oder ein oder mehrfach substituiert, insbesondere mit $OCH_3$; substituiert, sein kann,

**und/oder** X ausgewählt ist aus

H, F, Cl, OH, $CF_3$, O-$S(O_2)$-$C_6H_4$-p$CH_3$ bzw. $OC(O)R^7$ mit $R^7$ = H; $C_1$-$C_4$-Alkyl oder $C_2$-$C_4$-Alkenyl, verzweigt oder unverzweigt, einoder mehrfach substituiert oder unsubstituiert,

vorzugsweise H, F, Cl, OH, O-$S(O_2)$-$C_6H_4$-p$CH_3$, $OC(O)R^7$ mit $R^7$ = $C_1$-$C_4$-Alkyl, vorzugsweise $CH_3$, insbesondere H oder OH,

oder

, wenn die Verbindung kein X aufweist, gemäß Formel Ia zwischen C-Atom A und C-Atom B oder C-Atom B und C-Atom C eine Doppelbindung ausgebildet ist

**und/oder** $R^3$ und $R^4$ unabhängig voneinander ausgewählt sind aus

$C_1$-$C_4$-Alkyl, verzweigt oder unverzweigt, einfach oder mehrfach substituiert oder unsubstituiert, vorzugsweise $CH_3$;

oder

$R^3$ und $R^4$ zusammen ein $C_3$-$C_7$-Cycloalkyl, gesättigt oder ungesättigt, einfach oder mehrfach substituiert oder unsubstituiert, bilden,

**und/oder** $R^1$, $R^2$, X, $R^3$ und/oder $R^4$ eine der in den vorangehenden Ansprüchen genannten Bedeutungen haben.

**14.** Arzneimittel gemäß Anspruch 12 enthaltend als Wirkstoff mindestens ein Aminomethyl-Phenyl-Cyclohexanonderivat ausgewählt aus der Gruppe

- *rac-cis*-[4-Dimethylaminomethyl-3-hydroxy-3-(3-methoxy-phenyl)-cyclohexanon],
- *rac-cis*-[4-Dimethylaminomethyl-3-(3-methoxy-phenyl)-cyclohexanon],

- *rac-trans*-[4-Dimethylaminomethyl-3-(3-methoxy-phenyl)-cyclohexanon],
- *rac-cis*-[4-Dimethylaminomethyl-3-(3-hydroxy-phenyl)-cyclohexanon,
- *rac-trans*-[4-Dimethylaminomethyl-3-(3-hydroxy-phenyl)-cyclohexanon oder
- 4-Dimethylaminomethyl-3-(3-methoxy-phenyl)-cyclohex-2-enon
- 4-Dimethylaminomethyl-3-phenyl-cyclohex-2-enon; e
- 4-Dimethylaminomethyl-3-(3-hydroxy-phenyl)-cyclohex-2-enon;
- rac-trans-4-Dimethylaminomethyl-3-phenyl-cyclohexanon;
- rac-cis-4-Dimethylaminomethyl-3-phenyl-cyclohexanon;
- 4-Dimethylaminomethyl-3-(4-methoxy-phenyl)-cyclohex-2-enon;
- 4-Dimethylaminomethyl-3-naphthalen-2-yl-cyclohex-2-enon;
- rac-trans-4-Dimethylaminomethyl-3-hydroxy-3-naphthalen-2-ylcyclohexanon;
- rac-trans-4-Dimethylaminomethyl-3-(3-fluoro-phenyl)-cyclohexanon;
- rac-cis-4-Dimethylaminomethyl-3-(3-fluoro-phenyl)-cyclohexanon;
- rao-cis-4-Dimethylaminomethyl-3-hydroxy-3-(2-methoxy-phenyl)-cyclohexanon
- rac-cis-4-Dimethylaminomethyl-3-hydroxy-3-(6-methoxy-naphthalen-2-yl)-cyclohexanon;
- 4-Dirnethylaminomethyl-3-(6-methoxy-naphthalen-2-yl)-cyclohex-2-enon;
- rac-cis-3-Biphenyl-4-yl-4-dimethylaminomethyl-3-hydroxy-cyclohexanon
- 3-(3-Difluoromethyl-phenyl)-4-dimethylaminomethyl-cyclohex-2-enon;
- 4-Dimethylaminomethyl-3-(3-fluoro-phenyl)-cyclohex-2-enon;
- 3-(3,4-Difluoro-phenyl)-4-dimethylaminomethyl-cyclohex-2-enon;
- 4-Dimethylaminomethyl-3-(4-fluoro-phenyl)-cyclohex-2-enon;
- 4-Dimethylaminomethyl-3-(4-trifluoromethyl-phenyl)-cyclohex-2-enon;
- 3-(3,5-Dichloro-phenyl)-4-dimethylaminomethyl-cyclohex-2-enon;
- 3-(3,5-Difluoro-phenyl)-4-dimethylaminomethyl-cyclohex-2-enon;
- rac-cis-3-(3,5-Difluoro-phenyl)-4-dimethylaminomethyl-3-hydroxycyclohexanon

als freie Base oder in Form eines mit einer physiologisch verträglichen Säure gebildeten Salzes, insbesondere des Hydrochloridsalzes.

**15.** Verwendung mindestens eines Aminomethyl-Phenyl-Cyclohexanonderivats der allgemeinen Formel I bzw. Ia

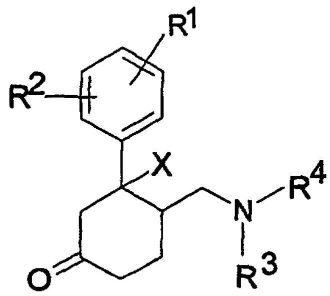

I

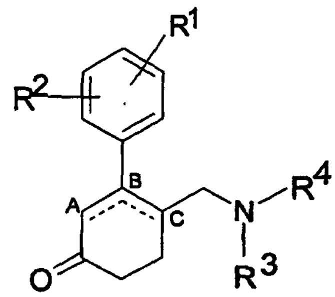

Ia

, worin
$R^1$ und $R^2$ unabhängig voneinander ausgewählt sind aus $R^5$ oder $YR^5$ mit Y = $C_1$-$C_{10}$-Alkyl, $C_2$-$C_{10}$-Alkenyl oder $C_2$-$C_{10}$-Alkinyl, verzweigt oder unverzweigt, einfach oder mehrfach substituiert oder unsubstituiert, wobei $R^5$ ausgewählt ist aus

H, F, Cl, Br, I, CN, $NO_2$, $C_1$-$C_8$-Alkyl, $C_2$-$C_8$-Alkenyl oder $C_2$-$C_8$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^6$ ersetzt ist, mit $R^6$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

Aryl oder Heteroaryl, jeweils einfach oder mehrfach substituiert oder unsubstituiert;

$OR^7$, $OC(O)R^7$, $OC(O)OR^7$, $OC(S)R^7$, $C(O)R^7$, $C(O)OR^7$, $C(S)R^7$, $C(S)OR^7$, $SR^7$, $S(O)R^7$ bzw. $S(O_2)R^7$, wobei $R^7$ ausgewählt ist aus

H, $C_1$-$C_{18}$-Alkyl, $C_2$-$C_{18}$-Alkenyl oder $C_2$-$C_{18}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^8$ ersetzt ist, mit $R^8$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

Alkylaryl, gesättigt oder ungesättigt, einfach oder mehrfach substituiert oder unsubstituiert; Aryl oder Heteroaryl, jeweils einfach oder mehrfach substituiert oder unsubstituiert; oder

$NR^9R^{10}$, $C(O)NR^9R^{10}$ oder $S(O_2)NR^9R^{10}$, wobei $R^9$ und $R^{10}$ unabhängig voneinander ausgewählt sind aus

H, $C_1$-$C_{18}$-Alkyl, $C_2$-$C_{18}$-Alkenyl oder $C_2$-$C_{18}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^{11}$ ersetzt ist, mit $R^{11}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert; Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder

$R^9$ und $R^{10}$ zusammen ein $C_3$-$C_7$-Cycloalkyl, gesättigt oder ungesättigt, einfach oder mehrfach substituiert oder unsubstituiert, bilden, bzw. einen entsprechenden Heterocyclus, bei dem ein C-Atom im Ring durch S, O oder $NR^{12}$ ersetzt ist, mit $R^{12}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

oder

$R^1$ und $R^2$ zusammen -CH=CH-CH=CH- bilden, wobei das entstehende Naphthylsystem ein- oder mehrfach substituiert sein kann,

X ausgewählt ist aus

H, F, Cl, Br, I, $CF_3$, O-$S(O_2)$-$C_6H_4$-$pCH_3$, $OR^{13}$ oder $OC(O)R^{13}$, wobei $R^{13}$ ausgewählt ist aus

H; $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch N, S oder O ersetzt ist; Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder,

wenn die Verbindung kein X aufweist, gemäß Formel Ia zwischen C-Atom A und C-Atom B oder C-Atom B und C-Atom C eine Doppelbindung ausgebildet ist;

und

$R^3$, $R^4$ unabhängig voneinander ausgewählt sind aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch N, S oder O ersetzt ist; Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder

$R^3$ und $R^4$ zusammen ein $C_3$-$C_7$-Cycloalkyl, gesättigt oder ungesättigt, einfach oder mehrfach substituiert oder unsubstituiert,bilden, bzw. einen entsprechenden Heterocyclus, bei dem ein C-Atom im Ring durch S, O oder $NR^{14}$ ersetzt ist, mit $R^{14}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

in Form seiner Diastereomere oder Enantiomere sowie seiner freien Base oder eines mit einer physiologisch verträglichen Säure gebildeten Salzes, insbesondere des Hydrochloridsalzes, zur Herstellung eines Arzneimittels zur Behandlung von Schmerz.

**16.** Verwendung mindestens eines Aminomethyl-Phenyl-Cyclohexanonderivats der allgemeinen Formel I bzw. Ia

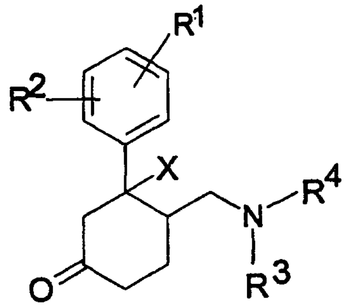

I

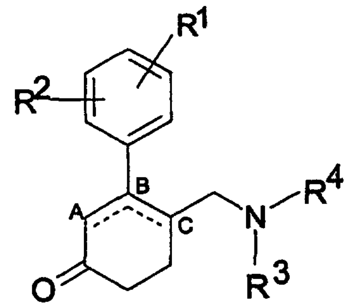

Ia

, worin $R^1$ und $R^2$ unabhängig voneinander ausgewählt sind aus $R^5$ oder $YR^5$ mit Y = $C_1$-$C_{10}$-Alkyl, $C_2$-$C_{10}$-Alkenyl oder $C_2$-$C_{10}$-Alkinyl, verzweigt oder unverzweigt, einfach oder mehrfach substituiert oder unsubstituiert, wobei $R^5$ ausgewählt ist aus

H, F, Cl, Br, I, CN, $NO_2$, $C_1$-$C_8$-Alkyl, $C_2$-$C_8$-Alkenyl oder $C_2$-$C_8$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^6$ ersetzt ist, mit $R^6$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

Aryl oder Heteroaryl, jeweils einfach oder mehrfach substituiert oder unsubstituiert;

$OR^7$, $OC(O)R^7$, $OC(O)OR^7$, $OC(S)R^7$, $C(O)R^7$, $C(O)OR^7$, $C(S)R^7$, $C(S)OR^7$, $SR^7$, $S(O)R^7$ bzw. $S(O_2)R^7$, wobei $R^7$ ausgewählt ist aus

H, $C_1$-$C_{18}$-Alkyl, $C_2$-$C_{18}$-Alkenyl oder $C_2$-$C_{18}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^8$ ersetzt ist, mit $R^8$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

Alkylaryl, gesättigt oder ungesättigt, einfach oder mehrfach substituiert oder unsubstituiert; Aryl oder Heteroaryl, jeweils einfach oder mehrfach substituiert oder unsubstituiert; oder

$NR^9R^{10}$, $C(O)NR^9R^{10}$ oder $S(O_2)NR^9R^{10}$, wobei $R^9$ und $R^{10}$ unabhängig voneinander ausgewählt sind aus

H, $C_1$-$C_{18}$-Alkyl, $C_2$-$C_{18}$-Alkenyl oder $C_2$-$C_{18}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^{11}$ ersetzt ist, mit $R^{11}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder

$R^9$ und $R^{10}$ zusammen ein $C_3$-$C_7$-Cycloalkyl, gesättigt oder ungesättigt, einfach oder mehrfach substituiert oder unsubstituiert, bilden, bzw. einen entsprechenden Heterocyclus, bei dem ein C-Atom im Ring durch S, O oder $NR^{12}$ ersetzt ist, mit $R^{12}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

oder

$R^1$ und $R^2$ zusammen -CH=CH-CH=CH- bilden, wobei das entstehende Naphthylsystem ein- oder mehrfach substituiert sein kann,

X ausgewählt ist aus

H, F, Cl, Br, I, $CF_3$, O-S($O_2$)-$C_6H_4$-p$CH_3$, $OR^{13}$ oder OC(O)$R^{13}$, wobei $R^{13}$ ausgewählt ist aus

H; $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch N, S oder O ersetzt ist; Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder,

wenn die Verbindung kein X aufweist, gemäß Formel Ia zwischen C-Atom A und C-Atom B oder C-Atom B und C-Atom C eine Doppelbindung ausgebildet ist;

und

$R^3$, $R^4$ unabhängig voneinander ausgewählt sind aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch N, S oder O ersetzt ist; Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder

$R^3$ und $R^4$ zusammen ein $C_3$-$C_7$-Cycloalkyl, gesättigt oder ungesättigt, einfach oder mehrfach substituiert oder unsubstituiert,bilden, bzw. einen entsprechenden Heterocyclus, bei dem ein C-Atom im Ring durch S, O oder $NR^{14}$ ersetzt ist, mit $R^{14}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

in Form seiner Diastereomere oder Enantiomere sowie seiner freien Base oder eines mit einer physiologisch verträglichen Säure gebildeten Salzes, insbesondere des Hydrochloridsalzes, zur Herstellung eines Arzneimittels zur Behandlung von inflammatorischen und allergischen Reaktionen, Depressionen, Drogen- und/oder Alkoholmißbrauch, Gastritis, cardiovaskulären Erkrankungen, Atemwegserkrankungen, Husten, seelischen Erkrankungen und/oder Epilepsie sowie insbesondere von Harninkontinenz, Juckreiz und/oder Diarrhoe.

**17.** Verwendung nach einem der Ansprüche 15 oder 16, **dadurch gekennzeichnet, daß** unabhängig voneinander in den allgemeinen Formeln I bzw. Ia

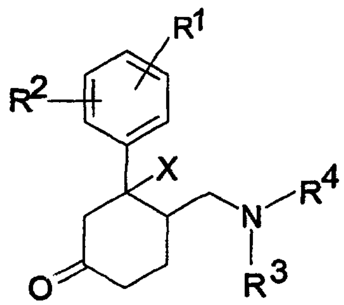

I

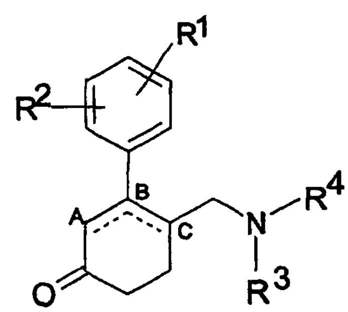

Ia

$R^1$ = $R^5$, wobei $R^5$ ausgewählt ist aus

H, F, Cl, Br, I, $CHF_2$, $CF_3$, $NO_2$, $NH_2$; $C_1$-$C_4$-Alkyl oder $C_2$-$C_4$-Alkenyl, verzweigt oder unverzweigt, einfach oder mehrfach substituiert oder unsubstituiert; Aryl substituiert oder unsubstituiert; $OR^7$, C(O)$OR^7$ bzw. $SR^7$ wobei $R^7$ ausgewählt ist aus

H; $C_1$-$C_4$-Alkyl, verzweigt oder unverzweigt, einfach oder mehrfach substituiert oder unsubstituiert; vorzugsweise H, $CF_3$ oder $CH_3$,

oder S($O_2$)$NR^9R^{10}$, wobei $R^9$ und $R^{10}$ unabhängig voneinander ausgewählt sind aus

H; $C_1$-$C_4$-Alkyl, verzweigt oder unverzweigt, einfach oder mehrfach substituiert oder unsubstituiert;

, wobei insbesondere bevorzugt ist, daß R1 = R5 mit R5 ausgewählt aus

H, F, Cl, OH, $CH_3$, $C_2H_5$, $C_2H_3$, $CHF_2$, $CF_3$, $SCH_3$, $OCF_3$, $OCH_3$, $OC_2H_5$, $C(O)OCH_3$, $C(O)OC_2H_5$ oder Phenyl,

**und/oder** $R^2 = R^5$, wobei $R^5$ ausgewählt ist aus

H, F, Cl, Br, I, $SCH_3$; $C_1$-$C_4$-Alkyl, $C_2$-$C_4$-Alkenyl verzweigt oder unverzweigt, einfach oder mehrfach substituiert oder unsubstituiert, vorzugsweise $CF_3$; $OR^7$, mit $R^7$ ausgewählt aus $C_1$-$C_4$-Alkyl, verzweigt oder unverzweigt, einfach oder mehrfach substituiert oder unsubstituiert, vorzugsweise $CH_3$;

**und/oder** $R^1$ und $R^2$ unterschiedliche Bedeutungen haben oder $R^1$ und $R^2$ zusammen -CH=CH-CH=CH- bilden, wobei das entstehende Naphthylsystem ein- oder mehrfach, vorzugsweise mit Halogen, $OC_{1-3}$-Alkyl oder $C_{1-3}$-Alkyl, unsubstituiert oder ein oder mehrfach substituiert, insbesondere mit $OCH_3$; substituiert, sein kann,

**und/oder** X ausgewählt ist aus

H, F, Cl, OH, $CF_3$, O-$S(O_2)$-$C_6H_4$-$pCH_3$ bzw. $OC(O)R^7$ mit $R^7$ = H; $C_1$-$C_4$-Alkyl oder $C_2$-$C_4$-Alkenyl, verzweigt oder unverzweigt, einoder mehrfach substituiert oder unsubstituiert,

vorzugsweise H, F, Cl, OH, O-$S(O_2)$-$C_6H_4$-$pCH_3$, $OC(O)R^7$ mit $R^7$ = $C_1$-$C_4$-Alkyl, vorzugsweise $CH_3$, insbesondere H oder OH;

oder

, wenn die Verbindung kein X aufweist, gemäß Formel Ia zwischen C-Atom A und C-Atom B oder C-Atom B und C-Atom C eine Doppelbindung ausgebildet ist

**und/oder** $R^3$ und $R^4$ unabhängig voneinander ausgewählt sind aus

$C_1$-$C_4$-Alkyl, verzweigt oder unverzweigt, einfach oder mehrfach substituiert oder unsubstituiert, vorzugsweise $CH_3$;

oder

$R^3$ und $R^4$ zusammen ein $C_3$-$C_7$-Cycloalkyl, gesättigt oder ungesättigt, einfach oder mehrfach substituiert oder unsubstituiert, bilden,

**und/oder** $R^1$, $R^2$, X, $R^3$ und/oder $R^4$ eine der in den vorangehenden Ansprüchen genannten Bedeutungen haben.

**18.** Verwendung gemäß einem der Ansprüche 15 oder 16, **dadurch gekennzeichnet, daß** als Wirkstoff mindestens ein Aminomethyl-Phenyl-Cyclohexanonderivat ausgewählt aus der Gruppe

- *rac-cis*-[4-Dimethylaminomethyl-3-hydroxy-3-(3-methoxy-phenyl)-cyclohexanon],
- *rac-cis*-[4-Dimethylaminomethyl-3-(3-methoxy-phenyl)-cyclohexanon],
- *rac-trans*-[4-Dimethylaminomethyl-3-(3-methoxy-phenyl)-cyclohexanon],
- *rac-cis*-[4-Dimethylaminomethyl-3-(3-hydroxy-phenyl)-cyclohexanon,
- *rac-trans*-[4-Dimethylaminomethyl-3-(3-hydroxy-phenyl)-cyclohexanon oder
- 4-Dimethylaminomethyl-3-(3-methoxy-phenyl)-cyclohex-2-enon
- 4-Dimethylaminomethyl-3-phenyl-cyclohex-2-enon; e
- 4-Dimethylaminomethyl-3-(3-hydroxy-phenyl)-cyclohex-2-enon;
- rac-trans-4-Dimethylaminomethyl-3-phenyl-cyclohexanon;
- rac-cis-4-Dimethylaminomethyl-3-phenyl-cyclohexanon;
- 4-Dimethylaminomethyl-3-(4-methoxy-phenyl)-cyclohex-2-enon;
- 4-Dimethylaminomethyl-3-naphthalen-2-yl-cyclohex-2-enon;
- rac-trans-4-Dimethylaminomethyl-3-hydroxy-3-naphthaden-2-ylcyclohexanon;
- rac-trans-4-Dimethylaminomethyl-3-(3-fluoro-phenyl)-cyclohexanon;
- rac-cis-4-Dimethylaminomethyl-3-(3-fluoro-phenyl)-cyclohexanon;
- rac-cis-4-Dimethylaminomethyl-3-hydroxy-3-(2-methoxy-phenyl)-cyclohexanon
- rac-cis-4-Dimethylaminomethyl-3-hydroxy-3-(6-methoxy-naphthalen-2-yl)-cyclohexanon;
- 4-Dimethylaminomethyl-3-(6-methoxy-naphthalen-2-yl)-cyclohex-2-enon;
- rac-cis-3-Biphenyl-4-yl-4-dimethylaminomethyl-3-hydroxy-cyclohexanon
- 3-(3-Difluoromethyl-phenyl)-4-dimethylaminomethyl-cyclohex-2-enon;
- 4-Dimethylaminomethyl-3-(3-fluoro-phenyl)-cyclohex 2-enon;
- 3-(3,4-Difluoro-phenyl)-4-dimethylaminomethyl-cyclohex-2-enon;
- 4-Dimethylaminomethyl-3-(4-fluoro-phenyl)-cyclohex-2-enon;
- 4-Dimethylaminomethyl-3-(4-trifluoromethyl-phenyl)-cyclohex-2-enon;
- 3-(3,5-Dichloro-phenyl)-4-dimethylaminomethyl-cyclohex-2-enon;
- 3-(3,5-Difluoro-phenyl)-4-dimethylaminomethyl-cyclohex-2-enon;
- rac-cis-3-(3,5-Difluoro-phenyl)-4-dimethylaminomethyl-3-hydroxycyclohexanon

als freie Base oder in Form eines mit einer physiologisch verträglichen Säure gebildeten Salzes, insbesondere des Hydrochloridsalzes verwendet wird.

**Claims**

**1.** Aminomethyl-phenyl-cyclohexanone derivatives of the general formula I and Ia, also in the form of their diastereomers or enantiomers and of their free base or of a salt formed with a physiologically tolerated acid, in particular the hydrochloride salt

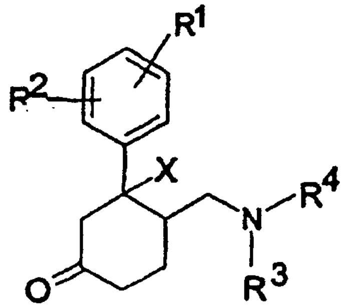

I

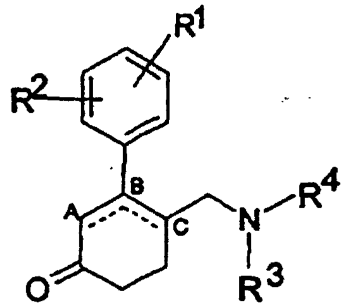

Ia

wherein $R^1$ and $R^2$ independently of one another are chosen from $R^5$ or $YR^5$, where Y = $C_1$-$C_{10}$-alkyl, $C_2$-$C_{10}$-alkenyl or $C_2$-$C_{10}$-alkinyl, branched or unbranched, and mono- or polysubstituted or unsubstituted, wherein $R^5$ is chosen from

H, F, Cl, Br, I, CN, $NO_2$; $C_1$-$C_8$-alkyl, $C_2$-$C_8$-alkenyl or $C_2$-$C_8$-alkinyl, in each case branched or unbranched and mono- or polysubstituted or unsubstituted; $C_3$-$C_7$-cycloalkyl, saturated or unsaturated and mono- or polysubstituted or unsubstituted, or a corresponding heterocyclic radical, in which one C atom in the ring is replaced by S, O or $NR^6$, where $R^6$ is chosen from

H, $C_1$-$C_{10}$-alkyl, $C_2$-$C_{10}$-alkenyl or $C_2$-$C_{10}$-alkinyl, in each case branched or unbranched and mono- or polysubstituted or unsubstituted;

aryl or heteroaryl, in each case mono- or polysubstituted or unsubstituted;

$OR^7$, $OC(O)R^7$, $OC(O)OR^7$, $OC(S)R^7$, $C(O)R^7$, $C(O)OR^7$, $C(S)R^7$, $C(S)OR^7$, $SR^7$, $S(O)R^7$ or $S(O_2)R^7$, wherein $R^7$ is chosen from

H, $C_1$-$C_{18}$-alkyl, $C_2$-$C_{18}$-alkenyl or $C_2$-$C_{18}$-alkinyl, in each case branched or unbranched and mono- or polysubstituted or unsubstituted; $C_3$-$C_7$-cycloalkyl, saturated or unsaturated and mono- or polysubstituted or unsubstituted, or a corresponding heterocyclic radical, in which one C atom in the ring is replaced by S, O or $NR^8$, where $R^8$ is chosen from

H, $C_1$-$C_{10}$-alkyl, $C_2$-$C_{10}$-alkenyl or $C_2$-$C_{10}$-alkinyl, in each case branched or unbranched and mono- or polysubstituted or unsubstituted;

alkylaryl, saturated or unsaturated and mono- or polysubstituted or unsubstituted; aryl or heteroaryl, in each case mono- or polysubstituted or unsubstituted; or

$NR^9R^{10}$, $C(O)NR^9R^{10}$ or $S(O_2)NR^9R^{10}$, wherein $R^9$ and $R^{10}$ independently of one another are chosen from

H, $C_1$-$C_{18}$-alkyl, $C_2$-$C_{18}$-alkenyl or $C_2$-$C_{18}$-alkinyl, in each case branched or unbranched and mono- or polysubstituted or unsubstituted; $C_3$-$C_7$-cycloalkyl, saturated or unsaturated and mono- or polysubstituted or unsubstituted, or a corresponding heterocyclic radical, in which one C atom in the ring is replaced by S, O or $NR^{11}$, where

$R^{11}$ is chosen from

H, $C_1$-$C_{10}$-alkyl, $C_2$-$C_{10}$-alkenyl or $C_2$-$C_{10}$-alkinyl, in each case branched or unbranched and mono- or polysubstituted or unsubstituted;

alkylaryl, saturated or unsaturated and mono- or polysubstituted or unsubstituted; aryl or heteroaryl, in each case mono- or polysubstituted or unsubstituted;

or

$R^9$ and $R^{10}$ together form a $C_3$-$C_7$-cycloalkyl, saturated or unsaturated and mono- or polysubstituted or unsubstituted, or a corresponding heterocyclic radical, in which one C atom in the ring is replaced by S, O or $NR^{12}$, where $R^{12}$ is chosen from

H, $C_1$-$C_{10}$-alkyl, $C_2$-$C_{10}$-alkenyl or $C_2$-$C_{10}$-alkinyl, in each case branched or unbranched and mono- or polysubstituted or unsubstituted;

or

$R^1$ and $R^2$ together form -CH=CH-CH=CH-, wherein the naphthyl system formed can be mono- or polysubstituted,

X is chosen from

H, F, Cl, Br, I, $CF_3$, O-S($O_2$)-$C_6H_4$-p$CH_3$, $OR^{13}$ or OC(O)$R^{13}$, wherein $R^{13}$ is chosen from

H; $C_1$-$C_{10}$-alkyl, $C_2$-$C_{10}$-alkenyl or $C_2$-$C_{10}$-alkinyl, in each case branched or unbranched and mono- or polysubstituted or unsubstituted; $C_3$-$C_7$-cycloalkyl, saturated or unsaturated and mono- or polysubstituted or unsubstituted, or a corresponding heterocyclic radical, in which one C atom in the ring is replaced by N, S or O; alkylaryl, saturated or unsaturated and mono- or polysubstituted or unsubstituted; aryl or heteroaryl, in each case mono- or polysubstituted or unsubstituted;

or

if the compound contains no X, according to formula Ia a double bond is formed between C atom A and C atom B or C atom B and C atom C;

and

$R^3$, $R^4$ independently of one another are chosen from

H, $C_1$-$C_{10}$-alkyl, $C_2$-$C_{10}$-alkenyl or $C_2$-$C_{10}$-alkinyl, in each case branched or unbranched and mono- or polysubstituted or unsubstituted; $C_3$-$C_7$-cycloalkyl, saturated or unsaturated and mono- or polysubstituted or unsubstituted, or a corresponding heterocyclic radical, in which one C atom in the ring is replaced by N, S or O; alkylaryl, saturated or unsaturated and mono- or polysubstituted or unsubstituted; aryl or heteroaryl, in each case mono- or polysubstituted or unsubstituted;

or

$R^3$ and $R^4$ together form a $C_3$-$C_7$-cycloalkyl, saturated or unsaturated and mono- or polysubstituted or unsubstituted, or a corresponding heterocyclic radical, in which one C atom in the ring is replaced by S, O or $NR^{14}$, where $R^{14}$ is chosen from

H, $C_1$-$C_{10}$-alkyl, $C_2$-$C_{10}$-alkenyl or $C_2$-$C_{10}$-alkinyl, in each case branched or unbranched and mono- or polysubstituted or unsubstituted

with the proviso that

if X = OH, $R^2$ = H, $R^3$ and $R^4$ are each $CH_3$, $R^1$ is not at the same time $R^5$, where $R^5$ = $OR^7$, where $R^7$ = H, $CH_3$ or an unsubstituted or substituted pyridyl, thienyl, thiazoyl or phenyl group, or $R^5$ = OC(O)$OR^7$, where $R^7$ = $C_1$-$C_5$-alkyl, or $R^5$ = OC(O)$R^7$, where $R^7$ = $C_{1-5}$-alkyl, NH-$C_6H_4$-$C_{1-3}$-alkyl, $C_6H_4$-OC(O)$C_{1-3}$-alkyl, $C_6H_4$-$CH_2$-N($C_{1-4}$-alkyl)$_2$, $C_6H_4$-$CH_2$-(N-4-morpholino) or CHZ'-NHZ", where Z' and Z" are identical or different and are chosen from H or $C_{1-6}$-alkyl,

or

if X = H or according to formula Ia a double bond is formed between C atom A and C atom B, $R^2$ = H, $R^3$ and $R^4$ in each case independently of one another are $C_{1-6}$-alkyl, aryl or $C_{3-7}$-cycloalkyl, $R^1$ is not at the same time H, $C_{1-6}$-alkoxy, O-$C_{3-7}$-cycloalkyl, O-aryl or 0-heterocyclyl.

2. Aminomethyl-phenyl-cyclohexanone derivatives according to claim 1, **characterized in that** $R^1$ = $R^5$, where $R^5$ is chosen from

H, F, Cl, Br, I, $CHF_2$, $CF_3$, $NO_2$, $NH_2$; $C_1$-$C_4$-alkyl or $C_2$-$C_4$-alkenyl, branched or unbranched and mono-or polysubstituted or unsubstituted; aryl, substituted or unsubstituted; $OR^7$, C(O)$OR^7$ or $SR^7$, wherein $R^7$ is chosen from

H; $C_1$-$C_4$-alkyl, branched or unbranched and mono- or polysubstituted or unsubstituted; preferably H, $CF_3$ or $CH_3$,

or S($O_2$)$NR^9R^{10}$, wherein $R^9$ and $R^{10}$ independently of one another are chosen from

H; $C_1$-$C_4$-alkyl, branched or unbranched and mono- or polysubstituted or unsubstituted;

wherein particularly preferably R1 = R5, where R5 is chosen from

H, F, Cl, OH, $CH_3$, $C_2H_5$, $C_2H_3$, $CHF_2$, $CF_3$, $SCH_3$, $OCF_3$, $OCH_3$, $OC_2H_5$, C(O)$OCH_3$, C(O)$OC_2H_5$ or phenyl

while $R^2$, X, $R^3$ and $R^4$ have one of the meanings mentioned in the preceding claims.

3. Aminomethyl-phenyl-cyclohexanone derivatives according to one of claims 1 or 2, **characterized in that** $R^2$ = $R^5$, wherein $R^5$ is chosen from

H, F, Cl, Br, I, $SCH_3$; $C_1$-$C_4$-alkyl, $C_2$-$C_4$-alkenyl, branched or unbranched and mono- or polysubstituted or

unsubstituted, preferably $CF_3$; $OR^7$, where $R^7$ is chosen from $C_1$-$C_4$-alkyl, branched or unbranched and mono- or polysubstituted or unsubstituted, preferably $CH_3$;

while $R^1$, X, $R^3$ and $R^4$ have one of the meanings mentioned in the preceding claims.

**4.** Aminomethyl-phenyl-cyclohexanone derivatives according to one of claims 1 to 3, **characterized in that** $R^1$ and $R^2$ have different meanings or

$R^1$ and $R^2$ together form -CH=CH-CH=CH-, wherein the naphthyl system formed can be mono- or polysubstituted, preferably by halogen, $OC_{1-3}$-alkyl or $C_{1-3}$-alkyl, unsubstituted or mono or polysubstituted, in particular by $OCH_3$.

**5.** Aminomethyl-phenyl-cyclohexanone derivatives according to one of claims 1 to 4, **characterized in that** X is chosen from

H, F, Cl, OH, $CF_3$, O-S($O_2$)-$C_6H_4$-p$CH_3$ or OC(O)$R^7$, where $R^7$ = H; $C_1$-$C_4$-alkyl or $C_2$-$C_4$-alkenyl, branched or unbranched and mono- or polysubstituted or unsubstituted, preferably H, F, Cl, OH, O-S($O_2$)-$C_6H_4$-p$CH_3$ or OC (O)$R^7$, where $R^7$ = $C_1$-$C_4$-alkyl, preferably $CH_3$, in particular H or OH,

or

if the compound contains no X, according to formula Ia a double bond is formed between C atom A and C atom B or C atom B and C atom C,

while $R^1$, $R^2$, $R^3$ and $R^4$ have one of the meanings mentioned in the preceding claims.

**6.** Aminomethyl-phenyl-cyclohexanone derivatives according to one of claims 1 to 5, **characterized in that** $R^3$ and $R^4$ independently of one another are chosen from $C_1$-$C_4$-alkyl, branched or unbranched and mono- or polysubstituted or unsubstituted, preferably $CH_3$,

or

$R^3$ and $R^4$ together form a $C_3$-$C_7$-cycloalkyl, saturated or unsaturated and mono- or polysubstituted or unsubstituted,

while $R^1$, $R^2$ and X have one of the meanings mentioned in the preceding claims.

**7.** Process for the preparation of aminomethyl-phenyl-cyclohexanone derivatives according to claim 1 according to formula Ia or according to formula I where X = H, **characterized in that** compounds of the formula II

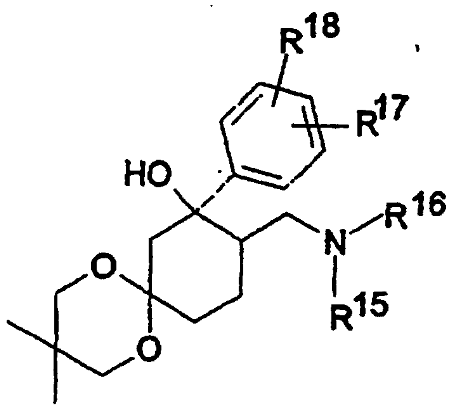

II

in which $R^{15}$ has a meaning corresponding to $R^3$, $R^{16}$ to $R^4$, $R^{17}$ to $R^1$ and $R^{18}$ to $R^2$ according to claim 1, are reacted with acids, preferably hydrochloric acid, formic acid or acetic acid, at room temperature and the product according to formula Ia is then either purified or hydrogenated with catalytically activated hydrogen, preferably with platinum or palladium as a catalyst absorbed on a support material, such as active charcoal, preferably in ethyl acetate or a $C_1$ - $C_4$-alkyl alcohol, under pressures of 0.1 to 10 bar and/or at temperatures of 20°C to 80°C, and the product according to formula I where X = H is then purified.

**8.** Process for the preparation of aminomethyl-phenyl-cyclohexanone derivatives according to claim 1 according to

formula I where X ≠ H, **characterized in that** compounds of the formula II

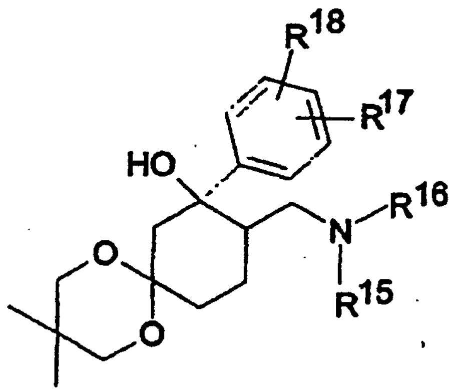

**II**

in which $R^{15}$ has a meaning corresponding to $R^3$, $R^{16}$ to $R^4$ , $R^{17}$ to $R^1$ and $R^{18}$ to $R^2$ according to claim 1, are reacted with acids, preferably hydrochloric acid, at temperatures of between 0°C and 5°C and the products according to formula I formed in this way, where X = OH and $R^{15}$ to $R^{18}$ are unchanged, are either purified or further processed, wherein, if X is to be F, Cl, Br, I or $CF_3$, the X = OH group is exchanged for F or Cl or Br or I or $CF_3$, and if X is to be $OR^{13}$, wherein $R^{13}$ has one of the meanings described in claim 1, the X = OH group is etherified with a halide of the formula III

$$R^{19}Cl$$

**III**

wherein $R^{19}$ has a meaning analogous to $R^{13}$, or if X = $O\text{-}S(O_2)\text{-}C_6H_4\text{-}CH_3$ or $OC(O)R^{13}$, wherein $R^{13}$ has one of the meanings described in claim 1, the X = OH group is esterified with an acid chloride of the formula IV

$$R^{20}COCl$$

**IV**

or $Cl\text{-}S(O_2)\text{-}C_6H_4\text{-}CH_3$, wherein $R^{20}$ has a meaning analogous to $R^{13}$, and finally the product is purified.

**9.** Process according to one of claims 7 or 8, **characterized in that** to prepare the starting substance according to formula II, 3,3-dimethyl-1,5-dioxa-spiro[5.5]-undecan-8-one

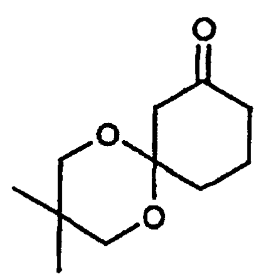

is first reacted with immonium salts of the formula V or with formaldehyde and an amine of the formula VI, wherein $R^{15}$ has a meaning corresponding to $R^3$ and $R^{16}$ to $R^4$ according to claim 1,

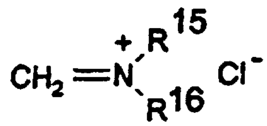

V

$R^{15}R^{16}NH$ VI

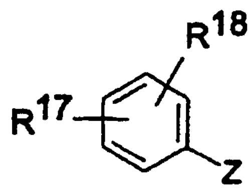

VII

and the Mannich bases obtained in this way are then reacted with an organometallic compound of the formula VII, in which Z denotes MgCl, MgBr, MgI or lithium and $R^{17}$ has a meaning corresponding to $R^1$ and $R^{18}$ to $R^2$ according to claim 1, at temperatures of between -70°C and 60°C, preferably in diethyl ether and/or tetrahydrofuran.

**10.** Process according to one of claims 7 to 9, **characterized in that** before the concluding purification a process product with at least one $C(O)OCH_3$, $OC(O)OCH_3$ and/or $C(S)OCH_3$ group is hydrolysed with KOH solution or NaOH solution in methanol at 40°C - 60°C.

**11.** Process according to one of claims 7 to 10, **characterized in that** in $R^{15}$ to $R^{20}$ according to formulae II to VII at least one OH group has been replaced by an $OSi(Ph)_2$tert-but group, at least one SH group has been replaced by an S-p-methoxybenzyl group and/or at least one $NH_2$ group has been replaced by an $NO_2$ group and, directly before purification of the end product, at least one $OSi(Ph)_2$tert-but group is split off with tetrabutylammonium fluoride in tetrahydrofuran and/or at least one p-methoxybenzyl group is split off with a metal amine, preferably sodium amine, and/or at least one $NO_2$ group is reduced to $NH_2$.

**12.** Medicaments comprising as the active compound at least one aminomethyl-phenyl-cyclohexanone derivative of the general formula I or Ia

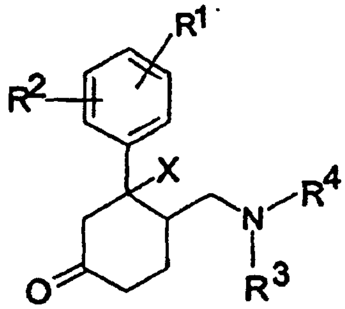

I

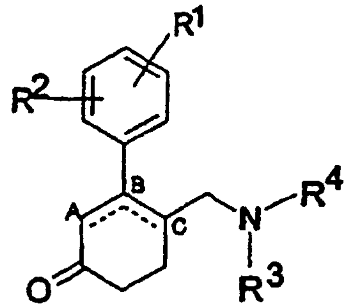

Ia

wherein

$R^1$ and $R^2$ independently of one another are chosen from $R^5$ or $YR^5$, where Y = $C_1$-$C_{10}$-alkyl, $C_2$-$C_{10}$-alkenyl or $C_2$-$C_{10}$-alkinyl, branched or unbranched and mono- or polysubstituted or unsubstituted, wherein $R^5$ is chosen from

H, F, Cl, Br, I, CN, $NO_2$, $C_1$-$C_8$-alkyl, $C_2$-$C_8$-alkenyl or $C_2$-$C_8$-alkinyl, in each case branched or unbranched and mono- or polysubstituted or unsubstituted; $C_3$-$C_7$-cycloalkyl, saturated or unsaturated and mono- or polysubstituted or unsubstituted, or a corresponding heterocyclic radical, in which one C atom in the ring is replaced by S, O or $NR^6$, where $R^6$ is chosen from

H, $C_1$-$C_{10}$-alkyl, $C_2$-$C_{10}$-alkenyl or $C_2$-$C_{10}$-alkinyl, in each case branched or unbranched and mono- or polysubstituted or unsubstituted;

aryl or heteroaryl, in each case mono- or polysubstituted or unsubstituted;

$OR^7$, $OC(O)R^7$, $OC(O)OR^7$, $OC(S)R^7$, $C(O)R^7$, $C(O)OR^7$, $C(S)R^7$, $C(S)OR^7$, $SR^7$, $S(O)R^7$ or $S(O_2)R^7$, wherein $R^7$ is chosen from

H, $C_1$-$C_{18}$-alkyl, $C_2$-$C_{18}$-alkenyl or $C_2$-$C_{18}$-alkinyl, in each case branched or unbranched and mono- or polysubstituted or unsubstituted; $C_3$-$C_7$-cycloalkyl, saturated or unsaturated and mono- or polysubstituted or unsubstituted, or a corresponding heterocyclic radical, in which one C atom in the ring is replaced by S, O or $NR^8$, where $R^8$ is chosen from

H, $C_1$-$C_{10}$-alkyl, $C_2$-$C_{10}$-alkenyl or $C_2$-$C_{10}$-alkinyl, in each case branched or unbranched and mono- or polysubstituted or unsubstituted;

alkylaryl, saturated or unsaturated and mono- or polysubstituted or unsubstituted; aryl or heteroaryl, in each case mono- or polysubstituted or unsubstituted; or

$NR^9R^{10}$, $C(O)NR^9R^{10}$ or $S(O_2)NR^9R^{10}$, wherein $R^9$ and $R^{10}$ independently of one another are chosen from

H, $C_1$-$C_{18}$-alkyl, $C_2$-$C_{18}$-alkenyl or $C_2$-$C_{18}$-alkinyl, in each case branched or unbranched and mono- or polysubstituted or unsubstituted; $C_3$-$C_7$-cycloalkyl, saturated or unsaturated and mono- or polysubstituted or unsubstituted, or a corresponding heterocyclic radical, in which one C atom in the ring is replaced by S, 0 or $NR^{11}$, where

$R^{11}$ is chosen from

H, $C_1$-$C_{10}$-alkyl, $C_2$-$C_{10}$-alkenyl or $C_2$-$C_{10}$-alkinyl, in each case branched or unbranched and mono- or polysubstituted or unsubstituted;

alkylaryl, saturated or unsaturated and mono- or polysubstituted or unsubstituted; aryl or heteroaryl, in each case mono- or polysubstituted or unsubstituted;

or

$R^9$ and $R^{10}$ together form a $C_3$-$C_7$-cycloalkyl, saturated or unsaturated and mono- or polysubstituted or unsubstituted, or a corresponding heterocyclic radical, in which one C atom in the ring is replaced by S, O or $NR^{12}$, where $R^{12}$ is chosen from

H, $C_1$-$C_{10}$-alkyl, $C_2$-$C_{10}$-alkenyl or $C_2$-$C_{10}$-alkinyl, in each case branched or unbranched and mono- or polysubstituted or unsubstituted;

or

$R^1$ and $R^2$ together form -CH=CH-CH=CH-, wherein the naphthyl system formed can be mono- or polysubstituted,

X is chosen from

H, F, Cl, Br, I, $CF_3$, O-$S(O_2)$-$C_6H_4$-$pCH_3$, $OR^{13}$ or $OC(O)R^{13}$, wherein $R^{13}$ is chosen from

H; $C_1$-$C_{10}$-alkyl, $C_2$-$C_{10}$-alkenyl or $C_2$-$C_{10}$-alkinyl, in each case branched or unbranched and mono- or polysubstituted or unsubstituted; $C_3$-$C_7$-cycloalkyl, saturated or unsaturated and mono- or polysubstituted or unsubstituted, or a corresponding heterocyclic radical, in which one C atom in the ring is replaced by N, S or O;

alkylaryl, saturated or unsaturated and mono- or polysubstituted or unsubstituted; aryl or heteroaryl, in each case mono- or polysubstituted or unsubstituted;

or

if the compound contains no X, according to formula Ia a double bond is formed between C atom A and C atom B or C atom B and C atom C;

and

$R^3$, $R^4$ independently of one another are chosen from

H, $C_1$-$C_{10}$-alkyl, $C_2$-$C_{10}$-alkenyl or $C_2$-$C_{10}$-alkinyl, in each case branched or unbranched and mono- or polysubstituted or unsubstituted; $C_3$-$C_7$-cycloalkyl, saturated or unsaturated and mono- or polysubstituted or unsubstituted, or a corresponding heterocyclic radical, in which one C atom in the ring is replaced by N, S or O; alkylaryl, saturated or unsaturated and mono- or polysubstituted or unsubstituted; aryl or heteroaryl, in each case mono- or polysubstituted or unsubstituted;

or

$R^3$ and $R^4$ together form a $C_3$-$C_7$-cycloalkyl, saturated or unsaturated and mono- or polysubstituted or unsubstituted, or a corresponding heterocyclic radical, in which one C atom in the ring is replaced by S, O or $NR^{14}$, where $R^{14}$ is chosen from

H, $C_1$-$C_{10}$-alkyl, $C_2$-$C_{10}$-alkenyl or $C_2$-$C_{10}$-alkinyl, in each case branched or unbranched and mono- or polysubstituted or unsubstituted;

in the form of its diastereomers or enantiomers and of its free base or of a salt formed with a physiologically tolerated acid, in particular the hydrochloride salt.

13. Medicaments according to claim 12, **characterized in that**, independently of one another in the general formulae I and Ia

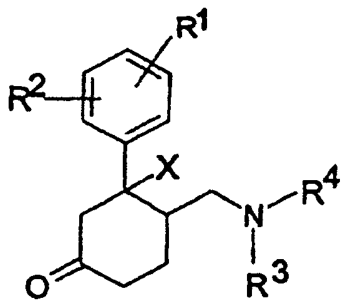

I

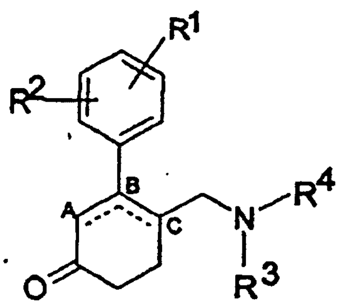

Ia

$R^1 = R^5$, wherein $R^5$ is chosen from

H, F, Cl, Br, I, $CHF_2$, $CF_3$, $NO_2$, $NH_2$; $C_1$-$C_4$-alkyl or $C_2$-$C_4$-alkenyl, branched or unbranched and mono- or polysubstituted or unsubstituted; aryl, substituted or unsubstituted; $OR^7$, $C(O)OR^7$ or $SR^7$, wherein $R^7$ is chosen from

H; $C_1$-$C_4$-alkyl, branched or unbranched and mono- or polysubstituted or unsubstituted; preferably H, $CF_3$ or $CH_3$,

or $S(O_2)NR^9R^{10}$, wherein $R^9$ and $R^{10}$ independently of one another are chosen from

H; $C_1$-$C_4$-alkyl, branched or unbranched and mono- or polysubstituted or unsubstituted;

wherein particularly preferably R1 = R5, where R5 is chosen from

H, F, Cl, OH, $CH_3$, $C_2H_5$, $C_2H_3$, $CHF_2$, $CF_3$, $SCH_3$, $OCF_3$, $OCH_3$, $OC_2H_5$, $C(O)OCH_3$, $C(O)OC_2H_5$ or phenyl,

**and/or** $R^2 = R^5$, wherein $R^5$ is chosen from

H, F, Cl, Br, I, $SCH_3$; $C_1$-$C_4$-alkyl, $C_2$-$C_4$-alkenyl, branched or unbranched and mono- or polysubstituted or

unsubstituted, preferably $CF_3$; $OR^7$, where $R^7$ is chosen from $C_1$-$C_4$-alkyl, branched or unbranched and mono- or polysubstituted or unsubstituted, preferably $CH_3$;

**and/or** $R^1$ and $R^2$ have different meanings or $R^1$ and $R^2$ together form -CH=CH-CH=CH-, wherein the naphthyl system formed can be mono- or polysubstituted, preferably by halogen, $OC_{1-3}$-alkyl or $C_{1-3}$-alkyl, unsubstituted or mono or polysubstituted, in particular by $OCH_3$,

**and/or** X is chosen from

H, F, Cl, OH, $CF_3$, O-S($O_2$)-$C_6H_4$-p$CH_3$ or OC(O)$R^7$, where $R^7$ = H; $C_1$-$C_4$-alkyl or $C_2$-$C_4$-alkenyl, branched or unbranched and mono- or polysubstituted or unsubstituted,

preferably H, F, Cl, OH, O-S($O_2$)-$C_6H_4$-p$CH_3$ or OC(O)$R^7$, where $R^7$ = $C_1$-$C_4$-alkyl, preferably $CH_3$, in particular H or OH,

or

if the compound contains no X, according to formula Ia a double bond is formed between C atom A and C atom B or C atom B and C atom C,

**and/or** $R^3$ and $R^4$ independently of one another are chosen from

$C_1$-$C_4$-alkyl, branched or unbranched and mono- or polysubstituted or unsubstituted, preferably $CH_3$;

or

$R^3$ and $R^4$ together form a $C_3$-$C_7$-cycloalkyl, saturated or unsaturated and mono- or polysubstituted or unsubstituted,

**and/or** $R^1$, $R^2$, X, $R^3$ and/or $R^4$ have one of the meanings mentioned in the preceding claims.

**14.** Medicaments according to claim 12, comprising as the active compound at least one aminomethyl-phenyl-cyclohexanone derivative chosen from the group consisting of

- *rac-cis*-[4-dimethylaminomethyl-3-hydroxy-3-(3-methoxyphenyl)-cyclohexanone],
- *rac-cis*-[4-dimethylaminomethyl-3-(3-methoxy-phenyl)-cyclohexanone],
- *rac-trans*-[4-dimethylaminomethyl-3-(3-methoxy-phenyl)-cyclohexanone],
- *rac-cis*-[4-dimethylaminomethyl-3-(3-hydroxy-phenyl)-cyclohexanone],
- *rac-trans*-[4-dimethylaminomethyl-3-(3-hydroxy-phenyl)-cyclohexanone] or
- 4-dimethylaminomethyl-3-(3-methoxy-phenyl)-cyclohex-2-enone
- 4-dimethylaminomethyl-3-phenyl-cyclohex-2-enone;
- 4-dimethylaminomethyl-3-(3-hydroxy-phenyl)-cyclohex-2-enone;
- rac-trans-4-dimethylaminomethyl-3-phenylcyclohexanone;
- rac-cis-4-dimethylaminomethyl-3-phenyl-cyclohexanone;
- 4-dimethylaminomethyl-3-(4-methoxy-phenyl)-cyclohex-2-enone;
- 4-dimethylaminomethyl-3-naphthalen-2-yl-cyclohex-2-enone;
- rac-trans-4-dimethylaminomethyl-3-hydroxy-3-naphthalen-2-yl-cyclohexanone;
- rac-trans-4-dimethylaminomethyl-3-(3-fluoro-phenyl)-cyclohexanone;
- rac-cis-4-dimethylaminomethyl-3-(3-fluoro-phenyl)-cyclohexanone;
- rac-cis-4-dimethylaminomethyl-3-hydroxy-3-(2-methoxyphenyl)-cyclohexanone
- rac-cis-4-dimethylaminomethyl-3-hydroxy-3-(6-methoxynaphthalen-2-yl)-cyclohexanone;
- 4-dimethylaminomethyl-3-(6-methoxy-naphthalen-2-yl)-cyclohex-2-enone;
- rac-cis-3-biphenyl-4-yl-4-dimethylaminomethyl-3-hydroxy-cyclohexanone
- 3-(3-difluoromethyl-phenyl)-4-dimethylaminomethylcyclohex-2-enone;
- 4-dimethylaminomethyl-3-(3-fluoro-phenyl)-cyclohex-2-enone;
- 3-(3,4-difluoro-phenyl)-4-dimethylaminomethylcyclohex-2-enone;
- 4-dimethylaminomethyl-3-(4-fluoro-phenyl)-cyclohex-2-enone;
- 4-dimethylaminomethyl-3-(4-trifluoromethyl-phenyl)-cyclohex-2-enone;
- 3-(3,5-dichloro-phenyl)-4-dimethylaminomethylcyclohex-2-enone;
- 3-(3,5-difluoro-phenyl)-4-dimethylaminomethylcyclohex-2-enone;
- rac-cis-3-(3,5-difluoro-phenyl)-4-dimethylaminomethyl-3-hydroxy-cyclohexanone

as the free base or in the form of a salt formed with a physiologically tolerated acid, in particular the hydrochloride salt.

**15.** Use of at least one aminomethyl-phenyl-cyclohexanone derivative of the general formula I or Ia

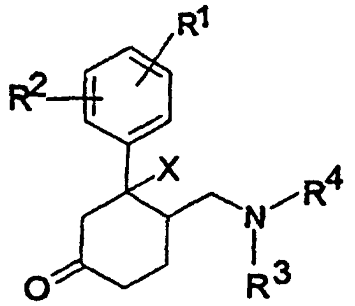

I

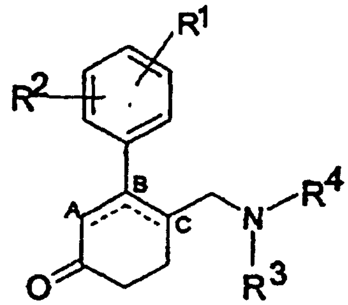

Ia

wherein

$R^1$ and $R^2$ independently of one another are chosen from $R^5$ or $YR^5$, where Y = $C_1$-$C_{10}$-alkyl, $C_2$-$C_{10}$-alkenyl or $C_2$-$C_{10}$-alkinyl, branched or unbranched and mono- or polysubstituted or unsubstituted, wherein $R^5$ is chosen from

H, F, Cl, Br, I, CN, $NO_2$, $C_1$-$C_8$-alkyl, $C_2$-$C_8$-alkenyl or $C_2$-$C_8$-alkinyl, in each case branched or unbranched and mono- or polysubstituted or unsubstituted; $C_3$-$C_7$-cycloalkyl, saturated or unsaturated and mono- or polysubstituted or unsubstituted, or a corresponding heterocyclic radical, in which one C atom in the ring is replaced by S, O or $NR^6$, where $R^6$ is chosen from

H, $C_1$-$C_{10}$-alkyl, $C_2$-$C_{10}$-alkenyl or $C_2$-$C_{10}$-alkinyl, in each case branched or unbranched and mono- or polysubstituted or unsubstituted;

aryl or heteroaryl, in each case mono- or polysubstituted or unsubstituted;

$OR^7$, $OC(O)R^7$, $OC(O)OR^7$, $OC(S)R^7$, $C(O)R^7$, $C(O)OR^7$, $C(S)R^7$, $C(S)OR^7$, $SR^7$, $S(O)R^7$ or $S(O_2)R^7$, wherein $R^7$ is chosen from

H, $C_1$-$C_{18}$-alkyl, $C_2$-$C_{18}$-alkenyl or $C_2$-$C_{18}$-alkinyl, in each case branched or unbranched and mono- or polysubstituted or unsubstituted; $C_3$-$C_7$-cycloalkyl, saturated or unsaturated and mono- or polysubstituted or unsubstituted, or a corresponding heterocyclic radical, in which one C atom in the ring is replaced by S, O or $NR^8$, where $R^8$ is chosen from

H, $C_1$-$C_{10}$-alkyl, $C_2$-$C_{10}$-alkenyl or $C_2$-$C_{10}$-alkinyl, in each case branched or unbranched and mono- or polysubstituted or unsubstituted;

alkylaryl, saturated or unsaturated and mono- or polysubstituted or unsubstituted; aryl or heteroaryl, in each case mono- or polysubstituted or unsubstituted; or

$NR^9R^{10}$, $C(O)NR^9R^{10}$ or $S(O_2)NR^9R^{10}$, wherein $R^9$ and $R^{10}$ independently of one another are chosen from

H, $C_1$-$C_{18}$-alkyl, $C_2$-$C_{18}$-alkenyl or $C_2$-$C_{18}$-alkinyl, in each case branched or unbranched and mono- or polysubstituted or unsubstituted; $C_3$-$C_7$-cycloalkyl, saturated or unsaturated and mono- or polysubstituted or unsubstituted, or a corresponding heterocyclic radical, in which one C atom in the ring is replaced by S, O or $NR^{11}$, where $R^{11}$ is chosen from

H, $C_1$-$C_{10}$-alkyl, $C_2$-$C_{10}$-alkenyl or $C_2$-$C_{10}$-alkinyl, in each case branched or unbranched and mono- or polysubstituted or unsubstituted;

alkylaryl, saturated or unsaturated and mono- or polysubstituted or unsubstituted; aryl or heteroaryl, in each case mono- or polysubstituted or unsubstituted;

or

$R^9$ and $R^{10}$ together form a $C_3$-$C_7$-cycloalkyl, saturated or unsaturated and mono- or polysubstituted or unsubstituted, or a corresponding heterocyclic radical, in which one C atom in the ring is replaced by S, O or $NR^{12}$, where $R^{12}$ is chosen from

H, $C_1$-$C_{10}$-alkyl, $C_2$-$C_{10}$-alkenyl or $C_2$-$C_{10}$-alkinyl, in each case branched or unbranched and mono- or polysubstituted or unsubstituted;

or

$R^1$ and $R^2$ together form -CH=CH-CH=CH-, wherein the naphthyl system formed can be mono- or polysubstituted,

X is chosen from

H, F, Cl, Br, I, $CF_3$, O-$S(O_2)$-$C_6H_4$-p$CH_3$, $OR^{13}$ or $OC(O)R^{13}$, wherein $R^{13}$ is chosen from

H; $C_1$-$C_{10}$-alkyl, $C_2$-$C_{10}$-alkenyl or $C_2$-$C_{10}$-alkinyl, in each case branched or unbranched and mono- or

polysubstituted or unsubstituted; $C_3$-$C_7$-cycloalkyl, saturated or unsaturated and mono- or polysubstituted or unsubstituted, or a corresponding heterocyclic radical, in which one C atom in the ring is replaced by N, S or O;

alkylaryl, saturated or unsaturated and mono- or polysubstituted or unsubstituted; aryl or heteroaryl, in each case mono- or polysubstituted or unsubstituted;

or

if the compound contains no X, according to formula Ia a double bond is formed between C atom A and C atom B or C atom B and C atom C;

and

$R^3$, $R^4$ independently of one another are chosen from

H, $C_1$-$C_{10}$-alkyl, $C_2$-$C_{10}$-alkenyl or $C_2$-$C_{10}$-alkinyl, in each case branched or unbranched and mono- or polysubstituted or unsubstituted; $C_3$-$C_7$-cycloalkyl, saturated or unsaturated and mono- or polysubstituted or unsubstituted, or a corresponding heterocyclic radical, in which one C atom in the ring is replaced by N, S or O; alkylaryl, saturated or unsaturated and mono- or polysubstituted or unsubstituted; aryl or heteroaryl, in each case mono- or polysubstituted or unsubstituted;

or

$R^3$ and $R^4$ together form a $C_3$-$C_7$-cycloalkyl, saturated or unsaturated and mono- or polysubstituted or unsubstituted, or a corresponding heterocyclic radical, in which one C atom in the ring is replaced by S, O or $NR^{14}$, where $R^{14}$ is chosen from

H, $C_1$-$C_{10}$-alkyl, $C_2$-$C_{10}$-alkenyl or $C_2$-$C_{10}$-alkinyl, in each case branched or unbranched and mono- or polysubstituted or unsubstituted;

in the form of its diastereomers or enantiomers and of its free base or of a salt formed with a physiologically tolerated acid, in particular the hydrochloride salt, for the preparation of a medicament for treatment of pain.

**16.** Use of at least one aminomethyl-phenyl-cyclohexanone derivative of the general formula I or Ia

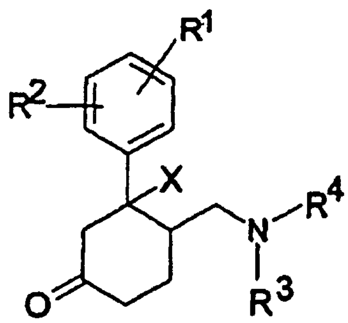

I

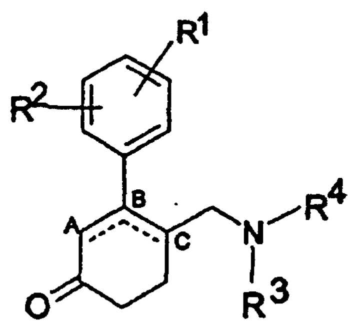

Ia

wherein

$R^1$ and $R^2$ independently of one another are chosen from $R^5$ or $YR^5$, where Y = $C_1$-$C_{10}$-alkyl, $C_2$-$C_{10}$-alkenyl or $C_2$-$C_{10}$-alkinyl, branched or unbranched and mono- or polysubstituted or unsubstituted, wherein $R^5$ is chosen from

H, F, Cl, Br, I, CN, $NO_2$, $C_1$-$C_8$-alkyl, $C_2$-$C_8$-alkenyl or $C_2$-$C_8$-alkinyl, in each case branched or unbranched and mono- or polysubstituted or unsubstituted; $C_3$-$C_7$-cycloalky, saturated or unsaturated and mono- or polysubstituted or unsubstituted, or a corresponding heterocyclic radical, in which one C atom in the ring is replaced by S, O or $NR^6$, where $R^6$ is chosen from

H, $C_1$-$C_{10}$-alkyl, $C_2$-$C_{10}$-alkenyl or $C_2$-$C_{10}$-alkinyl, in each case branched or unbranched and mono- or polysubstituted or unsubstituted;

aryl or heteroaryl, in each case mono- or polysubstituted or unsubstituted;

$OR^7$, $OC(O)R^7$, $OC(O)OR^7$, $OC(S)R^7$, $C(O)R^7$, $C(O)OR^7$, $C(S)R^7$, $C(S)OR^7$, $SR^7$, $S(O)R^7$ or $S(O_2)R^7$, wherein $R^7$ is chosen from

H, $C_1$-$C_{18}$-alkyl, $C_2$-$C_{18}$-alkenyl or $C_2$-$C_{18}$-alkinyl, in each case branched or unbranched and mono- or

polysubstituted or unsubstituted; $C_3$-$C_7$-cycloalkyl, saturated or unsaturated and mono- or polysubstituted or unsubstituted, or a corresponding heterocyclic radical, in which one C atom in the ring is replaced by S, O or $NR^8$, where $R^8$ is chosen from

H, $C_1$-$C_{10}$-alkyl, $C_2$-$C_{10}$-alkenyl or $C_2$-$C_{10}$-alkinyl, in each case branched or unbranched and mono- or polysubstituted or unsubstituted;

alkylaryl, saturated or unsaturated and mono- or polysubstituted or unsubstituted; aryl or heteroaryl, in each case mono- or polysubstituted or unsubstituted; or

$NR^9R^{10}$, $C(O)NR^9R^{10}$ or $S(O_2)NR^9R^{10}$, wherein $R^9$ and $R^{10}$ independently of one another are chosen from

H, $C_1$-$C_{18}$-alkyl, $C_2$-$C_{18}$-alkenyl or $C_2$-$C_{18}$-alkinyl, in each case branched or unbranched and mono- or polysubstituted or unsubstituted; $C_3$-$C_7$-cycloalkyl, saturated or unsaturated and mono- or polysubstituted or unsubstituted, or a corresponding heterocyclic radical, in which one C atom in the ring is replaced by S, O or $NR^{11}$, where $R^{11}$ is chosen from

H, $C_1$-$C_{10}$-alkyl, $C_2$-$C_{10}$-alkenyl or $C_2$-$C_{10}$-alkinyl, in each case branched or unbranched and mono- or polysubstituted or unsubstituted;

alkylaryl, saturated or unsaturated and mono- or polysubstituted or unsubstituted; aryl or heteroaryl, in each case mono- or polysubstituted or unsubstituted;

or

$R^9$ and $R^{10}$ together form a $C_3$-$C_7$-cycloalkyl, saturated or unsaturated and mono- or polysubstituted or unsubstituted, or a corresponding heterocyclic radical, in which one C atom in the ring is replaced by S, O or $NR^{12}$, where $R^{12}$ is chosen from

H, $C_1$-$C_{10}$-alkyl, $C_2$-$C_{10}$-alkenyl or $C_2$-$C_{10}$-alkinyl, in each case branched or unbranched and mono- or polysubstituted or unsubstituted;

or

$R^1$ and $R^2$ together form -CH=CH-CH=CH-, wherein the naphthyl system formed can be mono- or polysubstituted,

X is chosen from

H, F, Cl, Br, I, $CF_3$, O-S($O_2$)-$C_6H_4$-p$CH_3$, $OR^{13}$ or $OC(O)R^{13}$, wherein $R^{13}$ is chosen from

H; $C_1$-$C_{10}$-alkyl, $C_2$-$C_{10}$-alkenyl or $C_2$-$C_{10}$-alkinyl, in each case branched or unbranched and mono- or polysubstituted or unsubstituted; $C_3$-$C_7$-cycloalkyl, saturated or unsaturated and mono- or polysubstituted or unsubstituted, or a corresponding heterocyclic radical, in which one C atom in the ring is replaced by N, S or O; alkylaryl, saturated or unsaturated and mono- or polysubstituted or unsubstituted; aryl or heteroaryl, in each case mono- or polysubstituted or unsubstituted;

or

if the compound contains no X, according to formula Ia a double bond is formed between C atom A and C atom B or C atom B and C atom C;

and

$R^3$, $R^4$ independently of one another are chosen from

H, $C_1$-$C_{10}$-alkyl, $C_2$-$C_{10}$-alkenyl or $C_2$-$C_{10}$-alkinyl, in each case branched or unbranched and mono- or polysubstituted or unsubstituted; $C_3$-$C_7$-cycloalkyl, saturated or unsaturated and mono- or polysubstituted or unsubstituted, or a corresponding heterocyclic radical, in which one C atom in the ring is replaced by N, S or O; alkylaryl, saturated or unsaturated and mono- or polysubstituted or unsubstituted; aryl or heteroaryl, in each case mono- or polysubstituted or unsubstituted;

or

$R^3$ and $R^4$ together form a $C_3$-$C_7$-cycloalkyl, saturated or unsaturated and mono- or polysubstituted or unsubstituted, or a corresponding heterocyclic radical, in which one C atom in the ring is replaced by S, 0 or $NR^{14}$, where $R^{14}$ is chosen from

H, $C_1$-$C_{10}$-alkyl, $C_2$-$C_{10}$-alkenyl or $C_2$-$C_{10}$-alkinyl, in each case branched or unbranched and mono- or polysubstituted or unsubstituted;

in the form of its diastereomers or enantiomers and of its free base or of a salt formed with a physiologically tolerated acid, in particular the hydrochloride salt, for the preparation of a medicament for treatment of inflammatory and allergic reactions, depressions, drug and/or alcohol abuse, gastritis, cardiovascular diseases, respiratory tract diseases, coughing, mental illnesses and/or epilepsy, and in particular urinary incontinence, itching and/or diarrhoea.

**17.** Use according to one of claims 15 or 16, **characterized in that**, independently of one another, in the general formulae I and Ia

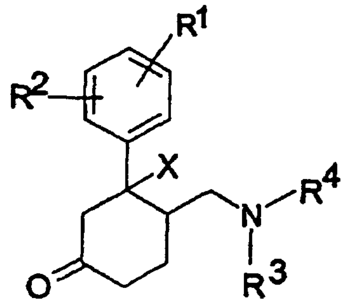

I

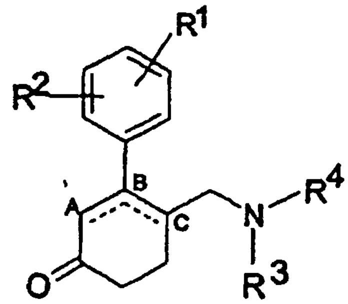

Ia

$R^1 = R^5$, wherein $R^5$ is chosen from
H, F, Cl, Br, I, $CHF_2$, $CF_3$, $NO_2$, $NH_2$; $C_1$-$C_4$-alkyl or $C_2$-$C_4$-alkenyl, branched or unbranched and mono- or polysubstituted or unsubstituted; aryl, substituted or unsubstituted; $OR^7$, $C(O)OR^7$ or $SR^7$, wherein $R^7$ is chosen from
H; $C_1$-$C_4$-alkyl, branched or unbranched and mono- or polysubstituted or unsubstituted; preferably H, $CF_3$ or $CH_3$,
or $S(O_2)NR^9R^{10}$, wherein $R^9$ and $R^{10}$ independently of one another are chosen from
H; $C_1$-$C_4$-alkyl, branched or unbranched and mono- or polysubstituted or unsubstituted;
wherein particularly preferably R1 = R5, where R5 is chosen from
H, F Cl, OH, $CH_3$, $C_2H_5$, $C_2H_3$, $CHF_2$, $CF_3$, $SCH_3$, $OCF_3$, $OCH_3$, $OC_2H_5$, $C(O)OCH_3$, $C(O)OC_2H_5$ or phenyl,
**and/or** $R^2 = R^5$, wherein $R^5$ is chosen from
H, F, Cl, Br, I, $SCH_3$; $C_1$-$C_4$-alkyl, $C_2$-$C_4$-alkenyl, branched or unbranched and mono- or polysubstituted or unsubstituted, preferably $CF_3$; $OR^7$, where $R^7$ is chosen from $C_1$-$C_4$-alkyl, branched or unbranched and mono- or polysubstituted or unsubstituted, preferably $CH_3$;
**and/or** $R^1$ and $R^2$ have different meanings or $R^1$ and $R^2$ together form -CH=CH-CH=CH-, wherein the naphthyl system formed can be mono- or polysubstituted, preferably by halogen, $OC_{1-3}$-alkyl or $C_{1-3}$-alkyl, unsubstituted or mono or polysubstituted, in particular by $OCH_3$,
**and/or** X is chosen from
H, F, Cl, OH, $CF_3$, O-$S(O_2)$-$C_6H_4$-p$CH_3$ or $OC(O)R^7$, where $R^7$ = H; $C_1$-$C_4$-alkyl or $C_2$-$C_4$-alkenyl, branched or unbranched and mono- or polysubstituted or unsubstituted, preferably H, F, Cl, OH, O-$S(O_2)$-$C_6H_4$-p$CH_3$ or OC(O)$R^7$, where $R^7$ = $C_1$-$C_4$-alkyl, preferably $CH_3$, in particular H or OH;
or
if the compound contains no X, according to formula Ia a double bond is formed between C atom A and C atom B or C atom B and C atom C,
**and/or** $R^3$ and $R^4$ independently of one another are chosen from
$C_1$-$C_4$-alkyl, branched or unbranched and mono- or polysubstituted or unsubstituted, preferably $CH_3$;
or
$R^3$ and $R^4$ together form a $C_3$-$C_7$-cycloalkyl, saturated or unsaturated and mono- or polysubstituted or unsubstituted,
**and/or** $R^1$, $R^2$, X, $R^3$ and/or $R^4$ have one of the meanings mentioned in the preceding claims.

**18.** Use according to one of claims 15 or 16, **characterized in that** at least one aminomethyl-phenyl-cyclohexanone derivative chosen from the group consisting of

- *rac-cis*-[4-dimethylaminomethyl-3-hydroxy-3-(3-methoxy-phenyl)-cyclohexanone],
- *rac-cis*-[4-dimethylaminomethyl-3-(3-methoxyphenyl)-cyclohexanone],
- *rac-trans*-[4-dimethylaminomethyl-3-(3-methoxyphenyl)-cyclohexanone],
- *rac-cis*-[4-dimethylaminomethyl-3-(3-hydroxyphenyl)-cyclohexanone],

- *rac-trans*-[4-dimethylaminomethyl-3-(3-hydroxyphenyl)-cyclohexanone) or
- 4-dimethylaminomethyl-3-(3-methoxy-phenyl)-cyclohex-2-enone
- 4-dimethylaminomethyl-3-phenyl-cyclohex-2-enone;
- 4-dimethylaminomethyl-3-(3-hydroxy-phenyl)-cyclohex-2-enone;
- rac-trans-4-dimethylaminomethyl-3-phenylcyclohexanone;
- rac-cis-4-dimethylaminomethyl-3-phenyl-cyclohexanone;
- 4-dimethylaminomethyl-3-(4-methoxy-phenyl)-cyclohex-2-enone;
- 4-dimethylaminomethyl-3-naphthalen-2-yl-cyclohex-2-enone;
- rac-trans-4-dimethylaminomethyl-3-hydroxy-3-naphthalen-2-yl-cyclohexanone;
- rac-trans-4-dimethylaminomethyl-3-(3-fluoro-phenyl)-cyclohexanone;
- rac-cis-4-dimethylaminomethyl-3-(3-fluoro-phenyl)-cyclohexanone;
- rac-cis-4-dimethylaminomethyl-3-hydroxy-3-(2-methoxyphenyl)-cyclohexanone
- rac-cis-4-dimethylaminomethyl-3-hydroxy-3-(6-methoxynaphthalen-2-yl)-cyclohexanone;
- 4-dimethylaminomethyl-3-(6-methoxy-naphthalen-2-yl)-cyclohex-2-enone;
- rac-cis-3-biphenyl-4-yl-4-dimethylaminomethyl-3-hydroxy-cyclohexanone
- 3- (3-difluoromethyl-phenyl)-4-dimethylaminomethylcyclohex-2-enone;
- 4-dimethylaminomethyl-3-(3-fluoro-phenyl)-cyclohex-2-enone;
- 3-(3,4-difluoro-phenyl)-4-dimethylaminomethylcyclohex-2-enone;
- 4-dimethylaminomethyl-3-(4-fluoro-phenyl)-cyclohex-2-enone;
- 4-dimethylaminomethyl-3-(4-trifluoromethyl-phenyl)-cyclohex-2-enone;
- 3-(3,5-dichloro-phenyl)-4-dimethylaminomethylcyclohex-2-enone;
- 3-(3,5-difluoro-phenyl)-4-dimethylaminomethylcyclohex-2-enone;
- rac-cis-3-(3,5-difluoro-phenyl)-4-dimethylaminomethyl-3-hydroxy-cyclohexanone

as the free base or in the form of a salt formed with a physiologically tolerated acid, in particular the hydrochloride salt, is used as the active compound.

**Revendications**

**1.** Dérivés d'aminométhyl-phényl-cyclohexanone de formule générale I ou Ia, également sous la forme de leurs diastéréoisomères ou de leurs énantiomères ainsi que de leur base libre ou d'un sel formé avec un acide acceptable du point de vue physiologique, en particulier sous la forme du chlorhydrate,

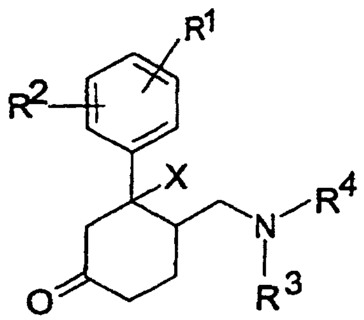

I

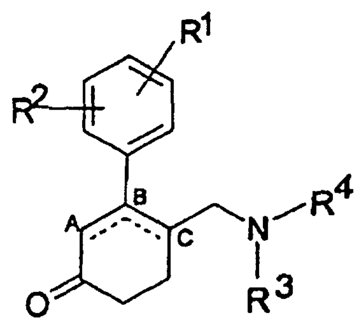

Ia

formules dans lesquelles
$R^1$ et $R^2$ sont choisis, indépendamment l'un de l'autre, parmi des restes $R^5$ ou $YR^5$ avec Y = alkyle en $C_1$ à $C_{10}$, alcényle en $C_2$ à $C_{10}$ ou alcynyle en $C_2$ à $C_{10}$, ramifié ou non ramifié, substitué une ou plusieurs fois ou non substitué, $R^5$ étant choisi entre

H, F, Cl, Br, I, CN, $NO_2$ ; alkyle en $C_1$ à $C_8$, alcényle en $C_2$ à $C_8$ ou alcynyle en $C_2$ à $C_8$, chacun ramifié ou non ramifié, substitué une ou plusieurs fois ou non substitué ; cycloalkyle en $C_3$ à $C_7$, saturé ou non saturé, substitué

une ou plusieurs fois ou non substitué, ou un hétérocycle correspondant dans lequel un atome de carbone du noyau est remplacé par S, O ou $NR^6$, $R^6$ étant choisi entre

H, alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ;

aryle ou hétéroaryle, chacun substitué une ou plusieurs fois ou non substitué ;

$OR^7$, $OC(O)R^7$, $OC(O)OR^7$, $OC(S)R^7$, $C(O)R^7$, $C(O)OR^7$, $C(S)R^7$, $C(S)OR^7$, $SR^7$, $S(O)R^7$ ou $S(O_2)R^7$, où $R^7$ est choisi entre

H, alkyle en $C_1$ à $C_{18}$, alcényle en $C_2$ à $C_{18}$ ou alcynyle en $C_2$ à $C_{18}$, chacun ramifié ou non ramifié, substitué une ou plusieurs fois ou non substitué ; cycloalkyle en $C_3$ à $C_7$, saturé ou non saturé, substitué une ou plusieurs fois ou non substitué, ou un hétérocycle correspondant, dans lequel un atome de carbone du noyau est remplacé par S, O ou $NR^8$, $R^8$ étant choisi entre

H, alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ;

alkylaryle, saturé ou non saturé, substitué une ou plusieurs fois ou non substitué ; aryle ou hétéroaryle, chacun substitué une ou plusieurs fois ou non substitué ; ou bien

$NR^9R^{10}$, $C(O)NR^9R^{10}$ ou $S(O_2)NR^9R^{10}$, $R^9$ et $R^{10}$ étant choisis indépendamment l'un de l'autre entre

H, alkyle en $C_1$ à $C_{18}$, alcényle en $C_2$ à $C_{18}$ ou alcynyle en $C_2$ à $C_{18}$, chacun ramifié ou non ramifié, substitué une ou plusieurs fois ou non substitué ; cycloalkyle en $C_3$ à $C_7$, saturé ou non saturé, substitué une ou plusieurs fois ou non substitué, ou un hétérocycle correspondant, dans lequel un atome de carbone du noyau est remplacé par S, O ou $NR^{11}$, $R^{11}$ étant choisi entre

H, alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ;

alkylaryle, saturé ou non saturé, substitué une ou plusieurs fois ou non substitué ; aryle ou hétéroaryle, chacun substitué une ou plusieurs fois ou non substitué ;

ou bien

$R^9$ et $R^{10}$ forment ensemble un reste cycloalkyle en $C_3$ à $C_7$, saturé ou non saturé, substitué une ou plusieurs fois ou non substitué, ou bien un hétérocycle correspondant dans lequel un atome de carbone du noyau est remplacé par S, O ou $NR^{12}$, $R^{12}$ étant choisi entre

H, alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ;

ou bien,

$R^1$ et $R^2$ forment ensemble un reste -CH=CH-CH=CH-, le système naphtyle formé pouvant alors être substitué une ou plusieurs fois,

X est choisi entre

H, F, Cl, Br, I, $CF_3$, $O-S(O_2)-C_6H_4-pCH_3$, $OR^{13}$ ou $OC(O)R^{13}$, où $R^{13}$ est choisi entre

H ; alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ; cycloalkyle en $C_3$ à $C_7$, saturé ou non saturé, substitué une ou plusieurs fois ou non substitué, ou un hétérocycle correspondant dans lequel un atome de carbone du noyau est remplacé par N, S ou O ; alkylaryle, saturé ou non saturé, substitué une ou plusieurs fois ou non substitué ; aryle ou hétéroaryle, chacun substitué une ou plusieurs fois ou non substitué ;

ou bien

lorsque le composé ne présente pas de reste X, une double liaison est formée entre l'atome de carbone A et l'atome de carbone B ou entre l'atome de carbone B et l'atome de carbone C conformément à la formule Ia ; et

$R^3$, $R^4$ sont choisis indépendamment l'un de l'autre entre

H ; alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ; cycloalkyle en $C_3$ à $C_7$, saturé ou non saturé, substitué une ou plusieurs fois ou non substitué, ou un hétérocycle correspondant dans lequel un atome de carbone du noyau est remplacé par N, S ou O ; alkylaryle, saturé ou non saturé, substitué une ou plusieurs fois ou non substitué ; aryle ou hétéroaryle, chacun substitué une ou plusieurs fois ou non substitué ;

ou bien

$R^3$ et $R^4$ forment ensemble un reste cycloalkyle en $C_3$ à $C_7$, saturé ou non saturé, substitué une ou plusieurs fois ou non substitué, ou bien un hétérocycle correspondant, dans lequel un atome de carbone du noyau est remplacé par S, 0 ou $NR^{14}$, $R^{14}$ étant choisi entre

H, alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ;

sous réserve que,

lorsque X = OH, $R^2$ = H et $R^3$ et $R^4$ sont chacun un groupe $CH_3$, $R^1$ ne puisse pas être en même temps $R^5$ avec

$R^5 = OR^7$ où $R^7$ = H, $CH_3$ ou un groupe pyridyle, thiényle, thiazoyle ou phényle non substitué ou substitué, ou bien $R^5 = OC(O)OR^7$ avec $R^7$ = alkyle en $C_1$ à $C_5$, ou bien $R^5 = OC(O)R^7$ avec $R^7$ = alkyle en $C_1$ à $C_5$, NH-$C_6H_4$-alkyle en $C_1$ à $C_3$, $C_6H_4$-OC(O)alkyle en $C_1$ à $C_3$, $C_6H_4$-$CH_2$-N (alkyle en $C_1$ à $C_4$)$_2$, $C_6H_4$-$CH_2$-(N-4-morpholino) ou CHZ'-NHZ" avec Z' et Z" identiques ou différents choisis entre H ou alkyle en $C_1$ à $C_6$,
ou que,
lorsque X = H ou une double liaison est formée entre l'atome de carbone A et l'atome de carbone B conformément à la formule Ia, $R^2$ = H et $R^3$ et $R^4$ représentent chacun, indépendamment l'un de l'autre, un reste alkyle en $C_1$ à $C_6$, aryle ou cycloalkyle en $C_3$ à $C_7$, $R^1$ ne puisse pas être en même temps H, un reste alkoxy en $C_1$ à $C_6$, O-cycloalkyle en $C_3$ à $C_7$, O-aryle ou O-hétérocyclyle.

2. Dérivés d' aminométhyl-phényl-cyclohexanone suivant la revendication 1, **caractérisés en ce que** $R^1 = R^5$, $R^5$ étant choisi entre
H, F, Cl, Br, I, $CHF_2$, $CF_3$, $NO_2$, $NH_2$ ; alkyle en $C_1$ à $C_4$ ou alcényle en $C_2$ à $C_4$, ramifié ou non ramifié, substitué une ou plusieurs fois ou non substitué ; aryle substitué ou non substitué ; $OR^7$, $C(O)OR^7$ ou $SR^7$, $R^7$ étant choisi entre
H ; alkyle en $C_1$ à $C_4$, ramifié ou non ramifié, substitué une ou plusieurs fois ou non substitué, avantageusement H, $CF_3$ ou $CH_3$,
ou bien S ($O_2$) $NR^9R^{10}$, $R^9$ et $R^{10}$ étant choisis indépendamment l'un de l'autre entre
H ; alkyle en $C_1$ à $C_4$, ramifié ou non ramifié, substitué une ou plusieurs fois ou non substitué ;
R1 étant très avantageusement égal à R5, R5 étant choisi entre
H, F, Cl, OH, $CH_3$, $C_2H_5$, $C_2H_3$, $CHF_2$, $CF_3$, $SCH_3$, $OCF_3$, $OCH_3$, $OC_2H_5$, $C(O)OCH_3$, $C(O)OC_2H_5$ ou phényle,
tandis que $R^2$, X, $R^3$ et $R^4$ ont l'une des définitions déjà mentionnées.

3. Dérivés d'aminométhyl-phényl-cyclohexanone suivant l'une des revendications 1 et 2, **caractérisés en ce que** $R^2 = R^5$, $R^5$ étant choisi entre
H, F, Cl, Br, I, $SCH_3$ ; alkyle en $C_1$ à $C_4$, alcényle en $C_2$ à $C_4$, ramifié ou non ramifié, substitué une ou plusieurs fois ou non substitué ; avantageusement $CF_3$ ; $OR^7$, $R^7$ étant choisi entre des restes alkyle en $C_1$ à $C_4$, ramifié ou non ramifié, substitué une ou plusieurs fois ou non substitué, avantageusement $CH_3$ ;
tandis que $R^1$, X, $R^3$ et $R^4$ ont l'une des définitions mentionnées dans les revendications précédentes.

4. Dérivés d'aminométhyl-phényl-cyclohexanone suivant l'une des revendications 1 à 3, **caractérisés en ce que** $R^1$ et $R^2$ ont des définitions différentes ou bien
$R^1$ et $R^2$ forment ensemble un groupe -CH=CH-CH=CH-, le système naphtyle produit pouvant être non substitué ou substitué une ou plusieurs fois, avantageusement avec un halogène, un radical O-alkyle en $C_1$ à $C_3$ ou alkyle en $C_1$ à $C_3$, en particulier avec $OCH_3$.

5. Dérivés d'aminométhyl-phényl-cyclohexanone suivant l'une des revendications 1 à 4, caractérisés en ce X est choisi entre
H, F, Cl, OH, $CF_3$, O-S($O_2$)-$C_6H_4$-p$CH_3$ ou $OC(O)R^7$ avec $R^7$ = H ; alkyle en $C_1$ à $C_4$ ou alcényle en $C_2$ à $C_4$, ramifié ou non ramifié, substitué une ou plusieurs fois ou non substitué, avantageusement H, F, Cl, OH, O-S ($O_2$)-$C_6H_4$-p$CH_3$, $OC(O)R^7$ avec $R^7$ = alkyle en $C_1$ à $C_4$, avantageusement $CH_3$, en particulier H ou OH ;
ou bien,
lorsque le composé ne porte pas de reste X, une double liaison est formée entre l'atome de carbone A et l'atome de carbone B ou entre l'atome de carbone B et l'atome de carbone C, conformément à la formule Ia,
tandis que $R^1$, $R^2$, $R^3$ et $R^4$ ont l'une des définitions mentionnées dans les revendications précédentes.

6. Dérivés d'aminométhyl-phényl-cyclohexanone suivant l'une des revendications 1 à 5, **caractérisés en ce que** $R^3$ et $R^4$ sont choisis indépendamment l'un de l'autre entre des restes alkyle en $C_1$ à $C_4$, ramifiés ou non ramifiés, substitués une ou plusieurs fois ou non substitués, avantageusement $CH_3$,
ou bien
$R^3$ et $R^4$ forment ensemble un reste cycloalkyle en $C_3$ à $C_7$, saturé ou non saturé, substitué une ou plusieurs fois ou non substitué,
tandis que $R^1$, $R^2$ et X ont l'une des définitions déjà mentionnées dans les revendications précédentes.

7. Procédé de production de dérivés d'aminométhylphényl-cyclohexanone suivant la revendication 1, de formule Ia ou de formule I avec X = H, **caractérisé en ce qu'**on fait réagir des composés de formule II

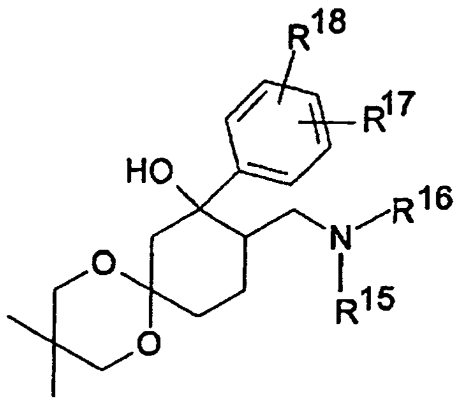

II

dans lesquels $R^{15}$ a une définition correspondant à $R^3$, $R^{16}$ une définition correspondant à $R^4$, $R^{17}$ une définition correspondant à $R^1$ et $R^{18}$ une définition correspondant à $R^2$ selon la revendication 1, avec des acides, avantageusement l'acide chlorhydrique, l'acide formique ou l'acide acétique, à la température ambiante, puis le produit de formule Ia est purifié ou hydrogéné avec de l'hydrogène activé par catalyse, avantageusement en présence d'un catalyseur au platine ou au palladium absorbé sur un support tel que le charbon actif, avantageusement dans l'ester éthylique d'acide acétique ou dans un alcool alkylique en $C_1$ à $C_4$, à des pressions de 0,1 à 10 bar et/ou à des températures de 20 à 80 °C puis le produit de formule I dans laquelle X représente H est purifié.

**8.** Procédé de production de dérivés d'aminométhylphényl-cyclohexanone suivant la revendication 1, de formule I dans laquelle X est différent de H, **caractérisé en ce qu'**on fait réagir des composés de formule II

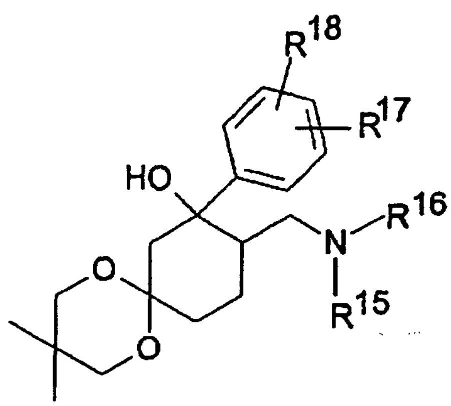

II

dans lesquels $R^{15}$ a une définition correspondant à $R^3$, $R^{16}$ une définition correspondant à $R^4$, $R^{17}$ une définition correspondant à $R^1$ et $R^{18}$ une définition correspondant à $R^2$ selon la revendication 1, avec des acides, avantageusement l'acide chlorhydrique, à des températures comprises entre 0 °C et 5 °C et, ou bien on purifie les produits ainsi obtenus de formule I dans laquelle X représente OH et $R^{15}$ à $R^{18}$ sont non modifiés, ou bien on poursuit leur traitement, auquel cas lorsque X doit représenter F, Cl, Br, I ou $CF_3$, le groupe OH est échangé contre F ou Cl ou Br ou I ou $CF_3$, lorsque X doit représenter $OR^{13}$, où $R^{13}$ a l'une des définitions indiquées dans la revendication 1, le groupe X = OH est éthérifié avec un halogénure de formule III

$$R^{19}Cl$$

III

lorsque $R^{19}$ a une définition analogue à celle de $R^{13}$, ou bien lorsque X représente $O\text{-}S(O_2)\text{-}C_6H_4\text{-}CH_3$ ou OC(O) $R^{13}$, où $R^{13}$ a l'une des définitions indiquées dans la revendication 1, le groupe X = OH est estérifié avec un chlorure d'acide de formule IV

$$R^{20}COCl$$

IV

ou $Cl\text{-}S(O_2)\text{-}C_6H_4\text{-}CH_3$, $R^{20}$ ayant une définition analogue à celle de $R^{13}$, et finalement, on purifie le produit.

**9.** Procédé suivant l'une des revendications 7 et 8, **caractérisé en ce que**, pour la préparation du composé de départ de formule II, on fait tout d'abord réagir de la 3,3-diméthyl-1,5-dioxa-spiro[5.5]undécane-8-one

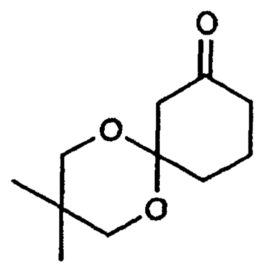

avec des sels d'immonium de formule V ou avec du formaldéhyde et une amine de formule VI, $R^{15}$ ayant une définition correspondant à celle de $R^3$ et $R^{16}$ ayant une définition correspondant à celle de $R^4$ selon la revendication 1,

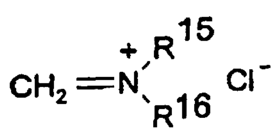

**V**

$$R^{15}R^{16}NH \qquad VI$$

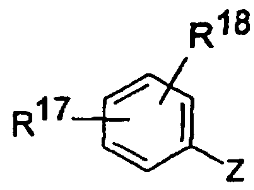

**VII**

puis on fait réagir les bases de Mannich ainsi obtenues avec un composé organométallique de formule VII dans laquelle Z représente MgCl, MgBr, MgI ou le lithium et $R^{17}$ a une définition correspondant à celle de $R^1$ et $R^{18}$ une définition correspondant à celle de $R^2$ selon la revendication 1, à des températures comprises entre -70°C et 60 °C, avantageusement dans l'éther de diéthyle et/ou dans le tétrahydrofuranne.

**10.** Procédé suivant l'une des revendications 7 à 9, **caractérisé en ce que** préalablement à la purification finale, un produit du procédé portant au moins un groupe $C(O)OCH_3$, $OC(O)OCH_3$ et/ou $C(S)OCH_3$ est saponifié avec une solution de KOH ou une solution de NaOH dans le méthanol à une température de 40 à 60 °C.

**11.** Procédé suivant l'une des revendications 7 à 10, **caractérisé en ce que** dans les restes $R^{15}$ à $R^{20}$ selon les formules II à VII, au moins un groupe OH a été remplacé par un groupe $OSi(Ph)_2$tertiobutyle, au moins un groupe SH a été remplacé par un groupe S-p-méthoxybenzyle et/ou au moins un groupe $NH_2$ a été remplacé par un groupe $NO_2$ et immédiatement avant la purification du produit final, au moins un groupe $OSi(Ph)_2$tertiobutyle est éliminé avec du fluorure de tétrabutylammonium dans le tétrahydrofuranne et/ou au moins un groupe p-méthoxybenzyle est éliminé avec un amidure métallique, avantageusement l'amidure de sodium et/ou au moins un groupe $NO_2$ est réduit en groupe $NH_2$.

**12.** Médicament contenant comme substance active au moins un dérivé d'aminométhyl-phénylcyclohexanone de formule générale I ou Ia

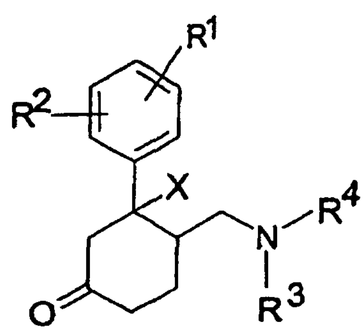

**I**

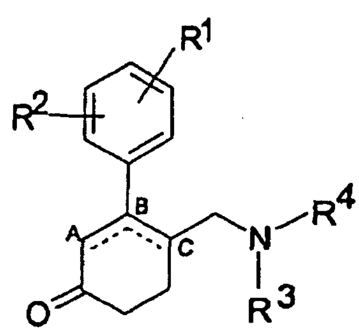

**Ia**

où

$R^1$ et $R^2$ sont choisis, indépendamment l'un de l'autre, parmi des restes $R^5$ ou $YR^5$ avec Y = alkyle en $C_1$ à $C_{10}$, alcényle en $C_2$ à $C_{10}$ ou alcynyle en $C_2$ à $C_{10}$, ramifié ou non ramifié, substitué une ou plusieurs fois ou non substitué, $R^5$ étant choisi entre

H, F, Cl, Br, I, CN, $NO_2$ ; alkyle en $C_1$ à $C_8$, alcényle en $C_2$ à $C_8$ ou alcynyle en $C_2$ à $C_8$, chacun ramifié ou non ramifié, substitué une ou plusieurs fois ou non substitué ; cycloalkyle en $C_3$ à $C_7$, saturé ou non saturé, substitué une ou plusieurs fois ou non substitué, ou un hétérocycle correspondant dans lequel un atome de carbone du noyau est remplacé par S, 0 ou $NR^6$, $R^6$ étant choisi entre

H, alkyle en $C_1$ à $C_{10}$, alcényle en $C_2$ à $C_{10}$ ou alcynyle en $C_2$ à $C_{10}$, chacun ramifié ou non ramifié,

substitué une ou plusieurs fois ou non substitué ;

aryle ou hétéroaryle, chacun substitué une ou plusieurs fois ou non substitué ;

$OR^7$, $OC(O)R^7$, $OC(O)OR^7$, $OC(S)R^7$, $C(O)R^7$, $C(O)OR^7$, $C(S)R^7$, $C(S)OR^7$, $SR^7$, $S(O)R^7$ ou $S(O_2)R^7$, où $R^7$ est choisi entre

H, alkyle en $C_1$ à $C_{18}$, alcényle en $C_2$ à $C_{18}$ ou alcynyle en $C_2$ à $C_{18}$, chacun ramifié ou non ramifié, substitué une ou plusieurs fois ou non substitué ; cycloalkyle en $C_3$ à $C_7$, saturé ou non saturé, substitué une ou plusieurs fois ou non substitué, ou un hétérocycle correspondant, dans lequel un atome de carbone du noyau est remplacé par S, 0 ou $NR^8$, $R^8$ étant choisi entre

H, alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ;

alkylaryle, saturé ou non saturé, substitué une ou plusieurs fois ou non substitué ; aryle ou hétéroaryle, chacun substitué une ou plusieurs fois ou non substitué ; ou bien

$NR^9R^{10}$, $C(O)NR^9R^{10}$ ou $S(O_2)NR^9R^{10}$, $R^9$ et $R^{10}$ étant choisis indépendamment l'un de l'autre entre

H, alkyle en $C_1$ à $C_{18}$, alcényle en $C_2$ à $C_{18}$ ou alcynyle en $C_2$ à $C_{18}$, chacun ramifié ou non ramifié, substitué une ou plusieurs fois ou non substitué ; cycloalkyle en $C_3$ à $C_7$, saturé ou non saturé, substitué une ou plusieurs fois ou non substitué, ou un hétérocycle correspondant, dans lequel un atome de carbone du noyau est remplacé par S, O ou $NR^{11}$, $R^{11}$ étant choisi entre

H, alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ;

alkylaryle, saturé ou non saturé, substitué une ou plusieurs fois ou non substitué ; aryle ou hétéroaryle, chacun substitué une ou plusieurs fois ou non substitué ;

ou bien

$R^9$ et $R^{10}$ forment ensemble un reste cycloalkyle en $C_3$ à $C_7$, saturé ou non saturé, substitué une ou plusieurs fois ou non substitué, ou bien un hétérocycle correspondant dans lequel un atome de carbone du noyau est remplacé par S, O ou

$NR^{12}$, $R^{12}$ étant choisi entre

H, alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ;

ou bien,

$R^1$ et $R^2$ forment ensemble un reste -CH=CH-CH=CH-, le système naphtyle formé pouvant alors être substitué une ou plusieurs fois,

X est choisi entre

H, F, Cl, Br, I, $CF_3$, $O-S(O_2)-C_6H_4-pCH_3$, $OR^{13}$ ou $OC(O)R^{13}$, où $R^{13}$ est choisi entre

H ; alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ; cycloalkyle en $C_3$ à $C_7$, saturé ou non saturé, substitué une ou plusieurs fois ou non substitué, ou un hétérocycle correspondant dans lequel un atome de carbone du noyau est remplacé par N, S ou 0 ; alkylaryle, saturé ou non saturé, substitué une ou plusieurs fois ou non substitué ; aryle ou hétéroaryle, chacun substitué une ou plusieurs fois ou non substitué ;

ou bien

lorsque le composé ne présente pas de reste X, une double liaison est formée entre l'atome de carbone A et l'atome de carbone B ou entre l'atome de carbone B et l'atome de carbone C conformément à la formule Ia ; et

$R^3$, $R^4$ sont choisis indépendamment l'un de l'autre entre

H ; alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ; cycloalkyle en $C_3$ à $C_7$, saturé ou non saturé, substitué une ou plusieurs fois ou non substitué, ou un hétérocycle correspondant dans lequel un atome de carbone du noyau est remplacé par N, S ou O ; alkylaryle, saturé ou non saturé, substitué une ou plusieurs fois ou non substitué ; aryle ou hétéroaryle, chacun substitué une ou plusieurs fois ou non substitué ;

ou bien

$R^3$ et $R^4$ forment ensemble un reste cycloalkyle en $C_3$ à $C_7$, saturé ou non saturé, substitué une ou plusieurs fois ou non substitué, ou bien un hétérocycle correspondant, dans lequel un atome de carbone du noyau est remplacé par S, O ou $NR^{14}$, $R^{14}$ étant choisi entre

H, alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ;

sous forme de ses diastéréoisomères ou énantiomères ainsi que de sa base libre ou d'un sel formé avec un acide acceptable du point de vue physiologique, en particulier sous forme du chlorhydrate.

13. Médicament suivant la revendication 12, **caractérisé en ce que** dans les formules générales I et Ia, indépendamment l'une de l'autre

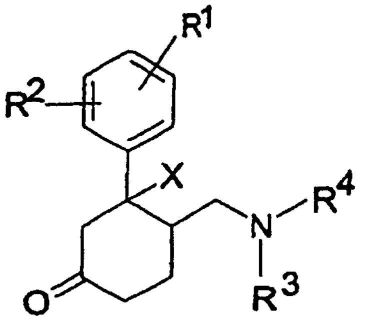

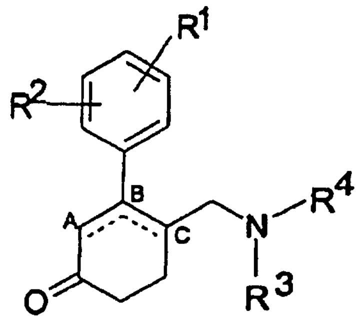

I

Ia

$R^1 = R^5$, $R^5$ étant choisi entre

H, F, Cl, Br, I, $CHF_2$, $CF_3$, $NO_2$, $NH_2$ ; alkyle en $C_1$ à $C_4$ ou alcényle en $C_2$ à $C_4$, ramifié ou non ramifié, substitué une ou plusieurs fois ou non substitué ; aryle substitué ou non substitué ; $OR^7$, $C(O)OR^7$ ou $SR^7$, $R^7$ étant choisi entre

H ; alkyle en $C_1$ à $C_4$, ramifié ou non ramifié, substitué une ou plusieurs fois ou non substitué ; avantageusement H, $CF_3$ ou $CH_3$,

ou bien $S(O_2)NR^9R^{10}$, où $R^9$ et $R^{10}$ sont choisis indépendamment l'un de l'autre entre

H ; alkyle en $C_1$ à $C_4$, ramifié ou non ramifié, substitué une ou plusieurs fois ou non substitué ;

$R^1$ identique à $R^5$ étant particulièrement apprécié, $R^5$ étant choisi entre

H, F, Cl, OH, $CH_3$, $C_2H_5$, $C_2H_3$, $CHF_2$, $CF_3$, $SCH_3$, $OCF_3$, $OCH_3$, $OC_2H_5$, $C(O)OCH_3$, $C(O)OC_2H_5$ ou phényle,

**et/ou** $R^2 = R^5$, $R^5$ étant choisi entre

H, F, Cl, Br, I, $SCH_3$ ; alkyle en $C_1$ à $C_4$, alcényle en $C_2$ à $C_4$ ramifié ou non ramifié, substitué une ou plusieurs fois ou non substitué, avantageusement $CF_3$ ; $OR^7$, $R^7$ étant choisi entre alkyle en $C_1$ à $C_4$, ramifié ou non ramifié, substitué une ou plusieurs fois ou non substitué, avantageusement $CH_3$ ;

**et/ou** $R^1$ et $R^2$ ont des définitions différentes, ou bien $R^1$ et $R^2$ forment ensemble un groupe - CH=CH-CH=CH-, le système naphtyle formé pouvant être substitué une ou plusieurs fois, avantageusement avec un halogène, un radical O-(alkyle en $C_1$ à $C_3$) ou alkyle en $C_1$ à $C_3$, non substitué ou substitué une ou plusieurs fois, en particulier $OCH_3$ ;

**et/ou** X est choisi entre H, F, Cl, OH, $CF_3$, O-$S(O_2)$-$C_6H_4$-p$CH_3$ ou $OC(O)R^7$, où $R^7$ représente H ; alkyle en $C_1$ à $C_4$ ou alcényle en $C_2$ à $C_4$, ramifié ou non ramifié, substitué une ou plusieurs fois ou non substitué,

avantageusement H, F, Cl, OH, O-$S(O_2)$-$C_6H_4$-p$CH_3$, $OC(O)R^7$, avec $R^7$ = alkyle en $C_1$ à $C_4$, avantageusement $CH_3$, en particulier H ou OH ;

ou bien

lorsque le composé ne porte pas de reste X, une double liaison est formée entre l'atome de carbone A et l'atome de carbone B ou entre l'atome de carbone B et l'atome de carbone C conformément à la formule la

**et/ou** $R^3$ et $R^4$ sont choisis indépendamment l'un de l'autre entre alkyle en $C_1$ à $C_4$, ramifiés ou non ramifiés, substitués une ou plusieurs fois ou non substitués, avantageusement $CH_3$ ;

ou bien

$R^3$ et $R^4$ forment ensemble un reste cycloalkyle en $C_3$ à $C_7$ saturé ou non saturé, substitué une ou plusieurs fois ou non substitué,

**et/ou** $R^1$, $R^2$, X, $R^3$ et/ou $R^4$ ont l'une des définitions mentionnées dans les revendications précédentes.

**14.** Médicament suivant la revendication 12, contenant comme substance active au moins un dérivé d'aminométhyl-phényl-cyclohexanone choisi dans le groupe :

- *rac-cis*-[4-diméthylaminométhyl-3-hydroxy-3-(3-méthoxyphényl)-cyclohexanone],
- *rac-cis*-[4-diméthylaminométhyl-3-(3-méthoxy-phényl)-cyclohexanone],

- *rac-trans*-[4-diméthylaminométhyl-3-(3-méthoxy-phényl)-cyclohexanone],
- *rac-cis*-[4-diméthylaminométhyl-3-(3-hydroxy-phényl)-cyclohexanone],
- *rac-trans*-[4-diméthylaminométhyl-3-(3-hydroxy-phényl)-cyclohexanone] ou
- 4-diméthylaminométhyl-3-(3-méthoxy-phényl)-cyclohex-2-énone,
- 4-diméthylaminométhyl-3-phényl-cyclohex-2-énone ;
- 4-diméthylaminométhyl-3-(3-hydroxy-phényl)-cyclohex-2-énone ;
- rac-trans-4-diméthylaminométhyl-3-phényl-cyclohexanone ;
- rac-cis-4-diméthylaminométhyl-3-phényl-cyclohexanone ;
- 4-diméthylaminométhyl-3-(4-méthoxy-phényl)-cyclohex-2-énone ;
- 4-diméthylaminométhyl-3-naphtalène-2-yl-cyclohex-2-énone ;
- rac-trans-4-diméthylaminométhyl-3-hydroxy-3-naphtalène-2-yl-cyclohexanone ;
- rac-trans-4-diméthylaminométhyl-3-(3-fluoro-phényl)-cyclohexanone ;
- rac-cis-4-diméthylaminométhyl-3-(3-fluoro-phényl)-cyclohexanone ;
- rac-cis-4-diméthylaminométhyl-3-hydroxy-3-(2-méthoxyphényl)-cyclohexanone ;
- rac-cis-4-diméthylaminométhyl-3-hydroxy-3-(6-méthoxynaphtalène-2-yl)-cyclohexanone ;
- 4-diméthylaminométhyl-3-(6-méthoxy-naphtalène-2-yl)-cyclohex-2-énone ;
- rac-cis-3-biphényl-4-yl-4-diméthylaminométhyl-3-hydroxycyclohexanone ;
- 3-(3-difluorométhyl-phényl)-4-diméthylaminométhylcyclohex-2-énone ;
- 4-diméthylaminométhyl-3-(3-fluoro-phényl)-cyclohex-2-énone ;
- 3-(3,4-difluoro-phényl)-4-diméthylaminométhyl-cyclohex-2-énone ;
- 4-diméthylaminométhyl-3-(4-fluoro-phényl)-cyclohex-2-énone ;
- 4-diméthylaminométhyl-3-(4-trifluorométhyl-phényl)-cyclohex-2-énone ;
- 3-(3,5-dichloro-phényl)-4-diméthylaminométhyl-cyclohex-2-énone ;
- 3-(3,5-difluoro-phényl)-4-diméthylaminométhyl-cyclohex-2-énone ;
- rac-cis-3-(3,5-difluoro-phényl)-4-diméthylaminométhyl-3-hydroxy-cyclohexanone

sous forme de la base libre ou d'un sel formé avec un acide acceptable du point de vue physiologique, en particulier sous forme du chlorhydrate.

**15.** Utilisation d'au moins un dérivé d'aminométhyl-phényl-cyclohexanone de formule générale I ou Ia

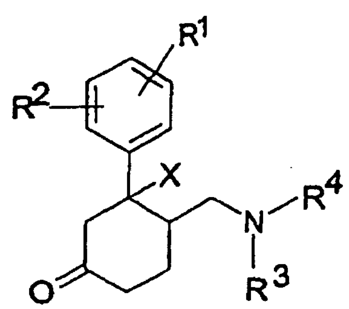

I

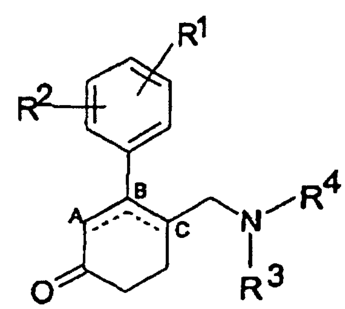

Ia

où
$R^1$ et $R^2$ sont choisis, indépendamment l'un de l'autre, parmi des restes $R^5$ ou $YR^5$ avec Y = alkyle en $C_1$ à $C_{10}$, alcényle en $C_2$ à $C_{10}$ ou alcynyle en $C_2$ à $C_{10}$, ramifié ou non ramifié, substitué une ou plusieurs fois ou non substitué, $R^5$ étant choisi entre

H, F, Cl, Br, I, CN, $NO_2$, alkyle en $C_1$ à $C_8$, alcényle en $C_2$ à $C_8$ ou alcynyle en $C_2$ à $C_8$, chacun ramifié ou non ramifié, substitué une ou plusieurs fois ou non substitué ; cycloalkyle en $C_3$ à $C_7$, saturé ou non saturé, substitué une ou plusieurs fois ou non substitué, ou un hétérocycle correspondant dans lequel un atome de carbone du noyau est remplacé par S, O ou $NR^6$, $R^6$ étant choisi entre

H, alkyle en $C_1$ à $C_{10}$, alcényle en $C_2$ à $C_{10}$ ou alcynyle en $C_2$ à $C_{10}$, chacun ramifié ou non ramifié,

substitué une ou plusieurs fois ou non substitué ;

aryle ou hétéroaryle, chacun substitué une ou plusieurs fois ou non substitué ;

$OR^7$, $OC(O)R^7$, $OC(O)OR^7$, $OC(S)R^7$, $C(O)R^7$, $C(O)OR^7$, $C(S)R^7$, $C(S)OR^7$, $SR^7$, $S(O)R^7$ ou $S(O_2)R^7$, où $R^7$ est choisi entre

H, alkyle en $C_1$ à $C_{18}$, alcényle en $C_2$ à $C_{18}$ ou alcynyle en $C_2$ à $C_{18}$, chacun ramifié ou non ramifié, substitué une ou plusieurs fois ou non substitué ; cycloalkyle en $C_3$ à $C_7$, saturé ou non saturé, substitué une ou plusieurs fois ou non substitué, ou un hétérocycle correspondant, dans lequel un atome de carbone du noyau est remplacé par S, O ou $NR^8$, $R^8$ étant choisi entre

H, alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ;

alkylaryle, saturé ou non saturé, substitué une ou plusieurs fois ou non substitué ; aryle ou hétéroaryle, chacun substitué une ou plusieurs fois ou non substitué ; ou bien

$NR^9R^{10}$, $C(O)NR^9R^{10}$ ou $S(O_2)NR^9R^{10}$, $R^9$ et $R^{10}$ étant choisis indépendamment l'un de l'autre entre

H, alkyle en $C_1$ à $C_{18}$, alcényle en $C_2$ à $C_{18}$ ou alcynyle en $C_2$ à $C_{18}$, chacun ramifié ou non ramifié, substitué une ou plusieurs fois ou non substitué ; cycloalkyle en $C_3$ à $C_7$, saturé ou non saturé, substitué une ou plusieurs fois ou non substitué, ou un hétérocycle correspondant, dans lequel un atome de carbone du noyau est remplacé par S, 0 ou $NR^{11}$, $R^{11}$ étant choisi entre

H, alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ;

alkylaryle, saturé ou non saturé, substitué une ou plusieurs fois ou non substitué ; aryle ou hétéroaryle, chacun substitué une ou plusieurs fois ou non substitué ;

ou bien

$R^9$ et $R^{10}$ forment ensemble un reste cycloalkyle en $C_3$ à $C_7$, saturé ou non saturé, substitué une ou plusieurs fois ou non substitué, ou bien un hétérocycle correspondant dans lequel un atome de carbone du noyau est remplacé par S, O ou $NR^{12}$, $R^{12}$ étant choisi entre

H, alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ;

ou bien,

$R^1$ et $R^2$ forment ensemble un reste -CH=CH-CH=CH-, le système naphtyle formé pouvant alors être substitué une ou plusieurs fois,

X est choisi entre

H, F, Cl, Br, I, $CF_3$, O-S $(O_2)$ $-C_6H_4-pCH_3$, $OR^{13}$ ou $OC(O)R^{13}$, où $R^{13}$ est choisi entre

H ; alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ; cycloalkyle en $C_3$ à $C_7$, saturé ou non saturé, substitué une ou plusieurs fois ou non substitué, ou un hétérocycle correspondant dans lequel un atome de carbone du noyau est remplacé par N, S ou O ; alkylaryle, saturé ou non saturé, substitué une ou plusieurs fois ou non substitué ; aryle ou hétéroaryle, chacun substitué une ou plusieurs fois ou non substitué ;

ou bien

lorsque le composé ne présente pas de reste X, une double liaison est formée entre l'atome de carbone A et l'atome de carbone B ou entre l'atome de carbone B et l'atome de carbone C conformément à la formule Ia ;

et

$R^3$, $R^4$ sont choisis indépendamment l'un de l'autre entre

H ; alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ; cycloalkyle en $C_3$ à $C_7$, saturé ou non saturé, substitué une ou plusieurs fois ou non substitué, ou un hétérocycle correspondant dans lequel un atome de carbone du noyau est remplacé par N, S ou O ; alkylaryle, saturé ou non saturé, substitué une ou plusieurs fois ou non substitué ; aryle ou hétéroaryle, chacun substitué une ou plusieurs fois ou non substitué ;

ou bien

$R^3$ et $R^4$ forment ensemble un reste cycloalkyle en $C_3$ à $C_7$, saturé ou non saturé, substitué une ou plusieurs fois ou non substitué, ou bien un hétérocycle correspondant, dans lequel un atome de carbone du noyau est remplacé par S, O ou $NR^{14}$, $R^{14}$ étant choisi entre

H, alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ;

sous forme de ses diastéréoisomères ou énantiomères ainsi que de sa base libre ou d'un sel formé avec un acide acceptable du point de vue physiologique, en particulier de son chlorhydrate, pour la préparation d'un médicament destiné au traitement de la douleur.

**16.** Utilisation d'au moins un dérivé d'aminométhyl-phényl-cyclohexanone de formule générale I ou Ia

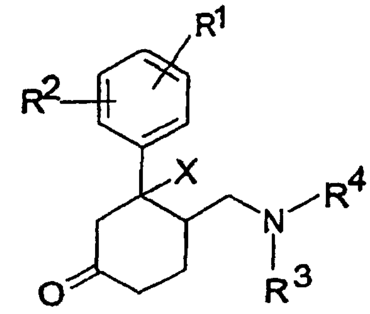

I

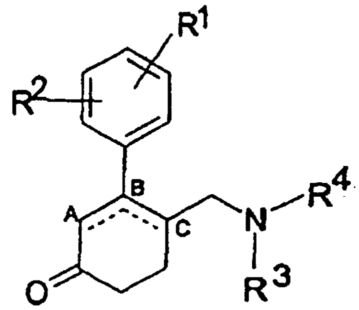

Ia

où

$R^1$ et $R^2$ sont choisis, indépendamment l'un de l'autre, parmi des restes $R^5$ ou $YR^5$ avec Y = alkyle en $C_1$ à $C_{10}$, alcényle en $C_2$ à $C_{10}$ ou alcynyle en $C_2$ à $C_{10}$, ramifié ou non ramifié, substitué une ou plusieurs fois ou non substitué, $R^5$ étant choisi entre

H, F, Cl, Br, I, CN, $NO_2$ ; alkyle en $C_1$ à $C_8$, alcényle en $C_2$ à $C_8$ ou alcynyle en $C_2$ à $C_8$, chacun ramifié ou non ramifié, substitué une ou plusieurs fois ou non substitué ; cycloalkyle en $C_3$ à $C_7$, saturé ou non saturé, substitué une ou plusieurs fois ou non substitué, ou un hétérocycle correspondant dans lequel un atome de carbone du noyau est remplacé par S, O ou $NR^6$, $R^6$ étant choisi entre

H, alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ;

aryle ou hétéroaryle, chacun substitué une ou plusieurs fois ou non substitué ;

$OR^7$, $OC(O)R^7$, $OC(O)OR^7$, $OC(S)R^7$, $C(O)R^7$, $C(O)OR^7$, $C(S)R^7$, $C(S)OR^7$, $SR^7$, $S(O)R^7$ ou $S(O_2)R^7$, où $R^7$ est choisi entre

H, alkyle en $C_1$ à $C_{18}$, alcényle en $C_2$ à $C_{18}$ ou alcynyle en $C_2$ à $C_{18}$, chacun ramifié ou non ramifié, substitué une ou plusieurs fois ou non substitué ; cycloalkyle en $C_3$ à $C_7$, saturé ou non saturé, substitué une ou plusieurs fois ou non substitué, ou un hétérocycle correspondant, dans lequel un atome de carbone du noyau est remplacé par S, O ou $NR^8$, $R^8$ étant choisi entre

H, alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ;

alkylaryle, saturé ou non saturé, substitué une ou plusieurs fois ou non substitué ; aryle ou hétéroaryle, chacun substitué une ou plusieurs fois ou non substitué ; ou bien

$NR^9R^{10}$, $C(O)NR^9R^{10}$ ou $S(O_2)NR^9R^{10}$, $R^9$ et $R^{10}$ étant choisis indépendamment l'un de l'autre entre

H, alkyle en $C_1$ à $C_{18}$, alcényle en $C_2$ à $C_{18}$ ou alcynyle en $C_2$ à $C_{18}$, chacun ramifié ou non ramifié, substitué une ou plusieurs fois ou non substitué ; cycloalkyle en $C_3$ à $C_7$, saturé ou non saturé, substitué une ou plusieurs fois ou non substitué, ou un hétérocycle correspondant, dans lequel un atome de carbone du noyau est remplacé par S, O ou $NR^{11}$, $R^{11}$ étant choisi entre

H, alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ;

alkylaryle, saturé ou non saturé, substitué une ou plusieurs fois ou non substitué ; aryle ou hétéroaryle, chacun substitué une ou plusieurs fois ou non substitué ;

ou bien

$R^9$ et $R^{10}$ forment ensemble un reste cycloalkyle en $C_3$ à $C_7$, saturé ou non saturé, substitué une ou plusieurs fois ou non substitué, ou bien un hétérocycle correspondant dans lequel un atome de carbone du noyau est remplacé par S, O ou $NR^{12}$, $R^{12}$ étant choisi entre

H, alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ;

ou bien,

$R^1$ et $R^2$ forment ensemble un reste -CH=CH-CH=CH-, le système naphtyle formé pouvant alors être substitué une ou plusieurs fois,

X est choisi entre

H, F, Cl, Br, I, $CF_3$, O-$S(O_2)$-$C_6H_4$-$pCH_3$, $OR^{13}$ ou $OC(O)R^{13}$, où $R^{13}$ est choisi entre

H ; alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ; cycloalkyle en $C_3$ à $C_7$, saturé ou non saturé, substitué une ou plusieurs fois ou non substitué, ou un hétérocycle correspondant dans lequel un atome de carbone du noyau est remplacé par N, S ou O ; alkylaryle, saturé ou non saturé, substitué une ou plusieurs fois ou non substitué ; aryle ou hétéroaryle, chacun substitué une ou plusieurs fois ou non substitué ;

ou bien

lorsque le composé ne présente pas de reste X, une double liaison est formée entre l'atome de carbone A et l'atome de carbone B ou entre l'atome de carbone B et l'atome de carbone C conformément à la formule Ia ;

et

$R^3$, $R^4$ sont choisis indépendamment l'un de l'autre entre

H ; alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ; cycloalkyle en $C_3$ à $C_7$, saturé ou non saturé, substitué une ou plusieurs fois ou non substitué, ou un hétérocycle correspondant dans lequel un atome de carbone du noyau est remplacé par N, S ou O ; alkylaryle, saturé ou non saturé, substitué une ou plusieurs fois ou non substitué ; aryle ou hétéroaryle, chacun substitué une ou plusieurs fois

ou non substitué ;

ou bien

$R^3$ et $R^4$ forment ensemble un reste cycloalkyle en $C_3$ à $C_7$, saturé ou non saturé, substitué une ou plusieurs fois ou non substitué, ou bien un hétérocycle correspondant, dans lequel un atome de carbone du noyau est remplacé par S, O ou $NR^{14}$, $R^{14}$ étant choisi entre

H, alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ;

sous forme de ses diastéréoisomères ou énantiomères ainsi que de sa base libre ou d'un sel formé avec un acide acceptable du point de vue physiologique, en particulier sous forme du chlorhydrate, pour la préparation d'un médicament destiné au traitement de réactions inflammatoires et allergiques, de dépressions, d'abus de drogue et/ou d'alcool, de gastrite, d'affections cardio-vasculaires, de maladies des voies respiratoires, de la toux, de maladies psychiques et/ou de l'épilepsie, ainsi que, en particulier, de l'incontinence urinaire, du prurit et/ou de la diarrhée.

**17.** Utilisation suivant l'une des revendications 15 et 16, **caractérisée en ce que**, dans les formules générales I et Ia, indépendamment l'une de l'autre

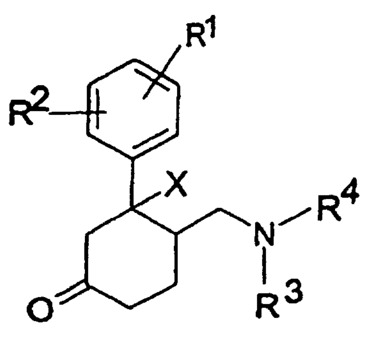

I

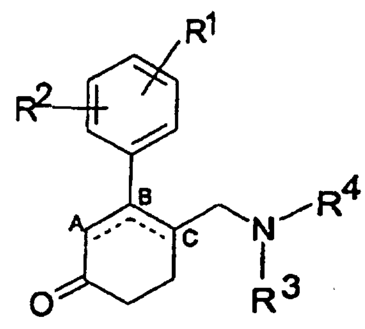

Ia

$R^1 = R^5$, $R^5$ étant choisi entre

H, F, Cl, Br, I, $CHF_2$, $CF_3$, $NO_2$, $NH_2$ ; alkyle en $C_1$ à $C_4$ ou alcényle en $C_2$ à $C_4$, ramifié ou non ramifié, substitué une ou plusieurs fois ou non substitué ; aryle substitué ou non substitué ; $OR^7$, $C(O)OR^7$ ou $SR^7$, $R^7$ étant choisi entre

H ; alkyle en $C_1$ à $C_4$, ramifié ou non ramifié, substitué une ou plusieurs fois ou non substitué ; avantageusement H, $CF_3$ ou $CH_3$,

ou bien $S(O_2)NR^9R^{10}$, où $R^9$ et $R^{10}$ sont choisis indépendamment l'un de l'autre entre

H ; alkyle en $C_1$ à $C_4$, ramifié ou non ramifié, substitué une ou plusieurs fois ou non substitué ;

$R^1$ identique à $R^5$ étant particulièrement apprécié, $R^5$ étant choisi entre

H, F, Cl, OH, $CH_3$, $C_2H_5$, $C_2H_3$, $CHF_2$, $CF_3$, $SCH_3$, $OCF_3$, $OCH_3$, $OC_2H_5$, $C(O)OCH_3$, $C(O)OC_2H_5$ ou phényle,

**et/ou** $R^2$ = $R^5$, $R^5$ étant choisi entre

H, F, Cl, Br, I, $SCH_3$ ; alkyle en $C_1$ à $C_4$, alcényle en $C_2$ à $C_4$ ramifié ou non ramifié, substitué une ou plusieurs fois ou non substitué, avantageusement $CF_3$ ; $OR^7$, $R^7$ étant choisi entre alkyle en $C_1$ à $C_4$, ramifié ou non ramifié, substitué une ou plusieurs fois ou non substitué, avantageusement $CH_3$ ;

**et/ou** $R^1$ et $R^2$ ont des définitions différentes, ou bien $R^1$ et $R^2$ forment ensemble un groupe - CH=CH-CH=CH-, le système naphtyle formé pouvant être substitué une ou plusieurs fois, avantageusement avec un halogène, un radical O-(alkyle en $C_1$ à $C_3$) ou alkyle en $C_1$ à $C_3$, non substitué ou substitué une ou plusieurs fois, en particulier $OCH_3$ ;

**et/ou** X est choisi entre

H, F, Cl, OH, $CF_3$, O-S($O_2$)-$C_6H_4$-p$CH_3$ ou OC(O)$R^7$, où $R^7$ représente H ; alkyle en $C_1$ à $C_4$ ou alcényle en $C_2$ à $C_4$, ramifié ou non ramifié, substitué une ou plusieurs fois ou non substitué,

avantageusement H, F, Cl, OH, O-S($O_2$)-$C_6H_4$-p$CH_3$, OC(O)$R^7$, avec $R^7$ = alkyle en $C_1$ à $C_4$, avantageusement $CH_3$, en particulier H ou OH ;

ou bien

lorsque le composé ne porte pas de reste X, une double liaison est formée entre l'atome de carbone A et l'atome de carbone B ou entre l'atome de carbone B et l'atome de carbone C conformément à la formule Ia

**et/ou** $R^3$ et $R^4$ sont choisis indépendamment l'un de l'autre entre alkyle en $C_1$ à $C_4$, ramifiés ou non ramifiés, substitués une ou plusieurs fois ou non substitués, avantageusement $CH_3$ ;

ou bien

$R^3$ et $R^4$ forment ensemble un reste cycloalkyle en $C_3$ à $C_7$, saturé ou non saturé, substitué une ou plusieurs fois ou non substitué,

**et/ou** $R^1$, $R^2$, X, $R^3$ et/ou $R^4$ ont l'une des définitions mentionnées dans les revendications précédentes.

**18.** Utilisation suivant la revendication 15 ou 16, **caractérisée en ce qu'**on choisit comme substance active au moins un dérivé d'aminométhyl-phénylcyclohexanone choisi dans le groupe :

- rac-cis-[4-diméthylaminométhyl-3-hydroxy-3-(3-méthoxyphényl)-cyclohexanone],
- *rac-cis*-[4-diméthylaminométhyl-3-(3-méthoxy-phényl)-cyclohexanone],
- rac-trans-[4-diméthylaminométhyl-3-(3-méthoxy-phényl)-cyclohexanone],
- *rac-cis*-[4-diméthylaminométhyl-3-(3-hydroxy-phényl)-cyclohexanone],
- rac-trans-[4-diméthylaminométhyl-3-(3-hydroxy-phényl)-cyclohexanone] ou
- 4-diméthylaminométhyl-3-(3-méthoxy-phényl)-cyclohex-2-énone,
- 4-diméthylaminométhyl-3-phényl-cyclohex-2-énone ;
- 4-diméthylaminométhyl-3-(3-hydroxy-phényl)-cyclohex-2-énone ;
- rac-trans-4-diméthylaminométhyl-3-phényl-cyclohexanone ;
- rac-cis-4-diméthylaminométhyl-3-phényl-cyclohexanone ;
- 4-diméthylaminométhyl-3-(4-méthoxy-phényl)-cyclohex-2-énone ;
- 4-diméthylaminométhyl-3-naphtalène-2-yl-cyclohex-2-énone ;
- rac-trans-4-diméthylaminométhyl-3-hydroxy-3-naphtalène-2-yl-cyclohexanone ;
- rac-trans-4-diméthylaminométhyl-3-(3-fluoro-phényl)-cyclohexanone ;
- rac-cis-4-diméthylaminométhyl-3-(3-fluoro-phényl)-cyclohexanone ;
- rac-cis-4-diméthylaminométhyl-3-hydroxy-3-(2-méthoxyphényl)-cyclohexanone ;
- rac-cis-4-diméthylaminométhyl-3-hydroxy-3-(6-méthoxynaphtalène-2-yl)-cyclohexanone ;
- 4-diméthylaminométhyl-3-(6-méthoxy-naphtalène-2-yl)-cyclohex-2-énone ;
- rac-cis-3-biphényl-4-yl-4-diméthylaminométhyl-3-hydroxycyclohexanone ;
- 3-(3-difluorométhyl-phényl)-4-diméthylaminométhylcyclohex-2-énone ;
- 4-diméthylaminométhyl-3-(3-fluoro-phényl)-cyclohex-2-énone ;
- 3-(3,4-difluoro-phényl)-4-diméthylaminométhyl-cyclohex-2-énone ;
- 4-diméthylaminométhyl-3-(4-fluoro-phényl)-cyclohex-2-énone ;
- 4-diméthylaminométhyl-3-(4-trifluorométhyl-phényl)-cyclohex-2-énone ;
- 3-(3,5-dichloro-phényl)-4-diméthylaminométhyl-cyclohex-2-énone ;
- 3-(3,5-difluoro-phényl)-4-diméthylaminométhyl-cyclohex-2-énone ;
- rac-cis-3-(3,5-difluoro-phényl)-4-diméthylaminométhyl-3-hydroxy-cyclohexanone

sous forme de la base libre ou d'un sel formé avec un acide acceptable du point de vue physiologique, en particulier sous forme du chlorhydrate.